(19) Europäisches Patentamt
European Patent Office
Office européen des brevets

(11) Veröffentlichungsnummer: **0 332 008**
**A2**

(12) # EUROPÄISCHE PATENTANMELDUNG

(21) Anmeldenummer: 89103416.7

(22) Anmeldetag: 27.02.89

(51) Int. Cl.4: C07D 233/64 , C07D 405/12 , C07D 277/30 , C07C 147/05 , C07K 5/06 , C07C 103/50 , C07D 231/12 , C07D 333/24 , C07D 417/12 , C07F 9/65 , C07D 417/06

Patentansprüche für folgende Vertragsstaaten: ES + GR

(30) Priorität: 04.03.88 CH 820/88
16.09.88 CH 3469/88
28.12.88 CH 4824/88

(43) Veröffentlichungstag der Anmeldung: 13.09.89 Patentblatt 89/37

(84) Benannte Vertragsstaaten: AT BE CH DE ES FR GB GR IT LI LU NL SE

(71) Anmelder: F. HOFFMANN-LA ROCHE AG
Postfach 3255
CH-4002 Basel(CH)

(72) Erfinder: Branca, Quirico
Lenzgasse 2
CH-4056 Basel(CH)
Erfinder: Neidhart, Werner, Dr.
Oltmannstrasse 14
D-7800 Freiburg im Breisgau(DE)
Erfinder: Ramuz, Henri, Prof. Dr.
Rheinparkstrasse 3
CH-4127 Birsfelden(CH)
Erfinder: Stadler, Heinz, Dr.
Waldhofstrasse 37
CH-4310 Rheinfelden(CH)
Erfinder: Wostl, Wolfgang, Dr.
Im Strick 2
D-7889 Grenzach-Wyhlen(DE)

(74) Vertreter: Lederer, Franz, Dr. et al
Patentanwält Dr. Franz Lederer Lucile
Grahnstrasse 22
D-8000 München 80(DE)

(54) **Peptidartige Aminosäurederivate.**

(57) Die Verbindungen der Formel

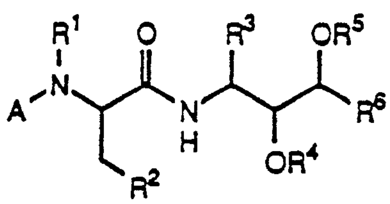

I

worin $R^1$, $R^2$, $R^3$, $R^4$ und $R^5$ die in der Beschreibung angegebene Bedeutung besitzen, $R^6$ eine der Gruppen

EP 0 332 008 A2

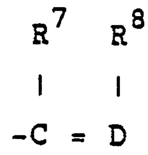

(a)

und

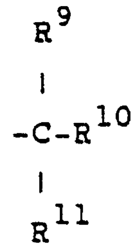

(b)

und A eine der Gruppen

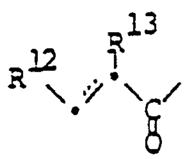

(c) und -Y-Z (d)

bedeuten, worin D eine Methingruppe oder ein Stickstoffatom, $R^7$ Alkyl, Aryl oder Arylalkl, $R^8$ Wasserstoff, Alkyl, Aryl oder Arylalkyl, oder $R^7$ und $R^8$ zusammen mit den beiden sie verbindenden Atomen, Aryl, Heteroaryl, Cycloalkenyl oder Heterocycloalkenyl, $R^9$ Wasserstoff oder Alkyl und $R^{10}$ und $R^{11}$ unabhängig voneinander je Alkyl, Aryl, Arylalkyl, Cycloalkyl oder die Gruppe

$-CH_2-X-R^{14}$ (e)

oder zusammen mit dem Kohlenstoffatom, an das sie gebunden sind, Cycloalkyl oder Heterocycloalkyl bedeuten, mit der Massgabe, dass, falls $R^9$ Alkyl bedeutet, $R^{10}$ und $R^{11}$ ebenfalls Alkyl bedeuten, die gestrichelte Linie eine zusätzliche Bindung bedeuten kann, $R^{12}$ $R^{13}$, $R^{14}$, Y, Z und X die in der Beschreibung angegebene Bedeutung besitzen,

in Form von optisch reinen Diastereomeren, Diastereomerengemischen, diastereomeren Racematen oder Gemischen von diastereomeren Racematen sowie pharmazeutisch verwendbare Salze davon hemmen die Wirkung des natürlichen Enzyms Renin und können demnach in Form pharmazeutischer Präparate bei der Bekämpfung bzw. Verhütung von Bluthochdruck und Herzinsuffizienz verwendet werden. Sie können aus den entsprechenden, nicht-acylierten Dihydroxyderivaten und gegebenenfalls nachfolgender O-Alkanoylierung bzw. Ueberführung in O-geschützte Derivate hergestellt werden.

## Peptidartige Aminosäurederivate

Die vorliegende Erfindung betrifft Aminosäurederivate. Im speziellen betrifft sie Aminosäurederivate der allgemeinen Formel

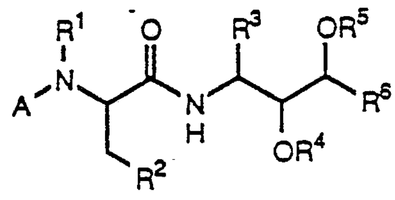

I

worin $R^1$ Wasserstoff oder Methyl, $R^2$ Aethyl, Propyl, Isopropyl, Imidazol-2-yl, Imidazol-4-yl, Pyrazol-3-yl, Thiazol-4-yl, Thien-2-yl oder t-Butoxy, $R^3$ Isobutyl, Cyclohexylmethyl oder Benzyl, $R^4$ und $R^5$ unabhängig voneinander je Wasserstoff, gegebenenfalls durch Amino, Monoalkylamino, Dialkylamino, Alkanoylamino, Alkanoylamino, Carboxy, Alkoxy oder Hydroxy ein-oder mehrfach substituiertes Alkanoyl oder eine O-Schutzgruppe oder zusammen eine cyclische O-Schutzgruppe, $R^6$ eine der Gruppen

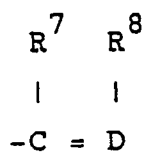

(a)

und

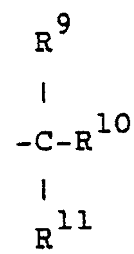

(b)

und A eine der Gruppen

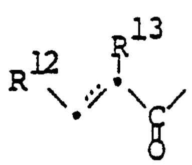

(c) und -Y-Z (d)

bedeuten, worin D eine Methingruppe oder ein Stickstoffatom, $R^7$ Alkyl, Aryl oder Arylalkyl, $R^8$ Wasserstoff, Alkyl, Aryl oder Arylalkyl, oder $R^7$ und $R^8$ zusammen mit den beiden sie verbindenden Atomen, Aryl, Heteroaryl, Cycloalkenyl oder Heterocycloalkenyl, $R^9$ Wasserstoff oder Alkyl und $R^{10}$ und $R^{11}$ unabhängig voneinander je Alkyl, Aryl, Arylalkyl, Cycloalkyl oder die Gruppe

$-CH_2-X-R^{14}$ (e)

oder zusammen mit dem Kohlenstoffatom, an das sie gebunden sind, Cycloalkyl oder Heterocycloalkyl bedeuten, mit der Massgabe, dass, falls $R^9$ Alkyl bedeutet, $R^{10}$ und $R^{11}$ ebenfalls Alkyl bedeuten, die gestrichelte Linie eine zusätzliche Bindung bedeuten kann, $R^{12}$ Phenyl, substituiertes Phenyl, Benzyl oder Naphthyl, und $R^{13}$ Wasserstoff, Alkoxycarbonylalkyl, Alkylcarbonylalkyl, Cycloalkylcarbonylalkyl, Heterocycloalkylcarbonylalkyl, Arylcarbonylalkyl, Aminocarbonylalkyl, substituiertes Aminocarbonylalkyl, Alkoxycarbonylhydroxyalkyl, Alkylcarbonylhydroxyalkyl, Cycloalkylcarbonylhydroxyalkyl, Heterocycloalkylcarbonylhydroxyalkyl, Arylcarbonylhydroxyalkyl, Aminocarbonylhydroxyalkyl, substituiertes Aminocarbonylhydroxyalkyl, Dialkoxyphosphoroxyalkyl, Diphenyloxyphosphoroxyalkyl, Arylalkyl, Alkoxycarbonylamino, Arylalkoxycarbonylamino, Alkylthioalkyl, Alkylsulfinylalkyl oder Alkylsulfonylalkyl bedeuten, mit der Massgabe, dass $R^{13}$ nicht Alkoxycarbonylamino oder Arylalkoxycarbonylamino bedeuten kann, wenn $R^{12}$ Phenyl, Benzyl oder α-Naphthyl bedeutet, Y den N-terminal mit Z verbundenen, bivalenten Rest von gegebenenfalls N- und/oder α-methyliertem Phenylalanin, Cyclohexylalanin, Tyrosin, O-Methyltyrosin, α-Naphthylalanin oder Homophenylalanin, Z Acyl, X ein Sauerstoff- oder Schwefelatom oder die Gruppe -NH- und $R^{14}$ Wasserstoff, Alkyl, Cycloalkyl, Arylalkyl, Cycloalkylalkyl, Alkylcarbonyl, Arylcarbonyl oder Arylalkylcarbonyl bedeuten,
in Form von optisch reinen Diastereomeren, Diastereomerengemischen, diastereomeren Racematen oder Gemischen von diastereomeren Racematen sowie pharmazeutisch verwendbare Salze dieser Verbindungen.

Diese Verbindungen sind neu und zeichnen sich durch wertvolle pharmakodynamische Eigenschaften aus.

Gegenstand der vorliegenden Erfindung sind die Verbindungen der Formel I und ihre pharmazeutisch verwendbaren Salze als solche und zur Anwendung als therapeutische Wirkstoffe, die Herstellung dieser Verbindungen, ferner diese enthaltende Arzneimittel und die Herstellung solcher Arzneimittel, sowie die Verwendung von Verbindungen der Formel I und ihrer pharmazeutisch verwendbaren Salze bei der Bekämpfung bzw. Verhütung von Krankheiten, bzw. bei der Verbesserung der Gesundheit, insbesondere bei der Bekämpfung bzw. Verhütung von Bluthochdruck und Herzinsuffizienz.

Der in der vorliegenden Beschreibung verwendete Ausdruck "Alkyl" - allein oder in Kombination - bedeutet geradkettige und verzweigte, gesättigte Kohlenwasserstoffreste mit 1-8, vorzugsweise 1-4, Kohlenstoffatomen, wie Methyl, Aethyl, n-Propyl, Isopropyl, n-Butyl, Isobutyl, sec.-Butyl, t-Butyl, Pentyl, Hexyl und dergleichen. Der Ausdruck "Alkoxy" bedeutet Alkyläthergruppen, worin der Ausdruck "Alkyl" die obige Bedeutung hat, wie Methoxy, Aethoxy, Propoxy, Isopropoxy, Butoxy, Isobutoxy, sec.-Butoxy, t-Butoxy und dergleichen. Der Ausdruck "Cycloalkyl" bedeutet gesättigte, cyclische Kohlenwasserstoffreste mit 3-8, vorzugsweise 3-6, Kohlenstoffatomen, wie Cyclopropyl, Cyclobutyl, Cyclopentyl, Cyclohexyl und dergleichen. Der Ausdruck "Alkanoyl" - allein oder in Kombination - bedeutet den Säurerest einer geradkettigen oder verzweigten Alkansäure mit 1-8, vorzugsweise 1-4, Kohlenstoffatomen, wie Formyl, Acetyl, Propionyl, Butyryl, Valeryl, Isovaleryl und dergleichen. Der Ausdruck "Heterocycloalkyl" betrifft in ähnlicher Weise gesättigte, 3-8-gliedrige, vorzugsweise 5- oder 6-gliedrige, cyclische Kohlenwasserstoffreste, in welchen eine oder zwei Methylengruppen durch ein oder zwei Sauerstoff- , Schwefel- oder gegebenenfalls durch Alkyl, Phenylalkyl, Alkylcarbonyl oder Alkylcarbonyloxy substituierte Stickstoffatome ersetzt sind, wie Piperidinyl, Pyrazinyl, N-Benzylpyrazinyl, Morpholinyl, N-Methylpiperidinyl, N-Benzylmorpholinyl und dergleichen. Der Ausdruck "Cycloalkenyl" betrifft einen ungesättigten Kohlenwasserstoffrest mit 3-8, vorzugsweise 3-6, Kohlenstoffatomen, wie 1-Cyclohexenyl, 1,4-Cyclohexadienyl und dergleichen. Der Ausdruck "Heterocycloalkenyl" betrifft in ähnlicher Weise ungesättigte, 3-8-gliedrige, vorzugsweise 5- oder 6-gliedrige, cyclische Kohlenwasserstoffreste, in welchen eine oder zwei Methylengruppen durch ein oder zwei Sauerstoff-, Schwefel- oder gegebenenfalls durch Alkyl, Phenylalkyl, Alkylcarbonyl oder Alkylcarbonyloxy substituierte Stickstoffatome ersetzt sind, wie Dihydropyranyl, Dihydropyridyl, Dihydrothienyl und dergleichen. Der Ausdruck "Aryl" bezeichnet einen gegebenenfalls durch Alkyl, Alkoxy, Alkylcarbonyloxy, Amino, Alkylamino, Dialkylamino, Alkylcarbonylamino, Hydroxy, Halogen, Trifluormethyl oder Nitro ein- oder mehrfach substituierten mono- oder bicyclischen aromatischen Kohlenwasserstoffrest mit 6-14 Kohlenstoffatomen, wie Phenyl, α- oder β-Naphthyl, Indenyl, Anthryl oder Phenanthryl und dergleichen. Der Ausdruck "Heteroaryl" bezeichnet einen gegebenenfalls an einem Stockstoffatom durch Alkyl, Phenyl oder Phenylalkyl und/oder an einem oder mehreren Kohlenstoffatomen durch Alkyl, Phenyl, Phenylalkyl, Halogen, Hydroxy, Alkoxy, Phenyalkoxy oder Oxo substituierten und teilweise gesättigten mono- oder bicyclischen aromatischen Kohlenwasserstoffrest, in welchem ein oder mehrere Kohlenstoffatome durch ein bis zwei Stickstoffatome und/oder ein Sauerstoff- oder Schwefelatom ersetzt sind, wie Pyrrolyl, Furyl, Thienyl, Imidazolyl, Pyrazolyl, Oxazolyl, Thiazolyl, Pyridyl, Pyrazinyl, Pyrimidinyl, Indolyl, Chinolyl, Isochinolyl, Chinoxalinyl, β-Carbolinyl oder ein benzanneliertes cyclopenta-, cyclohexa- oder cyclohepta-anneliertes Derivat davon, z.B. 2- oder 3-Pyrrolyl, Phenylpyrrolyl, z.B. 4- oder 5-Phenyl-2-pyrrolyl, 2-Furyl, 2-Thienyl, 2-Imidazolyl, 2-, 3- oder 4-Pyridyl, 2-, 3- oder 5-Indolyl, substituiertes 2-Indolyl, zum Beispiel 1-Methyl-, 5-Methyl-, 5-Methoxy-, 5-Benzyloxy-, 5-Chlor- oder 4,5-Dimethyl-2-indolyl, 1-Benzyl-2-indolyl, 1-Benzyl-3-indolyl, 4,5,6,7-Tetrahydro-2-indolyl, Cyclohepta[b]-5-pyrrolyl, 2-, 3- oder 4-Chinolyl, 4-Hydroxy-2-chinolyl, 1-, 3- oder 4-Isochinolyl, 1-Oxo-1,2-dihydro-3-isochinolyl, 2-Chinoxalinyl, 2-Benzofuranyl, 2-Benzoxazolyl, 2-Benzthiazolyl, Benz[e]indol-2-yl, β-Carbolin-3-yl und dergleichen. Der Ausdruck "Arylalkyl" bezeichnet geradkettige oder verzweigte Alkylgruppen, worin ein oder mehrere Wasserstoffatome durch Arylgruppen ersetzt sind, wie Benzyl, Diphenylmethyl, Trityl, α- oder β-Naphthylmethyl, 2-Phenyläthyl, 3-Phenyl-2-propyl, 4-Phenyl-3-butyl, 2-(α- oder β-Naphthyl)äthyl, 3-α-Naphthyl-2-propyl, 4-α-Naphthyl-3-butyl und der gleichen, wobei der aromatische Rest jeweils wie oben angegeben ein- oder mehrfach substituiert sein

kann. Der Ausdruck "substituiertes Phenyl" bezeichnet gegebenenfalls durch Alkyl, Alkoxy, Alkoxycarbonyl, Alkylcarbonyloxy, Hydroxy, Halogen oder Trifluormethyl ein- oder mehrfach substituiertes Phenyl, wie 4-Hydroxyphenyl, 4-Methoxyphenyl, 4-Methylphenyl, 4-Chlorphenyl und dergleichen. Der Ausdruck "substituiertes Amino" bedeutet eine durch Alkyl, Arylalkyl, Alkylcarbonyl, Alkoxycarbonyl oder Arylalkoxycarbonyl mono- oder di-substituierte oder eine gegebenenfalls durch ein Sauerstoff-, Schwefel- oder gegebenenfalls Alkyl-, Phenylalkyl-, Alkylcarbonyl- oder Alkylcarbonyloxy-substituiertes Stickstoffatom unterbrochene $C_3$-$C_6$-Alkylen disubstituierte Aminogruppe. Der Ausdruck "$C_3$-$C_6$-Alkylen" bezeichnet geradkettige oder verzweigte Reste mit 3-6 Kohlenstoffatomen, wie Trimethylen, Propylen, Tetramethylen, Pentamethylen, Hexamethylen und dergleichen. Der Ausdruck "Acyl" betrifft die Acylgruppe einer Carbonsäure, einer gegebenenfalls N-substituierten Carbaminsäure, einer Sulfonsäure oder einer gegebenenfalls N-substituierten Amidosulfonsäure, insbesondere solche mit den Teilformeln $R^a$-CO-, ($R^a$)($R^a$)N-CO-, $R^a$-$SO_2$-, oder ($R^a$)($R^a$)N-$SO_2$-, worin $R^a$ Wasserstoff, einen unsubstituierten oder substituierten, gesättigten, gegebenenfalls mit Amino, Monoalkylamino, Dialkylamino, Alkanoylamino oder Alkanoylamino funktionalisierten aliphatischen, cycloaliphatischen, cycloaliphatischen-aliphatischen Kohlenwasserstoffrest mit bis zu 10, vorzugsweise 6, Kohlenstoffatomen, einen unsubstituierten oder substituierten aromatischen, heteroaromatischen, aromatisch-aliphatischen oder heteroaromatisch-aliphatischen Kohlenwasserstoffrest mit bis zu 18, vorzugsweise 10, Kohlenstoffatomen oder einen unsubstituierten oder substituierten, gesättigten 5- oder 6-gliedrigen Heterocyclus bedeutet. Der Ausdruck "O-Schutzgruppe" bedeutet eine mit Base oder vorzugsweise mit Säure abspaltbare Schutzgruppe, wie der Tetrahydropyranyl- oder Methoxymethylrest, ein Alkylcarbonyloxymethyl- oder Alkoxycarbonyl oxymethylrest und dergleichen. Beispiele von "cyclischen O-Schutzgruppen" sind Acetale und Ketale, wie das Ketal von Aceton oder das Acetal von Pivalinaldehyd oder Benzaldehyd.

Ein unsubstituierter oder substituierter, gesättigter, aliphatischer, cycloaliphatischer oder cycloaliphatisch-aliphatischer Kohlenwasserstoffrest $R^a$ ist beispielsweise unsubstituiertes oder substituiertes Alkyl, Mono-, Bi- oder Tricycloalkyl oder Cycloalkylalkyl. "Substituiertes Alkyl" bedeuten einen Alkylrest, in welchem ein oder mehrere Wasserstoffatome durch Hydroxy, Alkoxy, Alkylcarbonyloxy, Halogen, Amino oder Oxo substituiert sein können, wobei die Substituenten nur dann in 1-Stellung des Alkylrestes stehen, wenn dieser in der Teilformel $R^a$-CO- vorhanden ist.

Beispiele von substituiertem Alkyl sind 2-Hydroxyäthyl, Methoxymethyl, 2-Methoxyäthyl, Acetoxymethyl, 2-Acetoxyäthyl, Chlormethyl, Brommethyl, 2-Chlor- oder 2-Bromäthyl, 2-Oxopropyl, 2-Oxobutyl.

Der Ausdruck "Bicycloalkyl" betrifft bicyclische gesättigte Kohlenwasserstoffreste mit 5-10, vorzugsweise 6-9, Kohlenstoffatomen, wie Bicyclo- [3.1.0]hex-1-yl, Bicyclo[3.1.0]hex-2-yl, Bicyclo[3.1.0]hex-3-yl, Bicyclo[4.1.0]hept-1-yl, Bicyclo[4.1.0]hept-4-yl, Bicyclo[2.2.1]hept-2-yl, Bicylo- [3.2.1]oct-2-yl, Bicyclo[3.3.0]-oct-3-yl, Bicyclo[3.3.1]non-9-yl, α- oder β-Decahydronaphthyl und dergleichen.

Der Ausdruck "Tricycloalkyl" betrifft einen tricyclischen gesättigten Kohlenwasserstoffrest mit 8-10 Kohlenstoffatomen, wie 1-Adamantyl.

Beispiele für Cycloalkylalkyl sind Cyclopropylmethyl, Cyclobutylmethyl, Cyclopentylmethyl, Cyclohexylmethyl und dergleichen.

Die genannten cycloaliphatischen und cycloaliphatisch-aliphatischen Reste können durch die gleichen Substituenten wie Alkyl substituiert sein .

Ein gegebenenfalls substituierter aromatischer oder aromatisch-aliphatischer Kohlenwasserstoffrest ist beispielsweise unsubstituiertes oder substituiertes Aryl oder Arylalkyl.

In einem heteroaromatischen oder heteroaromatisch-aliphatischen Kohlenwasserstoffrest ist der Heterocyclus mono-, bi- oder tricyclisch und enthält ein bis zwei Stickstoffatome und/oder ein Sauerstoff- oder Schwefelatom und ist mit einem seiner Ring-Kohlenstoffatome mit der Gruppe -CO-, >N-CO-, -$SO_2$ oder >N-$SO_2$- verknüpft. Beispiele solcher heteroaromatischer Kohlenwasserstoffreste sind Pyrrolyl, Furyl, Thienyl, Imidazolyl, Pyrazolyl, Oxazolyl, Thiazolyl, Pyridyl, Pyrazinyl, Pyrimidinyl, Indolyl, Chinolyl, Isochinolyl, Chinoxalinyl, β-Carbolinyl oder ein benzanneliertes cyclopenta-, cyclohexa- oder cyclohepta-anneliertes Derivat dieser Reste. Der heteroaromatische Rest kann an einem Stickstoffatom durch Alkyl, Phenyl oder Phenylalkyl, z.B. Benzyl, und/oder an einem oder mehreren Kohlenstoffatomen durch Alkyl, Phenyl, Phenylalkyl, Halogen, Hydroxy, Alkoxy, Phenylalkoxy oder Oxo substituiert und teilweise gesättigt sein. Beispiele solcher heteroaromatischer Reste sind 2- oder 3-Pyrrolyl, Phenylpyrrolyl, z.B. 4- oder 5-Phenyl-2-pyrrolyl, 2-Furyl, 2-Thienyl, 2-Imidazolyl, 2-, 3- oder 4-Pyridyl, 2-, 3- oder 5-Indolyl, substituiertes 2-Indolyl, zum Beispiel 1-Methyl-, 5-Methyl-, 5-Methoxy-, 5-Benzyloxy-, 5-Chlor- oder 4,5-Dimethyl-2-indolyl, 1-Benzyl-2-indolyl, 1-Benzyl-3-indolyl, 4,5,6,7-Tetrahydro-2-indolyl, Cyclohepta[b]-5-pyrrolyl, 2-, 3- oder 4-Chinolyl, 4-Hydroxy-2-chinolyl, 1-, 3- oder 4-Isochinolyl, 1-Oxo-1,2-dihyro-3-isochinolyl, 2-Chinoxalinyl, 2-Benzofuranyl, 2-Benzoxazolyl, 2-Benzthiazolyl, Benz[e]indol-2-yl, β-Carbolin-3-yl und dergleichen.

Beispiele heteroaromatisch-aliphatischer Kohlenwasserstoffreste sind 2- oder 3-Pyrrolylmethyl, 2-, 3-

oder 4-Pyridylmethyl, 2-(2-, 3- oder 4-Pyridyl)äthyl, 4-Imidazolylmethyl, 2-(4-Imidazolyl)äthyl, 2-Indolylmethyl, 3-Indolylmethyl, 2-(3-Indolyl)äthyl, 2-Chinolylmethyl und dergleichen.

Ein gesättigter 5- oder 6-gliedriger Heterocyclus hat mindestens ein Kohlenstoffatom, 1-3 Stickstoffatome und gegebenenfalls ein Sauerstoff- oder Schwefelatom als Ringglieder und ist mit einem seiner Ringkohlenstoffatome mit der Gruppe -CO-, >N-CO-, $-SO_2-$ oder $>N-SO_2-$ verknüpft. Der Heterocyclus kann an einem seiner Kohlenstoffatome oder an einem Ringstickstoffatom durch Alkyl, z.B. Methyl oder Aethyl, Phenyl oder Phenylalkyl, z.B. Benzyl, oder an einem seiner Kohlenstoffatome durch Hydroxy oder Oxo substituiert und/oder an zwei benachbarten Kohlenstoffatomen benzanneliert sein. Beispiele solcher Heterocyclen sind Pyrrolidin-3-yl, 4-Hydroxypyrrolidin-2-yl, 5-Oxopyrrolidin-2-yl, Piperidin-2-yl, Piperidin-3-yl, 1-Methylpiperidin-2-yl, 1-Methylpiperidin-3-yl, 1-Methylpiperidin-4-yl, Morpholin-2-yl, Morpholin-3-yl, Thiomorpholin-2-yl, Thiomorpholin-3-yl, 1,4-Dimethylpiperazin-2-yl, 2-Indolinyl, 3-Indolinyl, 1,2,3,4-Tetrahydrochinol-2-, -3- oder -4-yl, 1,2,3,4-Tetrahydroisochinol-1-, -3- oder -4-yl, 1-Oxo-1,2,3,4-tetrahydroisochinol-3-yl und dergleichen.

Der Ausdruck "pharmazeutisch verwendbare Salze" umfasst Salze mit anorganischen oder organischen Säuren, wie Salzsäure, Bromwasserstoffsäure, Salpetersäure, Schwefelsäure, Phosphorsäure, Zitronensäure, Ameisensäure, Maleinsäure, Essigsäure, Bernsteinsäure, Weinsäure, Methansulfonsäure, p-Toluolsulfonsäure und dergleichen. Solche Salze können im Hinblick auf den Stand der Technik und unter Berücksichtigung der Natur der in ein Salz zu überführenden Verbindung durch jeden Fachmann ohne weiteres hergestellt werden.

Die Verbindungen der Formel I besitzen mindestens drei asymmetrische Kohlenstoffatome und liegen deshalb in Form von optisch reinen Diastereomeren, Diastereomerengemischen, diastereomeren Racematen oder Gemischen von diastereomeren Racematen vor. Die vorliegende Erfindung umfasst alle Formen. Diastereomerengemische, diastereomere Racemate oder Gemische von diastereomeren Racematen können nach üblichen Methoden aufgetrennt werden, z.B. durch Säulenchromatographie, Dünnschichtchromatographie, HPLC und dergleichen.

Bevorzugt sind solche Verbindungen der Formel I, worin $R^1$ Wasserstoff bedeutet. $R^2$ bedeutet vorzugsweise Imidazol-2-yl, Imidazol-4-yl oder Thiazol-4-yl, besonders bevorzugt Imidazol-4-yl. Weiter sind solche Verbindungen der Formel I bevorzugt, worin $R^3$ Cyclohexylmethyl bedeutet. $R^4$ und $R^5$ bedeuten unabhängig voneinander vorzugsweise je Wasserstoff oder gegebenenfalls durch Methoxy einfach substituiertes Alkanoyl oder zusammen das Acetal von Pivalinaldehyd, besonders bevorzugt je Wasserstoff. Auch bevorzugt sind solche Verbindungen der Formel I, worin $R^6$ die Gruppe (b) bedeutet. Ebenfalls bevorzugt sind die Verbindungen der Formel I, worin A die Gruppe (c) bedeutet. Die bevorzugte Bedeutung von $R^9$ ist Wasserstoff. $R^{10}$ und $R^{11}$ bedeuten vorzugsweise je Alkyl oder zusammen mit dem Kohlenstoffatom, an das sie gebunden sind, Cycloalkyl, besonders bevorzugt Methyl, Aethyl, Cyclopropyl oder Cyclobutyl. $R^{12}$ bedeutet vorzugsweise Phenyl oder substituiertes Phenyl, besonders bevorzugt Phenyl. Die bevorzugte Bedeutung von $R^{13}$ ist Alkylcarbonylalkyl oder Alkylsulfonylalkyl, vorzugsweise $C_1$-$C_4$-Alkylcarbonylmethyl oder $C_1$-$C_4$-Alkylsulfonylmethyl. Falls A die Gruppe (d) bedeutet, so sind diejenigen Verbindungen der Formel I bevorzugt, worin Y den N-terminal mit Z verbundenen, bivalenten Rest von Phenylalanin bedeutet. Z bedeutet vorzugsweise die Gruppe $R^a$-CO-, worin $R^a$ einen gegebenenfalls substituierten, gesättigten aliphatischen Kohlenwasserstoffrest mit bis zu 10 Kohlenstoffatomen oder einen gegebenenfalls substituierten heteraromatischen Kohlenwasserstoffrest mit bis zu 18 Kohlenstoffatom bedeutet, ganz besonders bevorzugt die Gruppe $R^a$-CO-, worin $R^a$ einen gesättigten, aliphatischen Kohlenwasserstoffrest mit bis zu 6 Kohlenstoffatomen oder einen heteroaromatischen Rest mit bis 10 Kohlenstoffatomen bedeutet.

Aus dem obigen folgt, dass solche Verbindungen der Formel I ganz besonders bevorzugt sind, worin $R^1$ Wasserstoff, $R^2$ Imidazol-4-yl, $R^3$ Cyclohexylmethyl, $R^4$ und $R^5$ je Wasserstoff, $R^6$ die Gruppe (b), $R^9$ Wasserstoff, $R^{10}$ und $R^{11}$ je Methyl oder Aethyl oder zusammen mit dem Kohlenstoffatom, an das sie gebunden sind, Cyclopropyl oder Cyclobutyl, $R^{12}$ Phenyl und $R^{13}$ $C_1$-$C_4$-Alkylcarbonylmethyl oder $C_1$-$C_4$-Alkylsulfonylmethyl bedeuten.

Besondere Verbindungen der Formel I sind solche, worin $R^1$ Wasserstoff, $R^2$ Propyl, Imidazol-2-yl, Imidazol-4-yl, Thiazol-4-yl, Thien-2-yl oder t-Butoxy, $R^3$ Cyclohexylmethyl, $R^4$ und $R^5$ unabhängig voneinander je Wasserstoff, gegebenenfalls durch Dialkylamino, Carboxy oder Alkoxy einfach substituiertes Alkanoyl oder zusammen eine cyclische O-Schutzgruppe, $R^6$ die Gruppe (a) oder (b), A die Gruppe (c) oder (d), D eine Methingruppe, $R^7$ Alkyl, $R^8$ Wasserstoff oder Alkyl, oder $R^7$ und $R^8$ zusammen mit den beiden sie verbindenden Atomen, Aryl, Heteroaryl oder Cycloalkenyl, $R^9$ Wasserstoff oder Alkyl, $R^{10}$ und $R^{11}$ unabhängig voneinander je Alkyl oder die Gruppe (e) oder zusammen mit den Kohlenstoffatomen, an das sie gebunden sind, Cycloalkyl oder Heterocycloalkyl, $R^{12}$ Phenyl, substituiertes Phenyl, Benzyl oder Naphthyl, $R^{13}$ Alkylcarbonylalkyl, Heterocycloalkylcarbonylalkyl, Alkylcarbonylhydroxyalkyl, Dialkoxyphosphoroxyalkyl, Arylalkyl, Alkylsulfinylalkyl oder Alkylsulfonylalkyl, Y den N-terminal mit Z verbundenen, bivalenten Rest von

Phenylalanin, Z Acyl, X ein Sauerstoffatom und $R^{14}$ Wasserstoff oder Arylalkyl bedeuten.

Ganz besondere Verbindungen der Formel I sind diejenigen, worin $R^1$ Wasserstoff, $R^2$ Imidazol-2-yl, Imidazol-4-yl oder Thiazol-4-yl, besonders bevorzugt Imidazol-4-yl, $R^3$ Cyclohexylmethyl, $R^4$ und $R^5$ unabhängig voneinander je Wasserstoff, gegebenenfalls durch Methoxy einfach substituiertes Alkanoyl oder zusammen das Acetal von Pivalinaldehyd, besonders bevorzugt je Wasserstoff, $R^6$ die Gruppe (b), A die Gruppe (c), $R^9$ Wasserstoff, $R^{10}$ und $R^{11}$ unabhängig voneinander je Alkyl oder zusammen mit dem Kohlenstoffatom, an das sie gebunden sind, Cycloalkyl, besonders bevorzugt Methyl, Aethyl, Cyclopropyl oder Cyclobutyl, $R^{12}$ Phenyl oder substituiertes Phenyl, besonders bevorzugt Phenyl, und $R^{13}$ Alkylcarbonylalkyl oder Alkylsulfonylalkyl, besonders bevorzugt $C_1$-$C_4$-Alkylcarbonylmethyl oder $C_1$-$C_4$-Alkylsulfonylmethyl, bedeuten.

Speziell bevorzugte Verbindungen der Formel I sind:

- (S)-N-[(1S,2R,3RS)-1-(Cyclohexylmethyl)-4-äthyl-2,3-dihydroxyhexyl]-α-[(R)-α-(3,3-dimethyl-2-oxobutyl)-hydrocinnamamido]imidazol-4-propionamid;
- (S)-N-[(1S,2R,3S)-3-Cyclohexyl-1-(cyclohexylmethyl)-2,3-dihydroxypropyl]-α-[(R)-α-(3,3-dimethyl-2-oxobutyl)hydrocinnamamido]imidazol-4-propionamid;
- (S)-N-[(1S,2R,3S)-1-(Cyclohexylmethyl)-2,3-dihydroxy-4-methylpentyl]-α-[(R)-α-(3,3-dimethyl-2-oxobutyl)-hydrocinnamamido]imidazol-4-propionamid;
- (S)-α-[(S)-α-[(t-Butylsulfonyl)methyl]hydrocinnamamido]-N-[(1S,2R,3S)-1-(cyclohexylmethyl)-2,3-dihydroxy-3-phenylpropyl]imidazol-4-propionamid;
- (S)-α-[(S)-α-[(t-Butylsulfonyl)methyl]hydrocinnamamido]-N-[(1S,2R,3R)-1-(cyclohexylmethyl)-2,3-dihydroxy-3-phenylpropyl]imidazol-4-propionamid;
- (S)-α-[(S)-α-[(t-Butylsulfonyl)methyl]hydrocinnamamido]-N-[(1S,2R,3RS)-1-(cyclohexylmethyl)-2,3-dihydroxy-4,4-dimethylpentyl]imidazol-4-propionamid;
- (S)-α-[(S)-α-[(t-Butylsulfonyl)methyl]hydrocinnamamido]-N-[(1S,2R,3S)-1-(cyclohexylmethyl)-2,3-dihydroxy-4-methylpentyl]imidazol-4-propionamid;
- (S)-α-[(S)-α-[(t-Butylsulfonyl)methyl]hydrocinnamamido]-N-[(1S,2R,3S)-3-cyclohexyl-1-(cyclohexylmethyl)-2,3-dihydroxypropyl]imidazol-4-propionamid.

Weitere speziell bevorzugte Verbindungen der Formel I sind:

- (S)-α-[(S)-α-[(t-Butylsulfonyl)methyl]hydrocinnamamido]-N-[(1S,2R,3RS)-1-(cyclohexylmethyl)-2,3-dihydroxy-3-(2-furyl)propyl]imidazol-4-propionamid;
- (S)-N-[(1S,2R,3R oder S)-1-(Cyclohexylmethyl)-2,3-dihydroxy-3-[(R oder S)-tetrahydro-2-furyl]propyl]-α-[-(R)-α-(3,3-dimethyl-2-oxobutyl)hydrocinnamamido]imidazol-4-propionamid;
- (S)-α-[(S)-α-[(t-Butylsulfonyl)methyl]hydrocinnamamido]-N-[(1S,2R,3R oder S,Z)-1-(cyclohexylmethyl)-2,3-dihydroxy-4-methyl-4-hexenyl]imidazol-4-propionamid.

Noch weitere speziell bevorzugte Verbinungen der Formel I sind:

- (S)-N-[(1S,2R,3S)-1-(Cyclohexylmethyl)-3-cyclopropyl-2,3-dihydroxypropyl]-α-[(S)-α-[(morpholinocarbonyl)-methyl]hydrocinnamamido]imidazol-4-propionamid;
- (S)-α-[(S)-α-[(t-Butylsulfonyl)methyl]-1-naphthalinpropionamido]-N-[(1S,2R,3S)-1-(cyclohexylmethyl)-3-cyclopropyl-2,3-dihydroxypropyl]imidazol-4-propionamid;
- (S)-α-[(S)-2-[(t-Butylsulfonyl)methyl]-4-phenylbutyramido]-N-[(1S,2R,3S)-1-(cyclohexylmethyl)-3-cyclopropyl-2,3-dihydroxypropyl]imidazol-4-propionamid;
- (S)-α-[(R)-α-[(t-Butylsulfonyl)methyl]hydrocinnamamido]-N-[(1S,2R,3S)-1-(cyclohexylmethyl)-3-cyclopropyl-2,3-dihydroxypropyl]imidazol-4-propionamid;
- (S)-N-[(S)-1-[(2R oder S,4R,5S)-2-t-Butyl-5-isopropyl-1,3-dioxolan-4-yl]-2-cyclohexyläthyl]-α-[(S)-α-[(t-butylsulfonyl)methyl]hydrocinnamamido]imidazol-4-propionamid;
- (1R,2S)-1-[(S)-1-[[N-[(S)-α-[(t-Butylsulfonyl)methyl]cinnamoyl]-L-histidyl]amino]-2-cyclohexyläthyl-2-cyclopropyläthylen-bis(methoxyacetate).

Ganz speziell bevorzugte Verbindungen der Formel I sind:

- (S)-N-[(1S,2R,3S)-1-(Cyclohexylmethyl)-3-cyclopropyl-2,3-dihydroxypropyl]-α-[(R)-α-(3,3-dimethyl-2-oxobutyl)hydrocinnamamido]imidazol-4-propionamid;
- (S)-α-[(S)-α-[(t-Butylsulfonyl)methyl]hydrocinnamamido]-N-[(1S,2R,3S)-1-(cyclohexylmethyl)-3-cyclopropyl-2,3-dihydroxypropyl]imidazol-4-propionamid;
- (S)-α-[(S)-α-[(t-Butylsulfonyl)methyl]hydrocinnamamido]-N-[(1S,2R,3S)-3-cyclobutyl-1-(cyclohexylmethyl)-2,3-dihydroxypropyl)imidazol-4-propionamid;
- (S)-N-[(S)-1-[(4R,5S)-2-t-Butyl-5-cyclopropyl-1,3-dioxolan-4-yl]-2-cyclohexyläthyl]-α-[(S)-α-[(t-butylsulfonyl)-methyl]hydrocinnamamido]imidazol-4-propionamid;
- (1S,2R)-[(S)-1-[(S)-α-[(S)-α-[(t-Butylsulfonyl)methyl]hydrocinnamamido]imidazol-4-propionamido]-2-cyclohexyläthyl]-1-isopropyläthylen-bis(methoxyacetat);

- (S)-α-[(S)-α-[(t-Butylsulfonyl)methyl]hydrocinnamamido]-N-[(1S,2R,3S)-1-(cyclohexylmethyl)-3-cyclopentyl-2,3-dihydroxypropyl]imidazol-4-propionamid;
- (S)-α-[(S)-α-[(t-Butylsulfonyl)methyl]hydrocinnamamido]-N-[(1S,2R,3S)-1-(cyclohexylmethyl)-3-cyclopropyl-2,3-dihydroxypropyl]-4-thiazolpropionamid;
- (S)-α-[(S)-α-[(t-Butylsulfonyl)methyl]hydrocinnamamido]-N-[(1S,2S oder R,3S oder R,4SR)-1-(cyclohexylmethyl)-2,3,5-trihydroxypentyl]imidazol-4-propionamid.

Die Verbindungen der Formel I in Form von optisch reinen Diastereomeren, Diastereomerengemischen, Diastereomeren Racematen oder Gemischen oder diastereomeren Racematen sowie pharmazeutisch verwendbare Salze davon können hergestellt werden, indem man

a) eine Verbindung der allgemeinen Formel

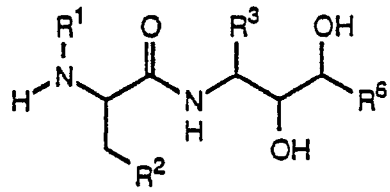

II

worin $R^1$, $R^2$, $R^3$ und $R^6$ die oben angegebene Bedeutung besitzen, mit einem die Gruppe

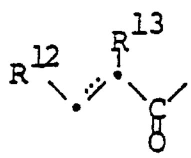

(c) oder -Y-Z (d)

worin $R^{12}$, $R^{13}$, Y, Z und die gestrichelte Linie die oben angegebene Bedeutung besitzen, abgebenden Acylierungsmittel umsetzt, oder

b) eine Verbindung der allgemeinen Formel

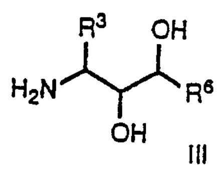

III

worin $R^3$ und $R^6$ die oben angegebene Bedeutung besitzen, mit einer Verbindung der allgemeinen Formel

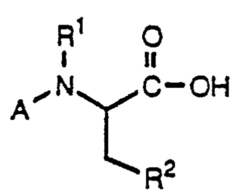

IV

worin $R^1$, $R^2$ und A die oben angegebene Bedeutung besitzen, oder einem aktivierten Derivat davon umsetzt, oder

c) eine Verbindung der Formel I, worin $R^4$ und $R^6$ je Wasserstoff bedeuten und die übrigen Symbole die oben angegebene Bedeutung besitzen, mit einem gegebenenfalls durch Amino, Monoalkylamino, Dialkylamino, Alkanoylamino, Alkanoyloxyamino, Carboxy, Alkoxy oder Hydroxy ein- oder mehrfach substituierten Alkanoylierungsmittel oder mit einem eine O-Schutzgruppen bildenden Mittel umsetzt, und

d) erwünschtenfalls ein Gemisch diastereomerer Racemate in die diastereomeren Racemate oder optisch reinen Diastereomeren auftrennt, und/oder

e) erwünschtenfalls ein Diastereomerengemisch in die optisch reinen Diastereomeren auftrennt, und/oder

f) erwünschtenfalls eine erhaltene Verbindung in ein pharmazeutisch verwendbares Salz überführt.

Die Acylierung einer Verbindung der Formel II erfolgt nach an sich bekannten Methoden. Geeignete Acylierungsmittel sind insbesondere aktivierte Säurederivate, wie Ester, gemischte Ester, Säurehalogenide und Säureanhydride oder gemischte Säureanhydride. Die Reaktion wird in einem unter den Reaktionsbedingungen inerten organischen Lösungsmittel oder Lösungsmittelgemisch bei einer Temperatur zwischen etwa 0 °C und der Raumtemperatur durchgeführt. Als Lösungsmittel kommen insbesondere aromatische Kohlenwasserstoffe, wie Benzol, Toluol oder Xylol, chlorierte Kohlenwasserstoffe, wie Methylenchlorid oder Chloroform, Aether, wie Diäthyläther, Tetrahydrofuran oder Dioxan, und dergleichen in Frage. Falls es sich beim Acylierungsmittel um ein Dipeptid handelt, erfolgt die Reaktion unter in der Peptidchemie üblichen Reaktionsbedingungen, d.h. vorzugsweise in Gegenwart eines Kondensationsmittels wie HBTU (O-Benzotriazolyl-N,N,N′,N′-tetramethyluronium-hexafluorophosphat), BOP (Benzotriazol-1-yloxy-bis-(dimethylamino)phosphonium-hexafluorophosphat), HOBT (N-Hydroxybenzotriazol), DBU (1,8-Diazabicyclo-[5.4.0]undec-7-en), DCC (Dicyclohexylcarbodiimid), EDC (N-Aethyl-N′(3-dimethylaminopropyl)carbodiimid-hydrochlorid), Hünigbase (Aethyldiisopropylamin), und dergleichen. Die Reaktion wird zweckmässigerweise in einem unter den Reaktionsbedingungen inerten organischen Lösungsmittel oder Lösungsmittelgemisch bei einer Temperatur zwischen etwa 0° und 50 °C, vorzugsweise bei etwa Raumtemperatur durchgeführt. Als Lösungsmittel kommen insbesondere Dimethylformamid, Methylenchlorid, Acetonitril, Tetrahydrofuran, und dergleichen in Frage.

Die Umsetzung einer Verbindung der Formel III mit einer Verbindung der Formel IV erfolgt ebenfalls nach an sich in der Peptid-Chemie bekannten Methoden, d.h. unter den gleichen Bedingungen wie oben für die Umsetzung einer Verbindung der Formel II mit einem Dipeptid angegeben wurde. Beispiele geeigneter aktivierter Derivate einer Verbindung der Formel IV sind Säurehalogenide, Säureanhydride, Säure azide, gemischte Anhydride, Ester, gemischte Ester, und dergleichen.

Die Umsetzung einer Verbindung der Formel I, worin $R^4$ und $R^5$ je Wasserstoff bedeuten, mit einem Alkanoylierungsmittel erfolgt ebenfalls nach an sich bekannten Methoden. Geeignete Alkanoylierungsmittel sind Alkansäureanhydride und -halogenide, vorzugsweise -chloride. Die Umsetzung erfolgt in einem unter den Reaktionsbedingungen inerten organischen Lösungsmittel oder Lösungsmittelgemisch bei einer Temperatur zwischen etwa Raumtemperatur und der Rückflusstemperatur des Reaktionsgemisches, vorzugsweise bei etwa Raumtemperatur. Die Reaktion kann in Gegenwart oder Abwesenheit eines Säurebindemittels, wie Natrium- oder Kaliumcarbonat, Pyridin, Triäthylamin und dergleichen durchgeführt werden. Die Umsetzung einer Verbindung der Formel I, worin $R^4$ und $R^5$ je Wasserstoff bedeuten, mit einem eine O-Schutzgruppe bildenden Mittel erfolgt ebenfalls in an sich bekannter Weise. So können beispielsweise die Tetrahydropyranyläther durch Umsetzen mit Dihydropyran in Gegenwart eines Säurekatalysators, wie p-Toluolsulfonsäure und dergleichen, und das Acetonketal durch Umsetzen mit 2,2-Dimethoxypropan in Gegenwart eines Säurekatalysators, wie p-Toluolsulfonsäure, hergestellt werden.

Die Ausgangsstoffe der Formel II sind neu und ebenfalls Gegenstand der vorliegenden Erfindung. Diese Verbindungen können hergestellt werden, indem man eine Verbindung der Formel III mit gegebenenfalls N-methyliertem Histidin, Leucin, Norleucin, Norvalin, Thiazolylalanin, Thienylalanin oder t-Butoxyserin umsetzt. Diese Umsetzung erfolgt ebenfalls nach in der Peptid-Chemie bekannten Methoden, d.h. unter den Reaktionsbedingungen, wie sie weiter oben für die Umsetzung einer Verbindung der Formel II mit einem Dipeptid beschrieben sind.

Die Ausgangsstoffe der Formel III sind ebenfalls neu und Gegenstand der vorliegenden Erfindung. Sie können beispielsweise hergestellt werden, indem man in einer Verbindung der allgemeinen Formel

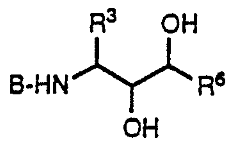

V

oder

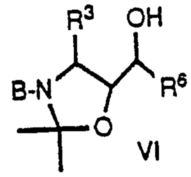

VI

worin B eine Aminoschutzgruppe bedeutet, vorzugsweise t-Butoxycarbonyl oder Benzyloxycarbonyl, und $R^3$ und $R^6$ die oben angegebene Bedeutung besitzen,
die Aminoschutzgruppe und gegebenenfalls gleichzeitig auch die O-Schutzgruppe abspaltet oder indem man eine Verbindung der allgemeinen Formel

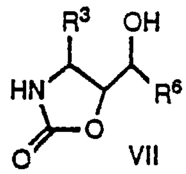

VII

worin $R^3$ und $R^6$ die oben angegebene Bedeutung besitzen,
mit einer Base behandelt.

Die Abspaltung der N-Schutzgruppe und gegebenenfalls O-Schutzgruppe erfolgt ebenfalls nach an sich bekannten Methoden, beispielsweise in einem unter den Reaktionsbedingungen inerten organischen Lösungsmittel oder Lösungsmittelgemisch bei einer Temperatur zwischen etwa 0° C und der Raumtemperatur mit einer Säure, wie Chlorwasserstoffsäure, Trifluoressigsäure, und dergleichen. Geeignete Lösungsmittel sind Aether, wie Tetrahydrofuran oder Dioxan, Alkohole, wie Methanol, oder chlorierte Kohlenwasserstoffe, wie Methylenchlorid, und dergleichen. Unter diesen Reaktionsbedingungen wird in einer Verbindung der Formel VI - wie bereits er wähnt - gleichzeitig der Oxazolidinring aufgespalten.

Die Umsetzung einer Verbindung der Formel VII mit einer Base erfolgt ebenfalls nach an sich bekannten Methoden in einem unter den Reaktionsbedingungen inerten Lösungsmittel oder Lösungsmittelgemisch bei einer Temperatur zwischen etwa Raumtemperatur und der Rückflusstemperatur. Geeignete Lösungsmittel sind beispielsweise Methanol, Aethanol, Dioxan, Tetrahydrofuran, Wasser oder Gemische davon. Als Basen kommen zweckmässigerweise Natrium-, Kalium- oder Bariumhydroxyd und dergleichen in Frage.

Die Ausgangsstoffe der Formel IV sind bekannt oder können in Analogie zur Herstellung der bekannten Verbindungen erhalten werden.

Die Verbindungen der Formeln V und VI sind ebenfalls neu und Gegenstand vorliegender Erfindung. Sie können beispielsweise durch Reduktion der entsprechenden Ketoverbindungen der allgemeinen Formel

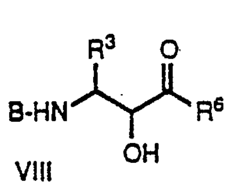

VIII

bzw.

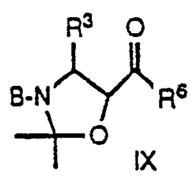

IX

worin B, $R^3$ und $R^6$ die oben angegebene Bedeutung besitzen,
hergestellt werden.

Die Reduktion einer Ketoverbindung der Formel VIII bzw. IX erfolgt ebenfalls nach an sich bekannten Methoden, beispielsweise mit einem komplexen Metallhydrid, wie Natriumborhydrid und dergleichen, in einem unter den Reaktionsbedingungen inerten organischen Lösungsmittel oder Lösungsmittelgemisch bei einer Temperatur zwischen etwa 0° C und etwa Raumtemperatur.

Die Verbindungen der Formel VII sind ebenfalls neu und Gegenstand vorliegender Erfindung. Sie können beispielsweise durch Umsetzen der entsprechenden Formylverbindungen der allgemeinen Formel

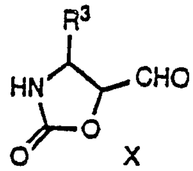

X

worin $R^3$ die oben angegebene Bedeutung besitzt,
mit einer den Rest $R^6$ abgebendenen metallorganischen Verbindung hergestellt werden.

Die Umsetzung mit eine metallorganischen Verbindung erfolgt ebenfalls nach an sich bekannten Methoden, beispielsweise in einem unter den Reaktionsbedingungen inerten Lösungsmittel, wie einem Aether, bei einer Temperatur zwischen etwa -100°C und 50°C in Abhängigkeit von der Natur der eingesetzten metallorganischen Verbindung. Wird eine Lithiumverbindung verwendet, erfolgt die Umsetzung vorzugsweise bei etwa -50°C bis etwa -80°C, während bei Verwendung einer Grignardverbindung die Reaktion vorzugsweise bei etwa Raumtemperatur durchgeführt wird.

Die Verbindungen der Formeln VIII, IX und X sind ebenfalls neu und Gegenstand vorliegender Erfindung. Die Verbindungen der Formel VIII können beispielsweise hergestellt werden; indem man einen Ester bzw. ein Cyanhydrin der allgemeinen Formel

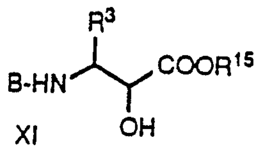

XI

bzw.

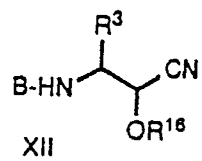

XII

worin B und $R^3$ die oben angegebene Bedeutung besitzen, $R^{15}$ Alkyl und $R^{16}$ vorzugsweise Trimethylsilyl bedeuten,
mit einer Verbindung der allgemeinen Formel
W-Mg-$R^6$ XIII
worin $R^6$ die oben angegebene Bedeutung besitzt und W Chlor, Brom oder Jod, vorzugsweise Brom, bedeutet,
in einer Grignard-Reaktion umsetzt. Diese Umsetzung erfolgt ebenfalls nach an sich bekannten Methoden, beispielsweise in einem unter den Reaktionsbedingungen inerten Lösungsmittel, wie einem Aether, bei einer Temperatur zwischen etwa 0°C und 50°C, vorzugsweise bei Raumtemperatur. Falls eine Verbindung der Formel XII eingesetzt wird, muss das intermediär auftretende Imin mit einer schwachen wässrigen Säure, wie Phosphorsäure, hydrolysiert werden, wobei gleichzeitig die Trimethylsilylgruppe abgespaltet wird.

Die Verbindungen der Formel IX können hergestellt werden, indem man beispielsweise einen Ester der allgemeinen Formel

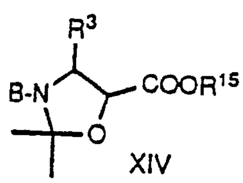

XIV

worin B, $R^3$ und $R^{15}$ die oben angegebene Bedeutung besitzen,
mit einer Lithiumverbindung der allgemeinen Formel
$R^6$-Li XV
worin $R^6$ die oben angegebene Bedeutung besitzt,
umsetzt. Auch diese Umsetzung erfolgt nach an sich bekannten Methoden, beispielsweise in einem unter den Reaktionsbedingungen inerten Lösungsmittel, wie einem Aether, bei einer Temperatur zwischen etwa -100°C und 0°C, vorzugsweise bei etwa -70°C.

Die Verbindungen der Formel X sind ebenfalls neu und Gegenstand vorliegender Erfindung. Sie können beispielsweise hergestellt werden, indem man eine Verbindung der allgemeinen Formel

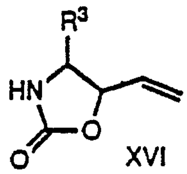

XVI

worin $R^3$ die oben angegebene Bedeutung besitzt,
einer reduktiven Ozonolyse unterwirft. Die Umsetzung mit Ozon erfolgt ebenfalls nach an sich bekannten Methoden, beispielsweise in Methanol oder Methylenchlorid als Lösungsmittel bei einer Temperatur zwischen etwa -100° C und -20° C. Die anschliessende Reduktion der Ozonide erfolgt ebenfalls nach an sich bekannten Methoden, beispielsweise durch Zugabe von Dimethylsulfid bei einer Temperatur zwischen etwa -50° C und der Raumtemperatur.

Die Verbindungen der Formeln XI, XII, XIII, XIV, XV und XVI sind bekannt oder können in Analogie zur Herstellung der bekannten Verbindungen erhalten werden.

Ein weiteres Verfahren zur Herstellung der Verbindungen der Formel V besteht darin, dass man eine Verbindung der allgemeinen Formel

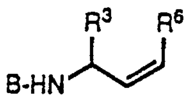

XVII

worin B, $R^3$ un $R^6$ die oben angegebene Bedeutung besitzen,
oxidiert. Auch die Oxidation einer Verbindung der Formel XVII erfolgt nach an sich bekannten Methoden in einem unter den Reaktionsbedingungen inerten organischen Losungsmittel oder Lösungsmittelgemisch bei einer Temperatur zwischen etwa Raumtemperatur und dem Siedepunkt des Lösungsmittels bzw. Lösungsmittelgemisches, vorzugsweise bei etwa Raumtemperatur. Als Oxidationsmittel eignet sich insbesondere Osmiumtetroxid. Als Lösungsmittel kommt insbesondere Pyridin und dergleichen in Frage.

Die Verbindungen der Formel XVII sind ebenfalls neu und Gegenstand vorliegender Erfindung. Sie können nach an sich bekannten Methoden hergestellt werden, beispielsweise durch Umsetzen eines Aldehyds der allgemeinen Formel

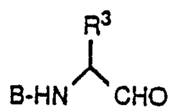

XVIII

worin B und $R^3$ die oben angegebene Bedeutung besitzen,
mit einem entsprechenden Wittig-Reagens. Die Umsetzung wird ebenfalls nach bekannten Methoden durchgeführt, beispielsweise in einem unter den Reaktionsbedingungen inerten Lösungsmittel, wie einem Aether, bei einer Temperatur zwischen etwa -50° C und Raumtemperatur.

Die Verbindungen der Formel XVIII sowie die entsprechenden Wittig-Reagentien sind bekannt oder können in Analogie zur Herstellung der bekannten Verbindungen erhalten werden.

Gemäss einem Alternativverfahren kann eine Verbindung der Formel VI auch so hergestellt werden, dass man eine Verbindung der allgemeinen Formel

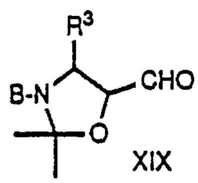

XIX

worin B und $R^3$ die oben angegebene Bedeutung besitzen, in einer Grignard-Reaktion mit einer Verbindung der Formel XIII umsetzt, d.h. unter den gleichen Bedingungen wie oben für die Umsetzung einer Verbindung der Formel XI mit einer Verbindung der Formel XIII angegeben wurde.

Die Verbindungen der Formel XIX sind bekannt oder können in Analogie zu den bekannten Verbindungen hergestellt werden.

Die verschiedenen Verfahren zur Herstellung der Verbindungen der Formeln III, V, VI und VII ausgehend von einer Verbindung der Formel XVIII sind im nachfolgenden Schema I zusammengefasst.

Schema I

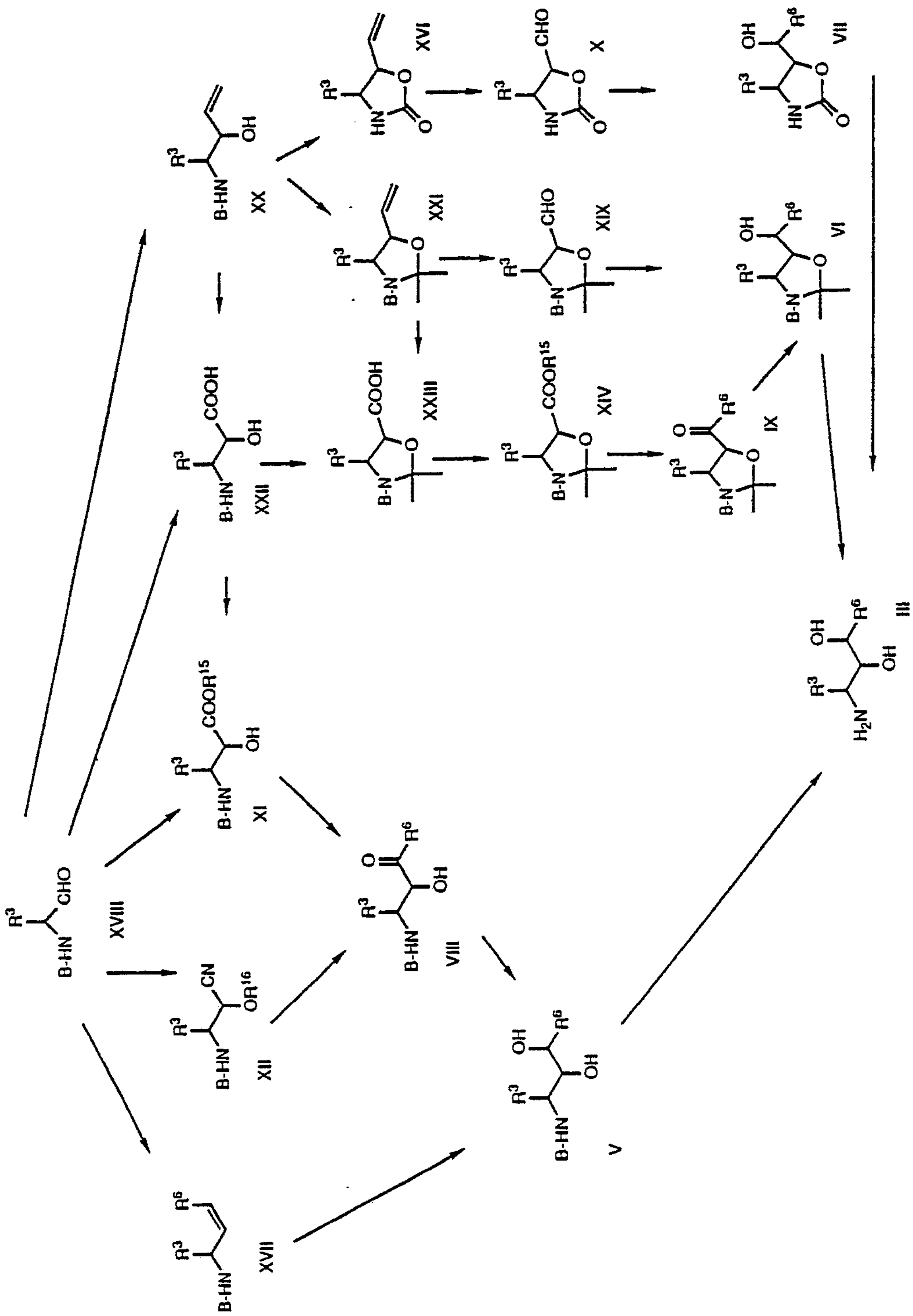
XX XVI X VII XXI XIX VI XXII XXIII XIV IX III XI VIII XVIII XII V XVII

Die Verbindungen der Formel I und ihre pharmazeutisch verwendbaren Salze weisen hemmende Wirkung des natürlichen Enzyms Renin auf. Letzteres gelangt aus den Nieren in das Blut und bewirkt dort die Spaltung von Angiotensinogen unter Bildung des Dekapeptids Angiotensin I, das dann in der Lunge, den Nieren und anderen Organen zum Octapeptid Angiotensin II gespalten wird. Angiotensin II erhöht den Blutdruck sowohl direkt durch arterielle Konstriktion, als auch indirekt durch die Freisetzung des Natriumionen zurückhaltenden Hormons Aldosteron aus den Nebennieren, womit ein Anstieg des extrazellulären Flüssigkeitsvolumens verbunden ist. Dieser Anstieg ist auf die Wirkung von Angiotensin II selber oder des daraus als Spaltprodukt gebildeten Heptapeptids Angiotensin III zurückzuführen. Hemmer der enzymatischen Aktivität von Renin bewirken eine Verringerung der Bildung von Angiotensin I und als Folge davon die Bilding einer geringeren Menge von Aniotensin II. Die verminderte Konzentration dieses aktiven Peptid-Hormons ist die unmittelbare Ursache für die blutdrucksenkende Wirkung von Renin-Hemmern.

Die Wirkung von Renin-Hemmern kann experimentell mittels des nachstehend beschriebenen In vitro-Tests gezeigt werden:

In vitro-Test mit reinem Human-Renin

Der Test wird in Eppendorf Röhrchen durchgeführt. Die Inkubationsmischung besteht aus (1) 100 µl Human-Renin in Puffer A (0,1M Natriumphosphatlösung, pH 7,4, enthaltend 0,1% Rinderserumalbumin, 0,1 % Natriumazid und 1 mM Aethylendiamintetraessigsäure), genügend für eine Renin-Akivität von 2-3 ng Angiotensin I/ ml/Std.; (2) 145 µl Puffer A; (3) 30 µl von 10 µM humanem Tetradekapeptid-Reninsubstrat (hTD) in 10 mM Salzsäure; (4) 15 µl Dimethylsulfoxid mit bzw. ohne Hemmer und (5) 10 µl einer 0,03 molaren Lösung von Hydroxychinolinsulfat in Wasser.

Die Proben werden drei Stunden bei 37° C bzw. 4° C in Triplikaten inkubiert. 2 x 100 µl Proben pro Versuchsröhrchen werden dann verwendet, um die Produktion von Angiotensin I via RIA (standard radioimmunoassay; Clinical Assay solid phase kit) zu messen. Kreuzreaktivitäten der verwendeten Antikörper im RIA sind: Angiotensin I 100 %; Angiotensin II 0,0013 %; hTD (Angiotensin I-Val-Ile-His-Ser-OH) 0,09 %. Die Produktion von Angiotensin I wird durch die Differenz zwischen dem Versuch bei 37° C und demjenigen bei 4° C bestimmt.

Folgende Kontrollen werden mitgeführt:

(a) Inkubation von hTD-Proben ohne Renin und ohne Hemmer bei 37° C und 4° C. Die Differenz zwischen diesen beiden Werten ergibt den Grundwert der Angiotensin I-Produktion.

(b) Inkubation von hTD-Proben mit Renin, jedoch ohne Hemmer bei 37° C und 4° C. Die Differenz zwischen diesen Werten ergibt den Maximalwert der Angiotensin I-Produktion.

In jeder Probe wird von der ermittelten Angiotensin I-Produktion der Grundwert der Angiotensin I-Produktion abgezogen. Die Differenz zwischen dem Maximalwert und dem Grundwert ergibt den Wert der maximalen Substrathydrolyse (=100%) durch Renin.

Die Resultate werden als $IC_{50}$-Werte angegeben, welche diejenige Konzentration des Hemmers bezeichnen, bei welcher die enzymatische Aktivität um 50% gehemmt wird. Die $IC_{50}$-Werte werden aus einer linearen Regressionskurve aus einem logit-log plot ermittelt.

Die in diesem Test erhaltenen Resultate sind in der folgenden Tabelle zusammengefasst:

Tabelle

| Verbindung | $IC_{50}$-Werte in µMol/lt. |
|---|---|
| A | 0,024 |
| B | 0,001 |
| C | 0,003 |
| D | 0,007 |
| E | 0,17 |
| F | 0,038 |
| G | 0,002 |
| H | 0,002 |

A= (S)-N-[(1S,2R,3RS)-1-(Cyclohexylmethyl)-4-äthyl-2,3-dihydroxyhexyl]-α-[(R)-α-(3,3-dimethyl-2-oxobutyl)-hydrocinnamamido]imidazol-4-propionamid;

B= (S)-N-[(1S,2R,3S)-3-Cyclohexyl-1-(cyclohexylmethyl)-2,3-dihydroxypropyl]-α-[(R)-α-(3,3-dimethyl-2-oxobutyl)hydrocinnamamido]imidazol-4-propionamid;

C= (S)-N-[(1S,2R,3S)-1-(Cyclohexylmethyl)-2,3-dihydroxy-4-methylpentyl]-α-[(R)-α-(3,3-dimethyl-2-oxobutyl)-hydrocinnamamido]imidazol-4-propionamid;

D= (S)-α-[(S)-α-[(t-Butylsulfonyl)methyl]hydrocinnamamido]-N-[(1S,2R,3S)-1-(cyclohexylmethyl)-2,3-dihydroxy-3-phenylpropyl]imidazol-4-propionamid;

E= (S)-α-[(S)-α-[(t-Butylsulfonyl)methyl]hydrocinnamamido]-N-[(1S,2R,3R)-1-(cyclohexylmethyl)-2,3-dihydroxy-3-phenylpropyl]imidazol-4-propionamid;

F= (S)-α-[(S)-α-[(t-Butylsulfonyl)methyl]hydrocinnamamido]-N-[(1S,2R,3RS)-1-(cyclohexylmethyl)-2,3-dihydroxy-4,4-dimethylpentyl]imidazol-4-propionamid.

G= (S)-α-[(S)-α-[(t-Butylsulfonyl)methyl]hydrocinnamamido]-N-[(1S,2R,3S)-1-(cyclohexylmethyl)-2,3-dihydroxy-4-methylpentyl]imidazol-4-propionamid;

H= (S)-α-[(S)-α-[(t-Butylsulfonyl)methyl]hydrocinnamamido]-N-[(1S,2R,3S)-3-cyclohexyl-1-(cyclohexylmethyl)-2,3-dihydroxypropyl]imidazol-4-propionamid.

Die Verbindungen der Formel I sowie deren pharmazeutisch verwendbare Salze können als Heilmittel, z.B. in Form pharmazeutischer Präparate,Verwendung finden. Die pharmazeutischen Präparate können enteral, wie oral, z.B. in Form von Tabletten, Lacktabletten, Dragees, Hart- und Weichgelatinekapseln, Lösungen, Emulsionen oder Suspensionen, nasal, z.B. in Form von Nasensprays, oder rektal, z.B. in Form von Suppositorien, verabreicht werden. Die Verabreichung kann aber auch parenteral, wie intramuskulär oder intravenös, z.B. in Form von Injektionslösungen, erfolgen.

Zur Herstellung von Tabletten, Lacktabletten, Dragees und Hartgelatinekapseln können die Verbindungen der Formel I sowie ihre pharmazeutisch verwendbaren Salze mit pharmazeutisch inerten, anorganischen oder organischen Excipientien verarbeitet werden. Als solche Excipientien kann man z.B. für Tabletten, Dragees und Hartgelatinekapseln, Laktose, Maisstärke oder Derivate davon, Talk, Stearinsäure oder deren Salze etc. verwenden.

Für Weichgelatinekapseln eignen sich als Excipientien z.B. vegatabile Oele, Wachse, Fette, halbfeste und flüssige Polyole etc.

Zur Herstellung von Lösungen und Sirupen eignen sich als Excipientien z.B. Wasser, Polyole, Saccharose, Invertzucker, Glukose etc.

Für Injektionslösungen eignen sich als Excipientien z.B. Wasser, Alkohole, Polyole, Glycerin, vegetabile Oele etc.

Für Suppositorien eignen sich als Excipientien z.B. natürliche oder gehärtete Oele, Wachse, Fette, halbflüssige oder flüssige Polyole etc.

Die pharmazeutischen Präparate können daneben noch Konservierungsmittel, Lösungsvermittler, viskositätserhöhen de Stoffe, Stabilisierungsmittel, Netzmittel, Emulgiermittel, Süssmittel, Färbemittel, Aromatisierungsmittel, Salze zur Veränderung des osmotischen Druckes, Puffer, Ueberzugsmittel oder Antioxidantien enthalten. Sie können auch noch andere therapeutisch wertvolle Stoffe enthalten.

Erfindungsgemäss kann man die Verbindungen der allgemeinen Formel I sowie deren pharmazeutisch verwendbare Salze bei der Bekämpfung bzw. Verhütung von Bluthochdruck und Herzinsuffizienz verwenden. Die Dosierung kann innerhalb weiter Grenzen variieren und ist natürlich in jedem einzelnen Fall den individuellen Gegebenheiten anzupassen. Im allgemeinen dürfte bei oraler Verabreichung eine Tagesdosis von etwa 3 mg bis etwa 3 g, vorzugsweise etwa 10 mg bis etwa 1 g, z.B. ungefähr 300 mg pro Person, verteilt auf vorzugsweise 1-3 Einzeldosen, die z.B. gleich gross sein können, angemessen sein, wobei aber die soeben angegebene obere Grenze auch überschritten werden kann, wenn sich dies als angezeigt erweisen sollte. Ueblicherweise erhalten Kinder die halbe Dosis von Erwachsenen.

Die folgenden Beispiele sollen die vorliegende Erfindung erläutern, sie jedoch in keiner Weise einschränken. Alle Temperaturen sind in Celsiusgraden angegeben. Es werden die folgenden Abkürzungen verwendet:

H-His-OH = L-Histidin
Boc = t-Butoxycarbonyl
Fmoc = 9-Fluorenylmethoxycarbonyl

## Beispiel 1

Ein Gemisch von 880 mg (2,23 mMol) (S)-α-Amino-N-[(1S,2R,3RS)-1-(cyclohexylmethyl)-4-äthyl -2,3-dihydroxyhexyl]imidazol-4-propionamid, 608 mg (2,45 mMol) (R)-α-(Pivaloylmethyl)hydrozimtsäure (vgl. EPA 0,184 550), 0,35 ml (2,76 mMol) 4-Aethylmorpholin, 662 mg (4,9 mMol) HBT und 529 mg (2,76 mMol) EDC in 20 ml Dimethylformamid wird über Nacht bei Raumtemperatur gerührt. Danach wird das Reaktionsgemisch im Hochvakuum zur Trockene eingedampft, der Rückstand auf ein Gemisch von Eis und 2N Natriumbicarbonat-Lösung gegossen und dreimal mit Essigester extrahiert. Die Extrakte werden nacheinander mit gesättigter Ammoniumchlorid-Lösung, 2N Natriumbicarbonat-Lösung und gesättigter Natriumchlorid-Lösung gewaschen und vereinigt. Nach dem Trocknen der organischen Lösung über Magnesiumsulfat wird das Lösungsmittel unter vermindertem Druck abgedampft, und der Rückstand (1,01 g) zur Reinigung an 70 g Kieselgel unter Verwendung eines 20:1:0,1-Gemisches von Methylenchlorid, Methanol und Ammoniak als Eluierungsmittel chromatographiert. Kristallisation des so erhaltenen Rohprodukts aus Methylenchlorid/Methanol/Aether Liefert 900 mg (S)-N-[(1S,2R,3RS)-1-(Cyclohexylmethyl)-4-äthyl -2,3-dihydroxyhexyl]-α-[(R)-α-(3,3-dimethyl -2-oxobutyl)hydrocinnamamido]imidazol-4-propionamid, Schmelzpunkt 160°, MS: 624 $(M)^+$.

Das als Ausgangsstoff eingesetzte (S)-α-Amino-N-[(1S,2R,3RS)-1-(cyclohexylmethyl)-4-äthyl -2,3-dihydroxyhexyl]imidazol-4-propionamid wurde wie folgt hergestellt:

Ein Gemisch von 87,9 g (300 mMol) 2-t-Butoxycarbonylamino-3(S)-cyclohexylpropylaldehyd, welcher seinerseits nach der von J. Boger et al. in J. Med. Chem., 28, 1779 (1985) beschriebenen Methode hergestellt worden war, 1,2 g eines 1:1-Gemisches von Kaliumcyanid und 18-Crown 6 und 65 ml (520 mMol) Trimethylsilylcyanid wird in einer Argonatmosphäre 1 Stunde bei Raumtemperatur gerührt. Dann wird das Reaktionsgemisch zur Trockene eingedampft, und der Rückstand zweimal nacheinander zunächst mit Toluol versetzt und danach wieder zur Trockene eingedampft. Der Rückstand wird in einem Gemisch von 200 ml Eisessig und 200 ml konz. Salzsäure gelöst, und das Reaktionsgemisch über Nacht zum Rückfluss erhitzt. Danach wird das Reaktionsgemisch unter vermindertem Druck eingedampft, und der Rückstand zwischen 800 ml Wasser und 800 ml Aether verteilt. Die organische Phase wird zweimal mit 400 ml Wasser gewaschen, und das Waschwasser mit der wässrigen Phase vereinigt. Dann wird das Wasser abgedampft, und der Rückstand zweimal nacheinander zunächst mit Toluol versetzt und danach wieder zur Trockene eingedampft. Zum Rückstand (83,7 g), der in 1 l Methanol gelöst wurde, werden in einer Argonatmosphäre unter Rühren und bei einer Temperatur zwischen -10° und -20° innerhalb von 30 Minuten 23,94 ml (330 mMol) Thionylchlorid getropft. Nach beendeter Zugabe wird das Reaktionsgemisch über Nacht bei Raumtemperatur gerührt und danach unter vermindertem Druck zur Trockene eingedampft. Der Rückstand wird zweimal nacheinander zunächst mit Toluol versetzt und danach wieder zur Trockene eingedampft. Ein Gemisch des so erhaltenen Rückstandes in 500 ml Dimethylformamid, 188 ml (1,35 Mol) Triäthylamin und 78 g (0,36 Mol) Di-t-butyldicarbonat wird über Nacht bei Raumtemperatur gerührt und danach im Hochvakuum eingedampft. Der Rückstand wird dreimal mit je 1,8 l Essigester extrahiert. Die drei organischen Extrakte werden nacheinander mit je 900 ml eiskalter 1N Schwefelsäure, 900 ml 2N Natriumbicarbonat-Lösung und 900 ml Wasser gewaschen, vereinigt, über Magnesiumsulfat getrocknet und eingedampft. Chromatographie des so erhaltenen Rohprodukts an 1,5 kg Kieselgel mit einem 92:8-Gemisch von Toluol und Essigester als Eluierungsmittel liefert 13,14 g t-Butyl [(1S,2R)-1-(cyclohexylmethyl)-2-hydroxy -3-(methoxycarbonyl)äthyl]carbamat als Oel, MS: 315 $(M)^+$, sowie 10,6 g des weniger polaren Epimeren t-Butyl [(1S,2S)-1-(cyclohexylmethyl)-2-hydroxy -3-(methoxycarbonyl)äthyl]carbamat ebenfalls als Oel, MS: 315 $(M)^+$.

Zu einer Lösung der aus 44,18 ml (345 mMol) 3-Brompentan (97%ig) und 8,39 g (345 Grammatom) Magnesiumspänen in 280 ml Tetrahyrofuran hergestellten Grignardverbindung wird bei ca. 30° eine Lösung von 2,72 g (7,57 mMol) t-Butyl [(1S,2R)-1-(cyclohexylmethyl)-2-hydroxy -3-(methoxycarbonyl)äthyl]-carbamat in 70 ml Tetrahydrofuran getropft, und das Reaktionsgemisch anschliessend 70 Stunden bei Raumtemperatur unter Argon gerührt. Danach giesst man das Raktionsgemisch auf 100 ml einer eiskalten gesättigten Ammoniumchlorid-Lösung, trennt die organische Phase ab und extrahiert die wässrige Phase noch zweimal mit je 300 ml Aether. Die beiden Aetherextrakte werden mit je 100 ml gesättigter Ammoniumchlorid-Lösung gewaschen, vereinigt und über Magnesiumsulfat getrocknet. Dann wird das Lösungsmittel unter vermindertem Druck abgedampft, und der Rückstand an 250 g Kieselgel mit einem 4:1-Gemisch von Hexan und Aether als Eluierungsmittel chromatographiert, wobei man 180 mg t-Butyl [-(1S,2R)-1-(cyclohexylmethyl)-4-äthyl-2-hydroxy -3-oxohexyl]carbamat als Oel erhält, MS: 355 $(M)^+$.

320 mg (0,9 mMol) t-Butyl [(1S,2R)-1-(cyclohexylmethyl)--4-äthyl-2-hydroxy -3-oxohexyl]carbamat in 10 ml Aethanol werden mit 34 mg (0,9 mMol) Natriumborhydrid 3 Stunden bei Raumtemperatur gerührt. Danach gibt man 0,5 ml Essigsäure zu, dampft das Reaktionsgemisch ein und extrahiert den Rückstand dreimal mit je 150 ml Essigester. Dann werden die drei organischen Extrakte mit je 70 ml 2N Natriumbicarbonat-Lösung und 70 ml gesättigter Natriumchlorid-Lösung gewaschen, vereinigt, über Magnesiumsulfat getrocknet, filtriert und eingedampft. Zur Reinigung wird der Rückstand an 30 g Kieselgel unter Verwendung eines 7:3-Gemisches von Hexan und Aether als Eluierungsmittel chromatographiert, wobei 260 mg t-Butyl [(1S,2R,3RS)-1-(cyclohexylmethyl)-4-äthyl -2,3-dihydroxyhexyl]carbamat als Oel erhalten werden, MS: 357 $(M)^+$.

540 mg (1,51 mMol) t-Butyl [(1S,2R,3RS)-1-(cyclohexylmethyl)-4-äthyl -2,3-dihydroxyhexyl]carbamat in 20 ml 1,58N Salzsäure in Dioxan werden über Nacht bei Raumtemperatur stehen gelassen. Das Reaktionsgemisch wird danach eingedampft, und der Rückstand zweimal nacheinander zunächst mit Toluol versetzt und dann wieder zur Trockene eingedampft. Ein Gemisch des so erhaltenen Rohprodukts mit 995 mg (1,66 mMol) $(Fmoc)_2$His-OH, 0,42 ml (3,22 mMol) 4-Aethylmor pholin, 449 mg (3,22 mMol) HBT und 347 mg (1,81 mMol) EDC in 20 ml Dimethylformamid wird über Nacht bei Raumtemperatur stehen gelassen. Danach wird das Reaktionsgemisch im Hochvakuum eingedampft, der Rückstand auf ein Gemisch von Eis und 90 ml 2N Natriumbicarbonat-Lösung gegossen und dreimal mit je 150 ml Essigester extrahiert. Die drei Essigesterextrakte werden nacheinander mit 70 ml gesättigter Ammoniumchlorid-Lösung, 70 ml 2N Natriumbicarbonat-Lösung und 70 ml gesättigter Natriumchlorid-Lösung gewaschen, vereinigt, über Magnesiumsulfat getrocknet, filtriert und eingedampft. Das erhaltene Rohprodukt (2,2 g) wird in 60 ml Methylenchlorid und 2 ml Piperidin 3 Stunden bei Raumtemperatur gerührt. Dann wird das Reaktionsgemisch eingedampft, und der Rückstand aus 50 ml Hexan trituriert und abfiltriert. Das Filtrat wird mit einem 8:1:0,1-Gemisch von Methylenchlorid, Methanol und Ammoniak als Eluierungsmittel an 70 g Kieselgel chromatographiert, wobei man 880 mg (S)-α-Amino-N-[(1S,2R,3RS)-1-(cyclohexylmethyl)-4-äthyl -2,3-dihydroxyhexyl]-imidazol-4-propionamid als Schaum erhält, MS: 394 $(M)^+$.

## Beispiel 2

In analoger Weise wie in Beispiel 1 beschrieben, wurden die folgenden Verbindungen hergestellt:

- Aus (S)-α-Amino-N- [(1S,2R,3S)-3-cyclohexyl -1-(cyclohexylmethyl)-2,3-dihydroxypropyl]imidazol -4-propionamid und (R)-α-(Pivaloylmethyl)hydrozimtsäure das (S)-N-[(1S,2R,3S)-3-Cyclohexyl-1-(cyclohexylmethyl) -2,3-dihydroxypropyl]-α-[(R)-α-(3,3-dimethyl -2-oxobutyl)hydrocinnamamido]imidazol-4-propionamid als weissen Festkörper, Schmelzpunkt >175° (Zers.; aus Methylenchlorid/Methanol/Hexan), MS: 636 $(M)^+$;
- aus (S)-α-Amino-N-[(1S,2R,3S)-3-cyclohexyl -1-(cyclohexylmethyl)-2,3-dihydroxypropyl]imidazol -4-propionamid und (S)-α-[(t-Butylsulfonyl)methyl]hydrozimtsäure (vgl. EPA 0.236 734) das (S)-α-[(S)-α-[(t-Butylsulfonyl)methyl]hydrocinnamamido]-N-[(1S,2R,3S)-3-cyclohexyl-1-(cyclohexylmethyl) -2,3-dihydroxypropyl]-imidazol-4-propionamid, MS: 673 $(M+l)^+$;
- aus (S)-α-Amino-N-[(1S,2R,3S) -1-(cyclohexylmethyl)-2,3-dihydroxy -4-methylpentyl]imidazol-4-propionamid und (R)-α-(Pivaloylmethyl)hydrozimtsäure das (S)-N-[(1S,2R,3S)-1-(Cyclohexylmethyl)-2,3-dihydroxy-4-methylpentyl]-α-[(R)-α-(3,3-dimethyl -2-oxobutyl)hydrocinnamamido]imidazol-4-propionamid als Festkörper vom Schmelzpunkt 153° (aus Methylenchlorid/Methanol/Hexan);
- aus (S)-α-Amino-N-[(1S,2R,3S) -1-(cyclohexylmethyl)-2,3-dihydroxy -4-methylpentyl]imidazol-4-propionamid und (S)-α-[(t-Butylsulfonyl)methyl]hydrozimtsäure das (S)-α-[(S)-α-[(t-Butylsulfonyl)methyl]-hydrocinnamamido]-N-[(1S,2R,3S)-1-(cyclohexylmethyl)-2,3-dihydroxy-4-methylpentyl]imidazol-4-propionamid als weissen Festkörper, Schmelzpunkt 114° (Zers.; aus Methylenchlorid/Aether), MS: 597 $(M+l)^+$.

Die als Ausgangsstoffe eingesetzten Propionamide wurden wie folgt hergestellt:

(S)-α-Amino-N-[(1S,2R,3S)-3-cyclohexyl-1-(cyclohexylmethyl)-2,3-dihydroxypropyl]imidazol-4-propionamid

In analoger Weise wie in Beispiel 1 beschrieben, wird t-Butyl [(1S,2R)-1-(cyclohexylmethyl-2-hydroxy -3-(methoxycarbonyl)äthyl]carbamat in einer Grignard-Reaktion mit Cyclohexylmagnesiumbromid zu t-Butyl [(1S,2R)-3-cyclohexyl-1-(cyclohexylmethyl)-2-hydroxy -3-oxopropyl]carbamat umgesetzt, welches nach in zu Beispiel 1 analoger Weise durchgeführten Reduktion mit Natriumborhydrid und chromatographi scher Auftrennung der erhaltenen Isomeren t-Butyl [(1S,2R,3S)-3-cyclohexyl-1-(cyclohexylmethyl) -2,3-dihydroxypropyl]carbamat liefert. Dann wird, wiederum in analoger Weise wie in Beispiel 1 beschrieben, die Boc-Schutzgruppe mit Salzsäure in Dioxan abgespalten, das erhaltene Rohprodukt mit $(Fmoc)_2$His-OH umgesetzt, und die beiden Schutzgruppen mit Piperidin abgespalten.

(S)-α-Amino-N-[(1S,2R,3S)-1-(cyclohexylmethyl)-2,3-dihydroxy-4-methylpentyl]imidazol-4-propionamid

Diese Verbindung wurde, ebenfalls in analoger Weise wie in Beispiel 1 beschrieben, durch Umsetzen von t-Butyl [(1S,2R)-1-(cyclohexylmethyl)-2-hydroxy -3-(methoxycarbonyl)äthyl]carbamat mit Isopropylmagnesiumbromid, Reduktion des erhaltenen t-Butyl [(1S,2R)-1-(cyclohexylmethyl)-2-hydroxy-4-methyl -3-oxopentyl]carbamats mit Natriumborhydrid, chromatographischer Auftrennung der erhaltenen Isomeren, Abspaltung der Boc-Schutzgruppe aus t-Butyl [(1S,2R,3S)-1-(cyclohexylmethyl)-2,3-dihydroxy -4-methylpentyl]carbamat mit Salzsäure in Dioxan, Umsetzung mit $(Fmoc)_2$His-OH und Abspaltung der beiden Schutzgruppen mit Piperidin erhalten.

## Beispiel 3

In analoger Weise wie in Beispiel 1 beschrieben, wurden die folgenden Verbindungen hergestellt:

- Aus (S)-α-Amino-N-[(1S,2R,3S) -1-(cyclohexylmethyl)-2,3-dihydroxy-3-phenylpropyl]imidazol-4-propionamid und (S)-α-[(t-Butylsulfonyl)methyl]hydrozimtsäure das (S)-α-[(S)-α -[(t-Butylsulfonyl)methyl]-hydrocinnamamido] -N-[(1S,2R,3S)-1-(cyclohexylmethyl)-2,3-dihydroxy-3-phenylpropyl]imidazol-4-propionamid als weissen Festkörper vom Schmelzpunkt 112° (aus Methylenchlorid/Diäthyläther/Hexan) sowie aus (S)-α-Amino- N-[(1S,2R,3S)-1-(cyclohexylmethyl) -2,3-dihydroxy-3-phenylpropyl]imidazol-4-propionamid und (S)-α-[(t-Butylsulfonyl)methyl]hydrozimtsäure das epimere (S)-α-[(t-Butylsulfonyl)methyl]-hydrocinnamamido]-N-[(1S,2R,3R)-1-(cyclohexylmethyl-2,3-dihydroxy-3-phenylpropyl]imidazol-4-propionamid als weissen Festkörper vom Schmelzpunkt 111° (Zers.; aus Methylenchlorid/Diäthyläther);
- aus (S)-α-Amino-N-[(1S,2R,3RS) -1-(cyclohexylmethyl)-2,3-dihydroxy -4,4-dimethylpentyl]imidazol-4-propionamid und (S)-α-[(t-Butylsulfonyl)methyl]hydrozimtsäure das (S)-α-[(S)-α -[(t-Butylsulfonyl)methyl]-hydrocinnamamido] -N-[(1S,2R,3RS)-1-(cyclohexylmethyl)-2,3-dihydroxy -4,4-dimethylpentyl]imidazol-4-propionamid als weissen Festkörper vom Schmelzpunkt 210° (aus Methylenchlorid/Methanol/Diäthyläther), MS: 646 $(M)^+$.

Die als Ausgangstoffe eingesetzte Propionamide wurden wie folgt hergestellt:

<u>(S)-α-Amino-N-[(1S,2R,3S)-1-(cyclohexylmethyl)-2,3-dihydroxy-3-phenylpropyl]imidazol-4-propionamid und (S)-α-Amino-N-[(1S,2R,3R)-1-(cyclohexylmethyl)-2,3-dihydroxy-3-phenylpropyl]imidazol-4-propionamid</u>

7,5 g t-Butyl [(1S,2R)-1-(cyclohexylmethyl)-2-hydroxy-3-(methoxycarbonyl)äthyl]carbamat werden in 50 ml Dimethoxypropan mit 150 mg p-Toluolsulfonsäure über Nacht bei 50° gerührt. Danach wird das Reaktionsgemisch auf eiskalte 2N Natriumbicarbonat-Lösung gegossen und dreimal mit je 300 ml Aether extrahiert. Die drei organischen Extrakte werden mit Wasser gewaschen, vereinigt, über Magnesiumsulfat getrocknet und zur Trockene eingedampft. Das zurückbleibende Oel (8,43 g) wird mit einem 95:5-Gemisch von Toluol und Essigester als Eluierungsmittel, enthaltend 1% Triäthylamin, an 250 g Kieselgel chromatographiert, wobei man 7,06 g 3-t-Butyl 5-methyl (4S,5R)-4-(cyclohexylmethyl)-2,2-dimethyl -3,5-oxazolidindicarboxylat als Oel erhält, MS: 355 $(M)^+$.

Zu 355 mg (1 mMol) 3-t-Butyl 5-methyl (4S,5R)-4-(cyclohexylmethyl)-2,2-dimethyl -3,5-oxazolidindicarboxylat in 20 ml Aether werden bei -75° 2,36 ml (2 mMol) einer 0,76N Lösung von Phenyllithium in Aether gespritzt, und das Reaktionsgemisch 30 Minuten bei dieser Temperatur gerührt, anschliessend auf 50 ml gesättigte Ammoniumchlorid-Lösung gegossen und dreimal mit je 150 ml Aether extrahiert. Die drei Aetherextrakte werden nacheinander mit je 70 ml 2N Natriumbicarbonat-Lösung und 70 ml gesättigter Natriumchlorid-Lösung gewaschen, vereinigt, über Magnesiumsulfat getrocknet, filtriert und eingedampft. Das zurückbleibende Oel (540 mg) wird mit einem 98:2-Gemisch von Toluol und Essigester als Eluierungsmittel an 30 g Kieselgel chromatographiert, wobei man 300 mg t-Butyl (4S,5R)-5-benzoyl-4-(cyclohexylmethyl)-2,2-dimethyl -3-oxazolidincarboxylat als weissen Festkörper erhält, MS: 401 $(M)^+$.

290 mg (0,72 mMol) t-Butyl (4S,5R)-5-benzoyl-4-(cyclohexylmethyl)-2,2-dimethyl -3-oxazolidincarboxylat in 5 ml Methanol werden mit 27 mg (0,7 mMol) Natriumborhydrid 1 Stunde bei Raumtemperatur gerührt. Danach wird das Reaktionsgemisch mit zwei Tropfen Eisessig versetzt und zur Trockene eingedampft. Der Rückstand wird an 30 g Kieselgel mit einem 4:1-Gemisch von Hexan und Aether als Eluierungsmittel chromatographiert, wobei eine Methylenchloridlösung des Rohprodukts auf die Säule gegeben wird. Man erhält auf diese Weise 60 mg t-Butyl (4S,5R)-4-(cyclohexylmethyl)-5-[(S)-α-hydroxybenzyl] -2,2-dimethyl-3-oxazolidincarboxylat als weissen Festkörper, MS: 403 $(M)^+$, sowie 210 mg des epimeren t-Butyl (4S,5R)-4-(cyclohexylmethyl)-5-[(R)-α-hydroxybenzyl] -2,2-dimethyl-3-oxazolidincarboxylat ebenfalls als weissen Festkörper, MS: 403 $(M)^+$.

Danach wird mit methanolischer Salzsäure je die Boc-Schutzgruppe aus den beiden epimeren Carboxylaten abgespalten, die erhaltenen Rohprodukte mit $(Fmoc)_2$His-OH gekoppelt und schliesslich jeweils die beiden Schutzgruppen mit Piperidin entfernt.

<u>(S)-α-Amino-N-[(1S,2R,3RS)-1-(cyclohexylmethyl)-2,3-dihydroxy-4,4-dimethylpentyl]imidazol-4-propionamid</u>

In analoger Weise wie oben beschrieben, wird durch Umsetzen von 3-t-Butyl 5-methyl (4S,5R)-4-(cyclohexylmethyl)-2,2-dimethyl -3,5-oxazolidindicarboxylat mit t-Butyllithium das t-Butyl (4S,5R)-4-(cyclohexylmethyl)-2,2-dimethyl-5-pivaloyl -3-oxazolidincarboxylat hergestellt, welches mit Natriumborhydrid zu t-Butyl (4S,5R)-4-(cyclohexylmethyl)-5-[(RS)-1-hydroxy -2,2-dimethylpropyl]-2,2-dimethyl-3-oxazolidincarboxylat reduziert wird. Abspaltung der Boc-Schutzgruppe mit methanolischer Salzsäure, Kuppeln mit

(Fmoc)$_2$His-OH und Abspalten der beiden Schutzgruppen mit Piperidin liefert (S)-α-Amino-N-[(1S,2R,3RS)-1-(cyclohexylmethyl) -2,3-dihydroxy-4,4-dimethylpentyl]imidazol-4-propionamid.

## Beispiel 4

In analoger Weise wie in Beispiel 1 beschrieben werden die folgenden Verbindungen hergestellt:

- Aus (S)-α-Amino-N-[(1S,2R,3S)-1-(cyclohexylmethyl)-3-cyclopropyl-2,3-dihydroxypropyl]imidazol-4-propionamid und (R)-α-(Pivaloylmethyl)hydrozimtsäure das (S)-N-[(1S,2R,3S)-1-(Cyclohexylmethyl)-3-cyclopropyl-2,3-dihydroxypropyl]-α-[(R)-α-(3,3-dimethyl-2-oxobutyl)hydrocinnamamido]imidazol-4-propionamid als Festkörper, Schmelzpunkt 136° (aus Methylenchlorid/Methanol/Aether/Hexan);
- Aus (S)-α-Amino-N-[(1R,2S,3R)-1-(cyclohexylmethyl)-2,3-dihydroxy-4-methylpentyl]imidazol-4-propionamid und (S)-α-[(t-Butylsulfonyl)methyl]hydrozimtsäure das (S)-α-[(S)-α-[(t-Butylsulfonyl)methyl]-hydrocinnamamido]-N-[(1R,2R,3R)-1-(cyclohexylmethyl)-2,3-dihydroxy-4-methylpentyl]imidazol-4-propionamid als weisser Festkörper, Schmelzpunkt 114° (Zers.; aus Methylenchlorid/Aether);
- Aus (S)-α-Amino-N-[(1S,2R,3S)-3-cyclohexyl-1-(cyclohexylmethyl)-2,3-dihydroxypropyl]imidazol-4-propionamid und (S)-α-[(t-Butylsulfonyl)methyl]hydrozimtsäure das (S)-α-[(S)-α-[(t-Butylsulfonyl)methyl]-hydrocinnamamido]-N-[(1S,2R,3S)-3-cyclohexyl-1-(cyclohexylmethyl)-2,3-dihydroxypropyl]imidazol-4-propionamid als weisser Festkörper, MS:673 $(M+H)^+$.
- Aus (S)-α-Amino-N-[(1R,2S,3R)-3-cyclohexyl-1-(cyclohexylmethyl)-2,3-dihydroxypropyl]imidazol-4-propionamid und (S)-α-[(t-Butylsulfonyl)methyl]hydrozimtsäure das (S)-α-[(S)-α-[(t-Butylsulfonyl)methyl]-hydrocinnamamido]-N-[(1R,2S,3R)-3-cyclohexyl-1-(cyclohexylmethyl)-2,3-dihydroxypropyl]imidazol-4-propionamid als weisser Festkörper, MS: 673 $(M+H)^+$.
- Aus (S)-α-Amino-N-[(1S,2R,3R,4RS)-1-(cyclohexylmethyl)-2,3-dihydroxy-4-methylhexyl]imidazol-4-propionamid und (S)-α-[(t-Butylsulfonyl)methyl]hydrozimtsäure das (S)-α-[(S)-α-[(t-Butylsulfonyl)methyl]-hydrocinnamamido]-N-[(1S,2R,3S,4RS)-1-(cyclohexylmethyl)-2,3-dihydroxy-4-methylhexyl]imidazol-4-propionamid als weisser Festkörper, MS: 647 $(M+H)^+$.
- Aus (S)-α-Amino-N-[(1S,2R,3S,4RS)-1-(cyclohexylmethyl)-2,3-dihydroxy-4-methylhexyl]imidazol-4-propionamid und (S)-α-[(t-Butylsulfonyl)methyl]hydrozimtsäure das (S)-α-[(S)-α-[(t-Butylsulfonyl)methyl]-hydrocinnamamido]-N-[(1S,2R,3R,4RS)-1-(cyclohexylmethyl)-2,3-dihydroxy-4-methylhexyl]imidazol-4-propionamid als weisser Festkörper, MS: 647 $(M+H)^+$.
- Aus (S)-α-Amino-N-[(1S,2R,3S)-1-(cyclohexylmethyl)-2,3-dihydroxy-4-methylpentyl]hexanamid und (S)-α-[-(t-Butylsulfonyl)methyl]hydrozimtsäure das (S)-α-[(t-Butylsulfonyl)methyl]-N-[(S)-1-[[(1S,2R,3S)-1-(cyclohexylmethyl)-2,3-dihydroxy-4-methylpentyl]carbamoyl]pentyl]hydrocinnamamid als weisser Festkörper, Schmelzpunkt 190° (aus Methylenchlorid/Aether/Hexan);
- Aus (S)-α-Amino-N-[(1R,2S,3R)-1-(cyclohexylmethyl)-2,3-dihydroxy-4-methylpentyl]hexanamid und (S)-α-[-(t-Butylsulfonyl)methyl]hydrozimtsäure das (S)-α-[(t-Butylsulfonyl)methyl]-N-[(S)-1-[[(1R,2S,3S)-1-(cyclohexylmethyl)-2,3-dihydroxy-4-methylpentyl]carbamoyl]pentyl]hydrocinnamamid als weisser Festkörper, Schmelzpunkt 182° (aus Methylenchlorid/Aether/Hexan);
- Aus (S)-α-Amino-N-[(1S,2R,3S)-1-(cyclohexylmethyl)-3-cyclopropyl-2,3-dihydroxypropyl]imidazol-4-propionamid und (S)-α-[(t-Butylsulfonyl)methyl]hydrozimtsäure das (S)-α-[(S)-α-[(t-Butylsulfonyl)methyl]-hydrocinnamamido]-N-[(1S,2R,3S)-1-(cyclohexylmethyl)-3-cyclopropyl-2,3-dihydroxypropyl]imidazol-4-propionamid als weisser Festkörper, Schmelzpunkt 100° (Zers.; aus Methylenchlorid/Aether/Hexan);
- Aus (S)-α-Amino-N-[(1R,2S,3R)-1-(cyclohexylmethyl)-3-cyclopropyl-2,3-dihydroxypropyl]imidazol-4-propionamid und (S)-α-[(t-Butylsulfonyl)methyl]hydrozimtsäure das (S)-α-[(S)-α-[(t-Butylsulfonyl)methyl]-hydrocinnamamido]-N-[(1R,2S,3R)-1-(cyclohexylmethyl)-3-cyclopropyl-2,3-dihydroxypropyl]imidazol-4-propionamid als weisser Festkörper, Schmelzpunkt 100° (Zers.; aus Methylenchlorid/Aether/Hexan);
- Aus (S)-α-Amino-N-[(1S,2R,3S)-3-cyclohexyl-1-(cyclohexylmethyl)-2,3-dihydroxypropyl]hexanamid und (S)-α-[(t-Butylsulfonyl)methyl]hydrozimtsäure das (S)-α-[(t-Butylsulfonyl)methyl]-N-[(S)-1-[[(1S,2R,3S)-3-cyclohexyl-1-(cyclohexylmethyl)-2,3-dihydroxypropyl]carbamoyl]pentyl]hydrocinnamamid als weisser Festkörper, Schmelzpunkt 187° (aus Methylenchlorid/Aether/Hexan);
- Aus (S)-α-Amino-N-[(1R,2S,3R)-3-cyclohexyl-1-(cyclohexylmethyl)-2,3-dihydroxypropyl]hexanamid und (S)-α-[(t-Butylsulfonyl)methyl]hydrozimtsäure das (S)-α-[(t-Butylsulfonyl)methyl]-N-[(S)-1-[[(1R,2S,3R)-3-cyclohexyl-1-(cyclohexylmethyl)-2,3-dihydroxypropyl]carbamoyl]pentyl]hydrocinnamamid als weisser Festkörper, Schmelzpunkt 188° (aus Methylenchlorid/Aether/Hexan);
- Aus (S)-α-Amino-N-[(1S,2R,3S)-1-(cyclohexylmethyl)-2,3-dihydroxy-4-methylpentyl]imidazol-4-propionamid und (S)-α-[(Diäthoxyphosphinyl)methyl]hydrozimtsäure (vgl. EPA 0 117 429) das Diäthyl [(S)-2-[[(S)-1-[[-

(1S,2R,3S)-1-(cyclohexylmethyl)-2,3-dihydroxy-4-methylpentyl]carbamoyl]-2-imidazol-4-yläthyl]carbamoyl]-3-phenylpropyl]phosphonat als weisser Festkörper, Schmelzpunkt 98° (aus Methylenchlorid/Aether/Hexan);

- Aus (S)-α-Amino-N-[(1S,2R,3S)-1-(cyclohexylmethyl)-2,3-dihydroxy-4-methylpentyl]imidazol-4-propionamid und Dibenzylessigsäure das (S)-N-[(1S,2R,3S)-1-(cyclohexylmethyl)-2,3-dihydroxy-4-methylpentyl]-α-(2,2-dibenzylacetamido)imidazol-4-propionamid als weisser Festkörper, Schmelzpunkt 193° (aus Methylenchlorid/Methanol/Aether/Hexan);
- Aus (S)-α-Amino-N-[(1S,2R,3S)-1-(cyclohexylmethyl)-2,3-dihydroxy-4-methylpentyl]imidazol-4-propionamid und (RS)-α-[(t-Butylsulfonyl)methyl]-m-methoxyhydrozimtsäure das (S)-α-[(R oder S)-α-[(t-Butylsulfonyl)-methyl]-m-methoxyhydrocinnamamido]-N-[(1S,2R,3S)-1-(cyclohexylmethyl)-2,3-dihydroxy-4-methylpentyl]-imidazol-4-propionamid als weisser Festkörper, Schmelzpunkt 153° (aus Methylenchlorid/Aether/Hexan) und das epimere (S)-α-[(S oder R)-α[t-Butylsulfonyl)methyl]-m-methoxyhydrocinnamamido]-N-[(1S,2R,3S)-1-(cyclohexylmethyl)-2,3-dihydroxy-4-methylpentyl]imidazol-4-propionamid als weisser Festkörper, Schmelzpunkt 108° (aus Methylenchlorid/Aether/Hexan);
- Aus (S)-α-Amino-N-[(1S,2R,3S)-1-(cyclohexylmethyl)-2,3-dihydroxy-4-methylpentyl]imidazol-4-propionamid und (RS)-α-[(t-Butylsulfonyl)methyl]-p-methoxyhydrozimtsäure das (S)-α-[(R oder S)-α-[(t-Butylsulfonyl)-methyl]-p-methoxyhydrocinnamamido]-N-[(1S,2R,3S)-1-(cyclohexylmethyl)-2,3-dihydroxy-4-methylpentyl]-imidazol-4-propionamid als weisser Festkörper, Schmelzpunkt 121° (aus Methylenchlorid/Aether/Hexan) und das epimere (S)-α-[(S oder R)-α-[t-Butylsulfonyl)methyl]-p-methoxyhydrocinnamamido]-N-[(1S,2R,3S)-1-(cyclohexylmethyl)-2,3-dihydroxy-4-methylpentyl]imidazol-4-propionamid als weisser Festkörper, Schmelzpunkt 109° (aus Methylenchlorid/Methanol/Aether/Hexan);
- Aus (S)-α-Amino-N-[(1S,2R,3S)-1-(cyolohexylmethyl)-2,3-dihydroxy-4-methylpentyl]imidazol-4-propionamid und (S)-α-[[(RS)-t-Butylsulfinyl)methyl]hydrozimtsäure das (S)-α-[(S)-α-[[(RS)-t-Butylsulfinyl)methyl]-hydrocinnamamido]-N-[(1S,2R,3S)-1-(cyclohexylmethyl)-2,3-dihydroxy-4-methylpentyl]imidazol-4-propionamid als weisser Festkörper, Schmelzpunkt 100° (Zers.; aus Methylenchlorid/Aether/Hexan);
- Aus (S)-α-Amino-N-[(1S,2R,3S)-1-(cyclohexylmethyl)-2,3-dihydroxy-4-methylpentyl]imidazol-4-propionamid und (2R,3R oder S)-2-Benzyl-3-hydroxy-5,5-dimethyl-4-oxohexansäure das (S)-N-[(1S,2R,3S)-1-(Cyclohexylmethyl)-2,3-dihydroxy-4-methylpentyl]-α-[(R)-α-[(R oder S)-1-hydroxy-2,3-dimethyl-2-oxobutyl]-hydrocinnamamido]imidazol-4-propionamid als weisser Festkörper, Schmelzpunkt 100° (Zers.; aus Methylenchlorid/Aether/Hexan);
- Aus (S)-α-Amino-N-[(1S,2R,3S)-1-(cyclohexylmethyl)-2,3-dihydroxy-4-methylpentyl]imidazol-4-propionamid und (2R,3S oder R)-2-Benzyl-3-hydroxy-5,5-dimethyl-4-oxohexansäure das (S)-N-[(1S,2R,3S)-1-(Cyclohexylmethyl)-2,3-dihydroxy-4-methylpentyl]-α-[(R)-α[(S oder R)-1-hydroxy-2,3-dimethyl-2-oxobutyl]-hydrocinnamamido]imidazol-4-propionamid als weisser Festkörper, Schmelzpunkt 100° (Zers.; aus Methylenchlorid/Aether/Hexan);
- Aus (S)-α-Amino-N-[(1S,2R,3S)-1-(cyclohexylmethyl)-3-cyclobutyl-2,3-dihydroxypropyl]imidazol-4-propionamid und (S)-α-[(t-Butylsulfonyl)methyl]hydrozimtsäure das (S)-α-[(S)-α-[(t-Butylsulfonyl)methyl]-hydrocinnamamido]-N-[(1S,2R,3S)-3-cyclobutyl-1-(cyclohexylmethyl)-2,3-dihydroxypropyl]imidazol-4-propionamid als weisser Festkörper, Schmelzpunkt 110° (Zers.; aus Methylenchlorid/Methanol/Aether/Hexan);
- Aus (S)-N-[(1S,2R,3S)-1-(Cyclohexylmethyl)-2,3-dihydroxy-4-methylpentyl]-α-[(3-phenyl-L-alanyl)amino]-imidazol-4-propionamid und 3-(3-Pyridyl)propionsäure das (S)-N-[(1S,2R,3S)-1-(Cyclohexylmethyl)-2,3-dihydroxy-4-methylpentyl]-α-[(S)-α-(3-pyridinpropionamido)hydrocinnamamido]imidazol-4-propionamid als weisser Festkörper, Schmelzpunkt 120° (aus Methylenchlorid/Aether/Hexan);
- Aus (S)-N-[(1S,2R,3S)-1-(Cyclohexylmethyl)-2,3-dihydroxy-4-methylpentyl]-α-[(3-phenyl-L-alanyl)amino]-imidazol-4-propionamid und 2-(2-Pyridyl)benzoesäure das (S)-N-[(1S, 2R,3S)-1-(Cyclohexylmethyl)-2,3-dihydroxy-4-methylpentyl]-α-[(S)-α-[2-(2-pyridyl)benzamido]hydrocinnamamido]imidazol-4-propionamid als weisser Festkörper, Schmelzpunkt 125° (aus Methylenchlorid/Aether/Hexan);
- Aus (S)-α-Amino-N-[(1S,2R,3S)-1-(cyclohexylmethyl)-3-cyclopropyl-2,3-dihydroxypropyl]imidazol-4-propionamid und 2-(RS)-Benzyl-3-morpholinocarbonylpropionsäure das (S)-N-[(1S,2R,3S)-1-(Cyclohexylmethyl)-3-cyclopropyl-2,3-dihydroxypropyl-α-[(R)-α-[(morpholinocarbonyl)methyl]hydrocinnamamido]imidazol-4-propionamid, MS: 624 $(M+H)^+$ und epimere (S)-N-[(1S,2R,3S)-1-(Cyclohexylmethyl)-3-cyclopropyl-2,3-dihydroxypropyl]-α-[(S)-α-[(morpholinocarbonyl)methyl]hydrocinnamamido]imidazol-4-propionamid, MS: 624 $(M+H)^+$;
- Aus (S)-α-Amino-N-[(1S,2R,3S)-1-(cyclohexylmethyl)-3-cyclopropyl-2,3-dihydroxypropyl]imidazol-4-propionamid und (RS)-[(t-Butylsulfonyl)methyl]-1-naphthalinpropionsäure das (S)-α-[(R)-α-[(t-Butylsulfonyl)methyl]-1-naphthalinpropionamido]-N-[(1S,2R,3S)-1-(cyclohexylmethyl)-3-cyclopropyl-2,3-dihydroxypropyl]imidazol-4-propionamid als Schaum, MS: 681 $(M+H)^+$, und das epimere (S)-α-[(S)-α-[(t-Butylsulfonyl)methyl]-1-naphthalinpropionamido]-N-[(1S,2R,3S)-1-(cyclohexylmethyl)-3-cyclopropyl-2,3-dihydroxypropyl]imidazol-4-propionamid in Form eines Schaums, MS: 681 $(M+H)^+$;

- Aus (S)-α-Amino-N-[(1S,2R,3S)-1-(cyclohexylmethyl)-3-cyclopropyl-2,3-dihydroxypropyl]imidazol-4-propionamid und (RS)-[(t-Butylsulfonyl)-4-phenylbuttersäure das (S)-α-[(S)-2-[(t-Butylsulfonyl)methyl]-4-phenylbutyramido]-N-[(1S,2R,3S)-1-(cyclohexylmethyl)-3-cyclopropyl-2,3-dihydroxypropyl]imidazol-4-propionamid in Form eines Schaums, MS: 645 $(M+H)^+$, und das epimere (S)-α-[(R)-2-[(t-Butylsulfonyl)-methyl]-4-phenylbutyramido]-N-[(1S,2R,3S)-1-(cyclohexylmethyl)-3-cyclopropyl-2,3-dihydroxypropyl]-imidazol-4-propionamid als Schaum, MS: 645 $(M+H)^+$;

- Aus (S)-α-Amino-N-[(1S,2R,3S)-1-(cyclohexylmethyl)-3-cyclopropyl-2,3-dihydroxypropyl]imidazol-2-propionamid und (S)-α-(t-Butylsulfonyl)methyl]hydrozimtsäure das (S)-α-[(R)-α-[(t-Butylsulfonyl)methyl]-hydrocinnamamido]-N-[(1S,2R,3S)-1-(cyclohexylmethyl)-3-cyclopropyl-2,3-dihydroxypropyl]imidazol-2-propionamid als amorphen Festkörper, MS: 631 $(M+H)^+$.

Die als Ausgangsstoffe eingesetzten Propionamide wurden wie folgt hergestellt:

(S)-α-Amino-N-[(1S,2R,3S)-1-(cyclohexylmethyl)-2,3-dihydroxy-4-methylpentyl]imidazol-4-propionamid und (S)-α-Amino-N-[(1R,2S,3R)-1-(cyclohexylmethyl)-2,3-dihydroxy-4-methylpentyl]imidazol-4-propionamid

4,0 g (100 mMol) 60%ige Natriumhydrid-Dispersion in Oel werden in 70 ml Dimethylsulfoxid und 350 ml Tetrahydrofuran suspendiert und bei 3° mit 21,8 ml (104 mMol) Hexamethyldisilazan versetzt. Nach 1-stündigem Rühren des Reaktionsgemisches wird eine Suspension von 41,75 g (104 mMol) Triphenylisobutylphosphoniumbromid (hergestellt durch 2-tägiges Kochen von äquimolaren Mengen Triphenylphosphin und Isobutylbromid in Toluol) in 250 ml Tetrahydrofuran innerhalb von 30 Minuten zugetropft. Das Reaktionsgemisch wird 1 Stunde bei Raumtemperatur gerührt, dann auf -70° abgekühlt und tropfenweise mit einer Lösung von 23g (90 mMol) t-Butoxycarbonylamino-(3S)-cyclohexylpropylaldehyd innerhalb von 45 Minuten versetzt. Dann lässt man das Reaktionsgemisch unter Rühren über Nacht langsam auf Raumtemperatur erwärmen. Danach werden zunächst 10 ml Methanol und dann 500 ml gesättigte Natrium-kalium-tartrat-lösung zugegeben. Das Reaktionsgemisch auf Eis gegossen und dreimal mit Essigester extrahiert. Die organischen Phasen werden mit Wasser und 2N Natriumbicarbonatlösung gewaschen, getrocknet und eingedampft. Chromatographie des Rohprodukts (39,6 g) zur Reinigung an einem Kilogramm Kieselgel unter Verwendung eines 85:15-Gemisches von Hexan und Aether liefert 13,82 g t-Butyl [(RS,Z)-1-(cyclohexylmethyl)-4-methyl-2-pentenyl]carbamat als Oel. Kristallisation aus Hexan liefert einen weissen Festkörper, der bei 74° schmilzt, MS: 296 $(M+H)^+$.

16 g (54,1 mMol) der obigen Verbindung, 21,96 g (162 mMol) 4-Methylmorpholin-4-oxid-monohydrat, 10 ml einer Lösung von 1 g Osmiumtetroxid in 199 ml t-Butanol und 1 ml 70%iges t-Butylhydroperoxid in 100 ml Tetrahydrofuran werden über Nacht bei Raumtemperatur gerührt. Danach werden 50 ml einer 38%igen Natriumbisulfitlösung zugegeben, das Reaktionsgemisch 1 Stunde bei Raumtemperatur gerührt, auf Eis gegossen und danach mit Aether extrahiert. Die organischen Phasen werden nacheinander mit gesättigter Ammoniumchloridlösung, 2M Natriumbicarbonatlösung und Wasser gewaschen. Nach dem Trocknen, Filtrieren und Eindampfen der organischen Lösung wurde der Rückstand (18,7 g) zur Reinigung an 1 kg Kieselgel unter Verwendung von einem 7:3-Gemisch von Hexan und Aether als Eluierungsmittel chromatographiert. Die schneller laufende Komponente (Rf-Wert 0,5 in einem 1:1-Gemisch von Hexan und Aether) ist t-Butyl [(1SR,2SR,3SR)-1-(cyclohexylmethyl)-2,3-dihydroxy-4-methylpentyl]carbamat. Auf diese Weise werden 7,52 g dieser Verbindung als weisser Schaum erhalten, MS: 330 $(M+H)^+$.

Diese Verbindung wurde in analoger Weise wie in Beispiel 1 beschrieben durch Abspaltung der Boc-Schutzgruppe mit Salzsäure in Dioxan, Umsetzung mit $(Fmoc)_2$His-OH, Abspaltung der beiden Schutzgruppen mit Piperidin und chromatographische Auftrennung der beiden Isomeren in (S)-α-Amino-N-[(1S,2R,3S)-1-(cyclohexylmethyl)-2,3-dihydroxy-4-methylpentyl]imidazol-4-propionamid und (S)-α-Amino-N-[(1R,2S,3R)-1-(cyclohexylmethyl)-2,3-dihydroxy-4-methylpentyl]imidazol-4-propionamid übergeführt.

In analoger Weise wie oben beschrieben wurden die folgenden Propionamide hergestellt:

-(S)-α-Amino-N-[(1S,2R,3S)-3-cyclohexyl-1-(cyclohexylmethyl)-2,3-dihydroxypropyl]imidazol-4-propionamid und das diastereomere (S)-α-Amino-N-[(1R,2S,3R)-3-cyclohexyl-1-(cyclohexylmethyl)-2,3-dihydroxypropyl]-imidazol-4-propionamid unter Verwendung von Cyclohexylmethylbromid anstelle von Isobutylbromid zur Herstellung des Wittig-Reagens;

-(S)-α-Amino-N-[(1S,2R,3S)-1-(cyclohexylmethyl)-3-cyclopropyl-2,3-dihydroxypropyl]imidazol-4-propionamid und das diastereomere (S)-α-Amino-N-[(1R,2S,3R)-1-(cyclohexylmethyl)-3-cyclopropyl-2,3-dihydroxypropyl]-imidazol-4-propionamid unter Verwendung von Cyclopropylmethylbromid anstelle von Isobutylbromid zur Herstellung des Wittig-Reagens;

-(S)-α-Amino-N-[(1S,2R,3S)-1-(cyclohexylmethyl)-2,3-dihydroxy-4-methylpentyl]hexanamid und das diastereomere (S)-α-Amino-N-[(1R,2S,3R)-1-(cyclohexylmethyl)-2,3-dihydroxy-4-methylpentyl]hexanamid unter Verwendung von $(Fmoc)_2$Nle-OH anstelle von $(Fmoc)_2$His-OH;
-(S)-α-Amino-N-[(1S,2R,3S)-3-cyclohexyl-1-(cyclohexylmethyl)-2,3-dihydroxypropyl]hexanamid und das diastereomere (S)-α-Amino-N-[(1R,2S,3R)-3-cyclohexyl-1-(cyclohexylmethyl)-2,3-dihydroxypropyl]hexanamid unter Verwendung von Cyclohexylmethylbromid anstelle von Isobutylbromid zur Herstellung des Wittig-Reagens und von $(Fmoc)_2$Nle-OH anstelle von $(Fmoc)_2$His-OH;
-(S)-α-Amino-N-[(1S,2R,3S)-1-(cyclohexylmethyl)-3-cyclopropyl-2,3-dihydroxypropyl]imidazol-2-propionamid unter Verwendung von Cyclopropylmethylbromid anstelle von Isobutylbromid zur Herstellung des Wittig-Reagens, von $(Boc)_2$iso-Bis-OH (vgl. U.S. PS 4.612.324) anstelle von $(Fmoc)_2$His-OH und unter Verwendung eines 1:1-Gemisches von Trifluoressigsäure und Methylenchlorid anstelle von Piperidin zur Abspaltung der beiden Schutzgruppen.

-(S)-α-Amino-N-[(1S,2R,3S,4RS)-1-(cyclohexylmethyl)-2,3-dihydroxy-4-methylhexyl]imidazol-4-propionamid und das diastereomere (S)-α-Amino-N-[(1R,2S,3R,4RS)-1-(cyclohexylmethyl)-2,3-dihydroxy-4-methylhexyl]-imidazol-4-propionamid

Diese Verbindungen wurden in analoger Weise wie in Beispiel 3 beschrieben durch Umsetzen von 3-t-Butyl 5-methyl (4S,5R)-4-(cyclohexylmethyl)-2,2-dimethyl-3,5-oxazolidindicarboxylat mit sec-Butyllithium, Reduktion mit Natriumborhydrid, chromatographischer Auftrennung der erhaltenen Isomeren, Abspaltung der Boc-Schutzgruppe mit methanolischer Salzsäure, Kuppeln mit $(Fmoc)_2$His-OH und Abspalten der beiden Schutzgruppen mit Piperidin erhalten.

-(S)-α-Amino-N-[(1S,2R,3S)-1-(cyclohexylmethyl)-3-cyclobutyl-2,3-dihydroxypropyl]imidazol-4-propionamid

Zu einer Lösung der aus 15 g (111 mMol) Bromcyclobutan und 2,7 g (111 mGrammatom) Magnesiumspänen in 150 ml Tetrahydrofuran hergestellen Grignardverbindung wird innerhalb von 40 Minuten bei 30° eine Lösung von 7,23 g (22,2 mMol) t-Butyl(4S,5R)-4-(cyclohexylmethyl)-5-formyl-2,2-dimethyl-3-oxazolidincarboxylat in 150 ml Tetrahydrofuran getropft, und das Reaktionsgemisch anschliessend über Nacht bei Raumtemperatur gerührt. Danach giesst man das Reaktionsgemisch auf 300 ml einer gesättigten Ammoniumchloridlösung und extrahiert dreimal mit je 600 ml Aether. Die Aetherextrakte werden mit Ammoniumchloridlösung gewaschen, getrocknet, filtriert und eingedampft. Das Rohprodukt (9,34 g) wird an 500 g Kieselgel mit einem 92,5:7,5-Gemisch von Toluol und Essigester als Eluierungsmittel chromatographiert, wobei man 2,44 g t-Butyl (4S,5R)-4-(cyclohexylmethyl)-5-[(S)-cyclobutylhydroxymethyl]-2,2-dimethyl-3-oxazolidincarboxylat als Oel, MS: 381 $(M)^+$, und 2,9 g der epimeren Verbindung t-Butyl (4S,5R)-4-(cyclohexylmethyl)-5-[(R)-cyclobutylhydroxymethyl]-2,2-dimethyl-3-oxazolidincarboxylat ebenfalls als Oel, MS: 381 $(M)^+$, erhält.

Die als Ausgangstoffe eingesetzten Säuren sind bekannt oder wurden wie folgt hergestellt:

(RS)-α-[(t-Butylsulfonyl)methyl-p-methoxyhydrozimtsäure

Ein Gemisch von 3,16 g (16,4 mMol) 2-(p-Methoxybenzyl)acrylsäure und 1,85 ml (16,4 mMol) t-Butylmercaptan in 10 ml Aethanol wird unter Eiskühlung topfenweise mit 30,4 ml 1,08N Natriumäthylat/Aethanol-Lösung versetzt. Danach wird das Reaktionssgemisch 1 Stunde bei Raumtemperatur gerührt, wobei sich ein Festkörper abscheidet. Anschliessend werden unter Eiskühlung 30,4 ml 2,5N Salzsäure in Dioxan zugetropft, und das Gemisch wird weitere 30 Minuten bei Raumtemperatur gerührt. Danach wird das Reaktionsgemisch unter vermindertem Druck eingedampft, und die erhaltene rohe (RS)-α-[(t-Butylthio)methyl]-p-methoxyhydrozimtsäure danach in 200 ml Methylenchlorid suspendiert. Die Suspension wird unter Eiskühlung portionenweise mit 5,66 g (32,8 mMol) 3-Chlorperbenzoesäure versetzt und dann über Nacht bei Raumtemperatur gerührt. Dann wird das Reaktionsgemisch nacheinander mit 10%iger Kaliumjodidlösung und Wasser gewaschen, über Natriumsulfat getrocknet und unter vermindertem Druck eingedampft. Der Rückstand wird mit Aether angerieben, der Festkörper abfiltriert, und die aetherische Phase unter vermindertem Druck eingedampft. Zur Reinigung wird der Rückstand an Kieselgel mit einem 5:4:1-Gemisch von Hexan, Essigester und Methanol als Eluierungsmittel chromatographiert, wobei man 3,8 g (RS)-α-[(t-Butylsulfonyl)methyl]-p-methoxyhydrozimtsäure als gelblichen Festkörper erhält, MS: 314 $(M)^+$.

In analoger Weise wie oben beschrieben wurde ausgehend von 2-(m-Methoxybenzyl)acrylsäure die

(RS)-α-[(t-Butylsulfonyl)methyl]-m-methoxyhydrozimtsäure hergestellt.

(S)-α-[[(RS)-t-Butylsulfinyl]methyl]hydrozimtsäure

In einem Sulfierkolben mit mechanischem Rührwerk, pH-Regelung sowie Tropftrichter werden 550 ml Wasser vorgelegt und unter starkem Rühren mit 4,85 g (16,36 mMol) (RS)-α-[[(RS)-t-Butylsulfinyl]methyl]-hydrozimtsäureäthylester (vgl. EPA 0 236 734) in 15 ml Dimethylsulfoxid versetzt. Die Reaktionslösung wird danach auf pH 7,5 gestellt, mit 200 mg α-Chymotrypsin versetzt und der pH mit 0,037N Calciumhydroxidlösung konstant gehalten. Nach Verbrauch von 220 ml dieser Calciumhydroxidlösung (ca. 45 Stunden) wird der unreagierte Ester mit Essigester extrahiert, die wässrigen Phasen mit 30%iger Schwefelsäure auf pH 2 gestellt und mit Essigester extrahiert. Die Extrakte werden nacheiander mit Wasser und gesättigter Kochsalzlösung gewaschen, über Magnesiumsulfat getrocknet und eingedampft wobei man 2,50 g (S)-α-[[-(RS)-t-Butylsulfinyl]methyl]hydrozimtsäure erhält, MS: 269 $(M+H)^+$.

(2R,3R oder S)-2-Benzyl-3-hydroxy-5,5-dimethyl-4-oxo-hexansäure

Zu 6,5 ml einer 1,6M Lösung von n-Butyllithium in Hexan werden unter Argon bei -70° 1,2 g (R)-α-(Pivaloylmethyl)hydrozimtsäure in 50 ml Tetrahydrofuran getropft. Nach 30-minütigem Rühren werden mittels eines Pulvertrichters 6 g Molybdänperoxid [$MoO_5$ • Pyridin • HMPA; E. Vedejes et al., J. Org. Chem., 43, 188 (1978)] zugegeben. Innerhalb von 30 Minuten lässt man danach die Temperatur auf -15° ansteigen und versetzt die Reaktionsmischung mit 50 ml gesättigter Natriumsulfitlösung. Anschliessend lässt man das Reaktionsgemisch auf Raumtemperatur erwärmen und extrahiert mit Aether. Die Aetherphase wird nacheinander mit Wasser und Natriumchloridlösung gewaschen, über Natriumsulfat getrocknet und unter vermindertem Druck eingedampft. Der oelige Rückstand (1 g) wird mit ätherischer Diazomethanlösung umgesetzt. Nach dem Eindampfen der Reaktionslösung erfolgt die Reinigung und Trennung der beiden diastereo meren Ester mittels Chromatographie an Kieselgel unter Verwendung eines 4:1-Gemisches von Hexan und Aether. Man erhält auf diese Weise 325 mg des weniger polaren Methyl (2R,3R oder S)-2-benzyl-3-hydroxy-5,5-dimethyl-4-oxohexanoats und 225 mg des polareren Methyl (2R,3S oder R)-2-benzyl-3-hydroxy-5,5-dimethyl-4-oxohexanoats in Form von farblosen Festkörpern.

Ein Gemisch von 300 mg (1,07 mMol) des weniger polaren Methyl (2R,3R oder S)-2-benzyl-3-hydroxy-5,5-dimethyl-4-oxohexanoats und 1,07 ml 1N Natronlauge in 10 ml Methanol wird 3 Stunden bei Raumtemperatur gerührt. Danach wird das Reaktionsgemisch mit 1,07 ml 1N Salzsäure neutralisiert und unter vermindertem Druck eingedampft. Der Rückstand wird mit Essigester angerieben, und der ungelöste Festkörper abgetrennt. Nach dem Eindampfen der Essigesterphase erhält man 180 mg (2R,3R oder S)-2-Benzyl-3-hydroxy-5,5-dimethyl-4-oxohexansäure als farblosen Festkörper, MS: 231 $(M-CH_3-H_2O)^+$.

In analoger Weise wurde aus Methyl (2R,3S oder R)-2-benzyl-3-hydroxy-5,5-dimethyl-4-oxohexanoat die epimere (2R,3S oder R)-2-Benzyl-3-hydroxy-5,5-dimethyl-4-oxohexansäure erhalten, MS: 231 $(M-CH_3-H_2O)^+$.

2-(RS)-Benzyl-3-morpholinocarbonylpropionsäure

1,0 g (4,2 mMol) 3-Aethoxycarbonyl-4-phenylbuttersäure werden in 25 ml Dimethylformamid gelöst und mit 0,37 g (4,2 mMol) Morpholin, 0,81 g (4,2 mMol) EDC und 1,3 g (8,4 mMol) HOBT versetzt und 48 Stunden bei Raumtemperatur gerührt. Danach wird die Reaktionslösung im Hochvakuum eingedampft, und der Rückstand in Essigester gelöst und nacheinander mit Wasser, gesättigter Natriumbicarbonatlösung und gesättigter Kochsalzlösung gewaschen. Dann wird die organische Phase über Natriumsulfat getrocknet und unter vermindertem Druck eingedampft, und der Rückstand unter Verwendung eines 95:5- Gemisches von Methylenchlorid und Aethanol an Kieselgel chromatographiert, wobei man 2-(RS)-1-Benzyl-3-morpholinocarbonylpropionsäureäthylester als farbloses Oel erhält, MS: 305 $(M)^+$.

0,42 g (1,4 mMol) des obigen Esters werden in 2 ml Aethanol gelöst und mit 2,1 ml (1,5 Moläquivalente) 1N Natronlauge versetzt. Die Reaktionslösung wird dann 4 Stunden bei 50° gerührt, und der Rückstand in Wasser gelöst und mit Aether gewaschen. Die wässrige Phase wird mit 2,3 ml 1N Salzsäure angesäuert und mit Methylenchlorid extrahiert. Die organischen Extrakte werden über Natriumsulfat getrocknet und unter vermindertem Druck eingedampft, wobei man 2-(RS)-Benzyl-3-morpholinocarbonylpropionsäure als farbloses Oel erhält, welches direkt in die nächste Stufe eingesetzt wird, MS: 277 $(M)^+$.

(RS)-t-Butylsulfonylmethyl-1-naphtalinpropionsäure und (RS)-t-Butylsulfonyl-4-phenylbuttersäure

Diese Verbindungen wurden in Analogie zu der in EPA 0.236.734 beschriebenen Synthese von (RS)-α-[(t-Butylsulfonyl)methyl]hydrozimtsäure ausgehend von 1-Naphthylmalonsäurediäthylester bzw. Phenyläthylmalonsäurediäthylester hergestellt. Die beiden Verbindungen weisen die folgenden Massenspektren auf:
(RS)-[(t-Butylsulfonyl)methyl]-1-naphtalinpropionsäure, MS: 334 $(M)^+$ und
(RS)-(t-Butylsulfonylmethyl)-4-phenylbuttersäure, MS: 298 $(M)^+$.

## Beispiel 5

50 mg (S)-α-[(S)-α-[(t-Butylsulfonyl)methyl]hydrocinnamamido]-N-[(1S,2R,3S)-1-(cyclohexylmethyl)-2,3-dihydroxy-4-methylpentyl]imidazol-4-propionamid und 50 mg p-Toluolsulfonsäure in 3 ml Dimethoxypropan werden über Nacht bei Raumtemperatur gerührt. Das Reaktionsgemisch wird danach auf 2N Natriumbicarbonatlösung gegossen und mit Essigester extrahiert. Die organischen Phasen werden mit Wasser gewaschen, getrocknet und eingedampft, wobei man (S)-α-[(S)-α-[(t-Butylsulfonyl)methyl]hydrocinnamamido]-N-[(S)-(cyclohexylmethyl)[(4R,5S)-5-isopropyl-2,2-dimethyl-1,3-dioxolan-4-yl]methyl]imidazol-4-propionamid als Festkörper erhält, MS: 673 $(M+H)^+$.

## Beispiel 6

100 mg (S)-α-[(S)-α-[(t-Butylsulfonyl)methyl]hydrocinnamamido]-N-[(1S,2R,3S)-1-(cyclohexylmethyl)-2,3-dihydroxy-4-methylpentyl]imidazol-4-propionamid und 60 mg Dimethylaminopyridin in 5 ml Pyridin und 0,2 ml Valerylchlorid werden über Nacht bei Raumtemperatur stehen gelassen. Danach wird das Reaktionsgemisch in Essigester aufgenommen, und die organische Phase nacheinander mit 2N Natriumcarbonatlösung, 2N Kupfersulfatlösung und Wasser gewaschen, getrocknet und eingedampft. Chromatographie des Rückstands an 15 g Kieselgel mit einem 200:10:1-Gemisch von Methylenchlorid, Methanol und Ammoniak liefert (1R,2S)-1-[(S)-1-[(S)-α-[(S)-α-[(t-Butylsulfonyl)methyl]hydrocinnamamido]imidazol-4-propionamido]-2-cyclohexyläthyl]-2-isopropyläthylendivalerat als Schaum, MS: 802 $(M+H)^+$, und (1S,2R,3S)-3-[(S)-α-[(S)-α-[(t-Butylsulfonyl)methyl]hydrocinnamamido]imidazol-4-propionamido]-4-cyclohexyl-2-hydroxy-1-isopropylbutylvalerat als Oel, MS: 717 $(M+H)^+$.

## Beispiel 7

100 mg (S)-α-[(S)-α-[(t-Butylsulfonyl)methyl]hydrocinnamamido]-N-[(1S,2R,3S)-1-(cyclohexylmethyl)-2,3-dihydroxy-4-methylpentyl]imidazol-4-propionamid, 200 mg Bernsteinsäureanhydrid und 200 mg Natriumcarbonat in 10 ml Dimethylformamid werden über Nacht bei 50° gerührt. Danach wird das Reaktionsgemisch in Essigester aufgenommen, und die organische Phase mit gesättigter Ammoniumchloridlösung und Wasser gewaschen getrocknet und eingedampft. Chromatographie des Rückstands an 20 g Kieselgel mit einem 90:10:1:0.5-Gemisch von Methylenchlorid, Methanol, Wasser und Essigsäure liefert (1S,2R,3S)-3-[(S)-α-[(S)-α-[(t-Butylsulfonyl)methyl]hydrocinnamamido]imidazol-4-propionamido]-4-cyclohexyl-1-isopropylbutylhydrogensuccinat als Schaum, MS: 733 $(M+H)^+$, und (1R,2S)-1-[(S)-1-[(S)-α-[(S)-α-[(t-Butylsulfonyl)methyl]hydrocinnamamido]imidazol-4-propionamido]-2-cyclohexyläthyl]-2-isopropyläthylen-bis-(hydrogen succinat) in Form eines Schaums, MS: 833 $(M+H)^+$.

## Beispiel 8

214 mg (S)-α-[(S)-α-[(t-Butylsulfonyl)methyl]hydrocinnamamido]-N-[(1S,2R,3S)-1-(cyclohexylmethyl)-2,3-dihydroxy-4-methylpentyl]imidazol-4-propionamid in 5 ml Pyridin und 5 ml Essigsäureanhydrid werden 2 Stunden auf 90° erhitzt und danach eingedampft, wobei man (1R,2S)-1-[(S)-1-[(S)-α-[(S)-α-[(t-Butylsulfonyl)-

methyl]hydrocinnamamido]imidazol-4-propionamido]-2-cyclohexyläthyl]-2-isopropyläthylendiacetat in Form eines Schaums als Rückstand erhält, Rf-Wert 0,2 in einem 140:10:1-Gemisch von Methylenchlorid, Methanol und Ammoniak.

## Beispiel 9

100 mg (S)-α-[(S)-α-[(t-Butylsulfonyl)methyl]hydrocinnamamido]-N-[(1S,2R,3S)-1-(cyclohexylmethyl)-2,3-dihydroxy-4-methylpentyl]imidazol-4-propionamid in 5 ml Pyridin, 2,5 ml Essigsäureanhydrid und 2,5 ml Ameisensäure werden 2 Stunden auf 90° erhitzt. Abdampfen der Lösungsmittel und Chromatographie an 20 g Kieselgel mit einem 140:10:1-Gemisch von Methylenchorid, Methanol und Ammoniak liefert (1R,2S)-1-[(S)-1-[(S)-α-[(S)-α-[(t-Butylsulfonyl)methyl]hydrocinnamamido]imidazol-4-propionamido]-2-cyclohexyläthyl]-2-isopropyläthylendiformat als weissen Festkörper, MS: 689 $(M+H)^+$.

## Beispiel 10

Zu 100 mg (S)-α-[(S)-α-[(t-Butylsulfonyl)methyl]hydrocinnamamido]-N-[(1S,2R,3S)-1-(cyclohexylmethyl)-2,3-dihydroxy-4-methylpentyl]imidazol-4-propionamid in 2 ml Pyridin wird bei 0° eine Mischung von 1 ml Essigsäureanhydrid und 1 ml Ameisensäure zugegeben. Das Reaktionsgemisch wird über Nacht bei Raumtemperatur stehen gelassen und danach am Rotationsverdampfer bei einer Badtemperatur von ungefähr 30° eingedampft. Der Rückstand wird in Aether gelöst, und die organische Lösung nacheinander mit 2N Natriumbicarbonatlösung, 2N Kupfersulfatlösung und Wasser gewaschen, getrocknet und eingedampft, wobei man (1S,2R,3S)-3-[(S)-α-[(S)-α-[(t-Butylsulfonyl)methyl]hydrocinnamamido]imidazol-4-propionamido]-4-cyclohexyl-2-hydroxy-1-isopropylbutylformat als Schaum erhält, MS: 661 $(M+H)^+$.

## Beispiel 11

130 mg (S)-α-[(S)-α-[(t-Butylsulfonyl)methyl]hydrocinnamamido]-N-[(1S,2R,3S)-1-(cyclohexylmethyl)-2,3-dihydroxy-4-methylpentyl]imidazol-4-propionamid in 130 ml p-Touolsufonsäure in 1 ml Trimethylacetaldehyd und 10 ml Methylenchlorid werden 24 Stunden bei Raumtemperatur stehen gelassen. Danach wird das Reaktionsgemisch in Aether aufgenommen, und die organische Phase nacheinander mit 2N Natriumbircarbonatlösung und Wasser gewaschen, getrocknet und eingedampft. Chromatographie des Rückstands an 30 g Kieselgel unter Verwendung eines 200:10:1-Gemisches von Methylenchlorid, Methanol und Ammoniak liefert (S)-N-[(S)-1-[(2R oder S,4R,5S)-2-t-Butyl-5-isopropyl-1,3-dioxolan-4- yl]-2-cyclohexyläthyl]-α-[(S)-α-[(t-butylsulfonyl)methyl]hydrocinnamamido]imidazol-4-propionamid als Schaum, MS: 701 $(M+H)^+$.

In analoger Weise wie oben beschrieben, wurde aus (S)-α-[(S)-α-[(t-Butylsulfonyl)methyl]-hydrocinnamamido]-N-[(1S,2R,3S)-1-(cyclohexylmethyl)-3-cyclopropyl-2,3-dihydroxypropyl]imidazol-4-propionamid und Trimethylacetaldehyd das (S)-N-[(S)-1-[(4R,5S)-2-t-Butyl-5-cyclopropyl-1,3-dioxolan-4-yl]-2-cyclohexyläthyl]-α-[(S)-α-[(t-butylsulfonyl)methyl]hydrocinnamamido]imidazol-4-propionamid hergestellt, MS: 699 $(M+H)^+$.

## Beispiel 12

100 mg (S)-α-[(S)-α-[(t-Butylsulfonyl)methyl]hydrocinnamamido]-N-[(1S,2R,3S)-1-(cyclohexylmethyl)-2,3-dihydroxy-4-methylpentyl]imidazol-4-propionamid in 2 ml Pyridin werden bei 0° mit 0,2 ml Methoxyacetylchlorid versetzt und über Nacht bei Raumtemperatur stehen gelassen. Eindampfen des Reaktionsgemisches und Chromatographie des Rückstands an 20 g Kieselgel mit einem 140:10:1-Gemisch von Methylenchlorid, Methanol und Ammoniak liefert (1S,2R)-[(S)-1-[(S)-α-[(S)-α-[(t-Butylsulfonyl)methyl]-hydrocinnamamido]imidazol-4-propionamido]-2-cyclohexyläthyl]-1-isopropyläthylen-bis(methoxyacetat) als Schaum, MS: 777 $(M+H)^+$.

In analoger Weise wie oben beschrieben, wurde aus (S)-α-[(S)-α-[(t-Butylsulfonyl)methyl]-

hydrocinnamamido]-N-[(1S,2R,3S)-1-(cyclohexylmethyl)-3-cycloproyl-2,3-dihydroxypropyl]imidazol-4-propionamid und Methoxyessigsäurechlorid das (1R,2S)-1-[(S)-1-[[N-[(S)-α-[(t-Butylsulfonyl)methyl]-cinnamoyl]-L-histidyl]amino]-2-cyclohexyläthyl]-2-cyclopropyläthylen-bis-(methoxyacetat) hergestellt, MS: 755 $(M+H)^+$.

## Beispiel 13

Ein Gemisch von 100 mg (0,37 mMol) (1S oder R,2R,3S)-3-Amino-4-cyclohexyl-1-(1-cyclohexan-1-yl)-1,2-butandiol und 257 mg (0,41 mMol) N-[(S)-α-[(t-Butylsulfonyl)methyl]hydrocinnamoyl]-3-(2,4-dinitrophenyl)-L-histidin in 15 ml Acetonitril wird unter Argon nacheinander bei Raumtemperatur mit 83 mg (0,82 mMol) Triäthylamin, 67 mg (0,41 mMol) HOBT und 168 mg (0,41 mMol) HBTU versetzt, und das Reaktionsgemisch danach 3 Stunden bei Raumtemperatur gerührt. Zur Aufarbeitung wird das Reaktionsgemisch unter vermindertem Druck auf ca. 1/4 des ursprünglichen Volumens eingeengt, dann mit 50 ml Essigester verdünnt und nacheinander mit Wasser, gesättigter Natriumbicarbonatlösung, Wasser und gesättigter Natriumchloridlösung gewaschen. Die organische Phase wird über Natriumsulfat getrocknet und unter vermindertem Druck eingedampft, und der Rückstand mit einem 98:2-Gemisch von Methylenchlorid und Methanol als Eluierungsmittel an Kieselgel chromatographiert, wobei 140 mg (0,17 mMol) (S)-α-[(S)-α-[(t-Butylsulfonyl)methyl]hydrocinnamido]-N-[(1S,2R,3S oder R)-1-(cyclohexylmethyl)-3-(1-cyclohexen-1-yl)-2,3-dihydroxypropyl]-1-(2,4-dinitrophenyl)imidazol-4-propionamid in Form eines gelben Schaums erhalten werden, MS:838 $(M+H)^+$. Dieser wird in 3 ml Dimethylformamid gelöst und mit 0,15 ml (1,7 mMol) Thiomilchsäure 3 Stunden bei Raumtemperatur gerührt. Danach wird die Reaktionslösung im Hochvakuum eingedampft, der Rückstand in 15 ml Essigester gelöst, und die Reaktionslösung nacheinander mit gesättigter Natriumcarbonatlösung, Wasser und gesättigter Natriumchloridlösung gewaschen. Die gelblich gefärbte Essigesterphase wird über Natriumsulfat getrocknet und unter vermindertem Druck eingedampft. Zur Reinigung wird der Rückstand (120 mg) an Kieselgel mit einem 95:5-Gemisch von Methylchlorid und Methanol, welches 0,1% Ammoniumhydroxyd enthält, als Eluierungsmittel chromatographiert. Nach dem Lyophilisieren aus Dioxan werden 85 mg (S)-α-[(S)-α-[(t-Butylsulfonyl)methyl]hydrocinnamamido]-N-[(1S,2R,3S)-3-(1-cyclohexen-1-yl)-1-(cyclohexylmethyl)-2,3-dihydroxypropyl]imidazol-4-propionamid als gelbliches Pulver erhalten, MS: 671 $(M+H)^+$.

Das als Ausgangsstoff eingesetzte (1S oder R,2R,3S)-3-Amino-4-cyclohexyl-1-(cyclohexen-1-yl)-1,2-butandiol wurde wie folgt hergestellt:

Man kühlt 54,5 g (228 mMol) (4S,5RS)-4-(Cyclohexylmethyl)-5-vinyl-2-oxazolidinon [vgl. J. Med. Chem., 30, 1729 (1987)] in 800 ml Methanol auf -78° ab und leitet 3,5 Stunden Ozongas (3 g/Stunde) bis zur auftretenden Blaufärbung durch die Lösung. Anschliessend wird noch 10 Minuten Ozongas (1g/Stunde), 5 Minuten Sauerstoffgas und 20 Minuten Argon durch Reaktionsgemisch geleitet, wobei sich dieses wieder entfärbt. Anschliessend gibt man 50 ml Dimethylsufid zu, entfernt das Kühlbad und rührt 1 Stunde bei Raumtemperatur. Dann lässt man das Reaktionsgemisch über Nacht im Kühlschrank stehen, rührt danach nochmals 30 Minuten und dampft zur Trockene ein. Der Rückstand wird dreimal mit je 600 ml Aether extrahiert, und die organische Phase zweimal mit je 300 ml Wasser gewaschen, über Magnesiumsulfat getrocknet, filtriert und eingedampft, wobei man 54,5 g eines Schaums erhält. Dieser Schaum wird in 150 ml Methanol gelöst und mit 31,51 g (228 mMol) Kaliumcarbonat versetzt und 3 Stunden bei Raumtemperatur unter Argon gerührt. Anschliessend giesst man das Reaktionsgemisch auf ein Gemisch von Eis und Wasser (300 ml), extrahiert dreimal mit je 600 ml Essigester, wäscht die organische Phase mit gesättigter Natriumchloridlösung, trocknet über Magnesiumsulfat, filtriert und dampft ein, wobei 48,5 g eines Schaums erhalten werden. Dieses Rohprodukt wird an 1,5 kg Kieselgel mit Essigester als Eluierungsmittel chromatographiert, wobei man 34,3 g (71%) (4S,5R)-4-(Cyclohexylmethyl)-2-oxo-5-oxazolidincarboxaldehyd als Schaum erhält MS: 211 $(M)^+$, Rf-Wert 0,15 in Essigester.

Zu einer Lösung von 5,88 g (36,5 mMol) 1-Bromcyclohexen [vgl. J. Org. Chem., 45, 5396 (1980)] in 40 ml Tetrahydrofuran werden bei -78° mit einer Spritze 65 ml (91,3 mMol) einer 1,4M Lösung von t-Butyllithium in Pentan gegeben. Nach beendeter Zugabe wird 30 Minuten bei 0° gerührt und anschliessend wieder auf -78° abgekühlt. Zu dieser gekühlten Reaktionslösung werden 1,46 g (6,9 mMol) (4S,5R)-4-(Cyclohexylmethyl)-2-oxo-5-oxazolidincarboxaldehyd, gelöst in 20 ml Tetrahydrofuran, getropft. Nach beendeter Zugabe lässt man das Reaktionsgemisch langsam auf Raumtemperatur erwärmen und rührt 3 Stunden weiter. Zur Aufarbeitung wird das Reaktionsgemisch unter vermindertem Druck eingeengt, mit Essigester verdünnt und nacheinander mit Ammoniumchlorid-und gesättigter Natriumchloridlösung gewaschen. Die organische Phase wird über Natriumsulfat getrocknet und unter vermindertem Druck eingedampft, und der Rückstand mit Aether als Eluierungsmittel an Kieselgel chromatographiert. Man erhält auf diese Weise 310 mg des weniger polaren (4S,5R)-4-(Cyclohexylmethyl)-5-[(S oder R)-1-(1-cyclohexen-1-yl)-hydroxymethyl]-2-oxazolidinons sowie 250 mg des polareren (4S,5R)-4-(Cyclohexylmethyl)-5-[(R oder S)-1-(1-cyclohexen-1-yl)hydroxymethyl]-2-oxazolidinons, MS für beide Epimere: 294 $(M+H)^+$.

Ein Gemisch von 310 mg (1,05 mMol) des oben genannten, weniger polaren Epimeren und 668 mg (2,1 mMol) Bariumhydroxyd [$Ba(OH_2 \bullet 8H_2O$] in 20 ml Dioxan und 20 ml Wasser wird 12 Stunden zum Rückfluss erhitzt. Nach dem Abkühlen auf Raumtemperatur wird Kohlendioxyd in das Reaktionsgemisch eingeleitet, der Niederschlag anschliessend abfiltriert und mit heissem Dioxan gewaschen. Die vereinigten Dioxanlösungen werden unter vermindertem Druck eingedampft, und der erhaltene Rückstand an Kieselgel mit einem 95:5-Gemisch von Methylenchlorid und Methanol, welches 0,1% Ammoniumhydroxyd enthält, chromatographiert. Man erhält auf diese Weise 115 mg (1S oder R,2R,3S)-3-Amino-4-cyclohexyl-1-(1-cyclohexen-1-yl)-1,2-butandiol als farblosen Festkörper, MS: 268 $(M+H)^+$.

Das als Ausgangsstoff eingesetze N-[(S)-α-[(t-Butylsulfonyl)methyl]hydrocinnamoyl]-3-(2,4-dinitrophenyl)-L-histidin wurde wie folgt hergestellt:

15,8 g (65,5 mMol) L-Histidinmethylester-dihydrochlorid, 18,6 g (S)-α-[(t-Butylsulfonyl)methyl]-hydrozimtsäure, 28,9 g BOP und 35,8 ml Hünigbase werden in 400 ml Aectonitril gelöst und anschliessend 12 Stunden bei Raumtemperatur gerührt. Nach üblicher Aufarbeitung wird das Rohprodukt durch Flash-Chromatographie an Kieselgel gereinigt, wobei man 28 g (98%) Methyl (S)-α-[(S)-α-[(t-butylsulfonyl)methyl]-hydrocinnamamido]imidazolpropionat als Harz erhält, Rf-Wert 0,25 in einem 20:1-Gemisch von Methylenchlorid und Methanol, MS: 435 $(M)^+$.

23,3 g (54,6 mMol) Methyl (S)-α-[(S)-α-[(t-butylsulfonyl)methyl]hydrocinnamamido]imidazolpropionat in 250 ml Methylenchlorid werden mit 7,43 ml (54,6 mMol) Triäthylamin versetzt. Anschliessend werden unter Eiskühlung 9,94 g (54,6 mMol) 2,4-Dinitro-1-fluorobenzol in 100 ml Methylenchlorid innerhalb von etwa 20 Minuten zugetropft, und das Reaktionsgemisch bis zur vollständigen Umsetzung bei Raumtemperatur gerührt, wobei dies nach 4 Stunden der Fall ist (dünnschichtchromatographisch überprüft). Uebliche Aufarbeitung des Reaktionsgemisches liefert 20,5 g (62%) N-[(S)-α-[(t-Butylsulfonyl)methyl]-hydrocinnamoyl]-1-(2,4-dinitrophenyl)-L-histidinmethylester als braunen Schaum, Rf-Wert 0,4 in einem 30:1-Gemisch von Methylenchlorid und Methanol, MS: 602 $(M+H)^+$.

20,5 g (34,07 mMol) N-[(S)-α-[(t-Butylsulfonyl)methyl]hydrocinnamoyl]-1-(2,4-dinitrophenyl)-L-histidinmethylester werden in 180 ml Dioxan gelöst, mit 85 ml (170,34 mMol) 2N Salzsäure versetzt und anschliessend 2,5 Stunden auf 80° erhitzt. Uebliche Aufarbeitung und Kristallisation aus Aether/Hexan liefert 15,7 g (78%) N-[(S)-α-[(t-Butylsulfonyl)methyl]hydrocinnamoyl]-3-(2,4-dinitrophenyl)-L-histidin in Form eines hellgelben amorphen Festkörpers, Rf-Wert 0,2 in einem 5:1-Gemisch von Methylenchlorid und Methanol, MS: 588 $(M+H)^+$.

## Beispiel 14

In analoger Weise wie in Beispiel 13 beschrieben wurden die folgenden Verbindungen hergestellt:

- Aus N-[(S)-α-[(t-Butylsulfonyl)methyl]hydrocinnamoyl]-3-(2,4-dinitrophenyl)-L-histidin und (2S,3R,4R oder S,Z)-2-Amino-1-cyclohexyl-5-methyl-5-hepten-3,4-diol das (S)-α-[(S)-α-[(t-Butylsulfonyl)methyl]-hydrocinnamido]-N-[(1S,2R,3R oder S,Z)-1-(cyclohexylmethyl)-2,3-dihydroxy-4-methyl-4-hexenyl]imidazol-4-propionamid als gelblicher Festkörper, MS: 671 $(M+H)^+$;
- aus N-[(S)-α-[(t-Butylsulfonyl)methyl]hydrocinnamoyl]-3-(2,4-dinitrophenyl)-L-histidin und (3S,4R,5S)-5-Amino-6-cyclohexyl-2-methyl-1-hexen-3,4-diol das (S)-α-[(S)-α-(t-Butylsulfonyl)methyl]hydrocinnamamido]-N-[(1S,2R,3S)-1-(cyclohexylmethyl)-2,3-dihydroxy-4-methyl-4-pentenyl]imidazol-4-propionamid als farbloser Festkörper, MS: 631 $(M+H)^+$;
- aus N-[(S)-α-[(t-Butylsulfonyl)methyl]hydrocinnamoyl]-3-(2,4-dinitrophenyl)-L-histidin und (3R,4R,5S)-5-Amino-6-cyclohexyl-2-methyl-1-hexen-3,4-diol das (S)-α-[(S)-α-[(t-Butylsulfonyl)methyl]hydrocinnamamido]-N-[(1S,2R,3R)-1-(cyclohexylmethyl)-2,3-dihydroxy-4-methyl-4-pentenyl]imidazol-4-propionamid als farbloser Festkörper, MS: 631 $(M+H)^+$.

Die als Ausgangstoffe eingesetzten Amine wurden wie folgt hergestellt:

(2S,3R,4R oder S,Z)-2-Amino-1-cyclohexyl-5-methyl-5-heptene-3,4-diol

Zu einer Lösung der aus 18,6 g (137 mMol) 2-Brom-2-buten (E/Z = 2:3) und 3,34 g (137 mGrammatome) Magnesiumspänen in 90 ml Tetrahydrofuran hergestellten Grignardver bindung wird bei 0° eine Lösung von 7,2 g (34 mMol) (4S,-5R)-4-(Cyclohexylmethyl)-2-oxo-5-oxazolidindcarboxaldehyd in 60 ml Tetrahydrofuran getropft. Man lässt das Reaktionsgemisch auf Raumtemperatur erwärmen und rührt weitere 15 Stunden. Zur Aufarbeitung wird das Reaktionsgemisch mit 100 ml eiskalter, gesättigter Ammoniumchloridlösung hydrolysiert, und die organische Phase abgetrennt. Die wässrige Phase wird zweimal mit je 300 ml Aether extrahiert. Die vereinigten organischen Phasen werden über Natriumsulfat getrocknet und unter vermindertem Druck eingedampft. Der Rückstand wird zur Reinigung an Kieselgel mit einem 3:1-Gemisch von Toluol und Essigester als Eluierungsmittel chromatographiert. Man erhält auf diese Weise drei Fraktionen bestehend aus den beiden Epimeren bzw. dem Epimerengemisch in der Reihenfolge zunehmender Polarität: 1,7 g (4S,5R)-4-(Cyclohexylmethyl)-5-[(S oder R,Z)-1-hydroxy-2-methyl-2-butenyl]-2-oxazolidinon, MS: 206 $(M-NH_2-COOH)^+$, 0,7 g (4S,-5R)-4-(Cyclohexylmethyl)-5-[(R oder S,E oder Z)-1-hydroxy-2-methyl-2-butenyl]-2-oxazolidinon, MS: 268 $(M+H)^+$, und 1,5 g (4S,5R)-4-(Cyclohexylmethyl)-5-[(R oder S,E oder Z)-1-hydroxy-2-methyl-2-butenyl]-2-oxazolidinon, MS: 268 $(M+H)^+$, jeweils als farblose Festkörper.

Ein Gemisch von 800 mg (3 mMol) (4S,5R)-4-(Cyclohexylmethyl)-5-[(S oder R,Z)-1-hydroxy-2-methyl-2-butenyl[-2-oxazolidinon, und 1,89 g (6 mMol) Bariumhydroxyd $[Ba(OH)_2 \cdot 8H_2O]$ in 20 ml Dioxan und 20 ml Wasser wird 6 Stunden zum Rückfluss erhitzt. Nach dem Abkühlen auf Raumtemperatur wird Kohlendioxyd in das Reaktionsgemisch eingeleitet, und der gebildete Niederschlag abfiltriert und mit heissem Dioxan gewaschen. Die vereinigten organischen Phasen werden unter vermindertem Druck eingedampft, und der verbleibende Rückstand mit einem 95:5-Gemisch von Methylenchlorid und Methanol, welches 0,1% Ammoniumhydroxyd enthält, an Kieselgel chromatographiert, wobei 461 mg (2S,3R,4R oder S,Z)-2-Amino-1-cyclohexyl-5-methyl-5-hepten-3,4-diol als farbloser Festkörper erhalten werden, MS: 242 $(M+H)^+$.

In analoger Weise wie oben beschrieben wird (4S,5R)-4-(Cyclohexylmethyl)-2-oxo-5-oxazoldincarboxaldehyd in einer Grignardreaktion mit Propenyl-2-magnesiumbromid zu den beiden epimeren Verbindungen (4S,5R)-4-(Cyclohexylmethyl)-5-[(S)-1-hydroxy-2-methylallyl]-2-oxazolidinon und (4S,5R)-4-(Cyclohexylmethyl)-5-[(R)-1-hydroxy-2-methylallyl]-2-oxazolidinon umgesetzt, welche nach Auftrennung zu (3S,4R,5S)-5-Amino-6-cyclohexyl-2-methyl-1-hexen-3,4-diol bzw. der epimeren Verbindung (3R,4R,5S)-5-Amino-6-cyclohexyl-2-methyl-1-hexen-3,4-diol hydrolysiert werden.

## Beispiel 15

In analoger Weise wie in Beispiel 13 beschrieben wurde durch Umsetzen von (1RS,2R,3S)-3-Amino-4-cyclohexyl-1-(2-furyl)-1,2-butandiol mit N-[(S)-α-[(t-Butylsulfonyl)methyl]hydrocinnamoyl]-3-(2,4-dinitrophenyl)-L-histidin das (S)-α-[(S)-α-[(t-Butylsulfonyl)methyl]hydrocinnamamido]-N-[(1S,2R,3RS)-1-(cyclohexylmethyl)-2,3-dihydroxy-3-(2-furyl)propyl]imidazol-4-propionamid (1:1 Epimerengemisch) als hellgelber Festkörper erhalten, MS: 657 $(M+H)^+$. Durch Chromatographie lässt sich das polarere epimere (S)-α-[(S)-α-[(t-Butylsulfonyl)methyl]hydrocinnamamido]-N[(1S,2R,3R oder S)-1-(cyclohexylmethyl)-2,3-dihydroxy-3-(2-furyl)propyl]imidazol-4-propionamid aus obigem Gemisch abtrennen, MS: 657 $(M+H)^+$.

Das als Ausgangsstoff eingesetzte (1RS,2R,3S)-3-Amino-4-cyclohexyl-1-(2-furyl)-1,2-butandiol wurde wie folgt hergestellt:

Umsetzung von (4S,5R)-4-(Cyclohexylmethyl)-2-oxo-5-oxazolidincarboxaldehyd mit 2-Lithiofuran (vgl. Chemistry Letters 1982, Seite 1169) liefert (4S,5R)-4-(Cyclohexylmethyl)-5-[(RS)-α-hydroxy-2-furfuryl]-2-oxazolidinon (1:1 Epimerengemisch) in Form eines Harzes, MS: 279 $(M)^+$. Basische Verseifung dieser Verbindung in analoger Weise wie in Beispiel 14 beschrieben, liefert (1RS,2R,3S)-3-Amino-4-cyclohexyl-1-(2-furyl)-1,2-butandiol als 1:1-Epimerengemisch, MS: 254 $(M+H)^+$.

## Beispiel 16

Man löst 750 mg (1,82 mMol) t-Butyl (4S,5R)-4-(cyclohexylmethyl)-5-[(R oder S)-hydroxy-(2-thiazolyl)-methyl]-2,2-dimethyl-3-oxazolidincarboxylat in 18 ml Methanol, fügt 6,2 ml 2N Salzsäure zu und erhitzt anschliessend 2 Stunden zum Rückfluss. Danach entfernt man das Lösungsmittel unter vermindertem Druck, nimmt den Rückstand in einem 10:1-Gemisch von Essigester und Methanol auf, trocknet die erhaltene Lösung über Natriumsulfat und dampft unter vermindertem Druck ein, wobei 500 mg (80%) des Amins als Dihydrochlorid in Form eines Harzes erhalten werden, welche ohne weitere Reinigung in die nächste Stufe eingesetzt werden.

Man löst 250 mg (0,73 mMol) das obigen Rohprodukts in 40 ml Acetonitril, gibt 323 mg (0,73 mMol) BOP, 354 mg (0,73 mMol) 1-(t-Butoxycarbonyl)-N-[(R)-α-(3,3-dimethyl-2-oxobutyl)hydrocinnamoyl)-L-histidin und 0,4 ml (2,33 mMol) Hünigbase zu und rührt die Reaktionslösung 14 Stunden bei Raumtemperatur. Nach üblicher Aufarbeitung erhält man 324 mg (60%) eines braun-gelben Harzes, welches in 10 ml Methanol gelöst, mit 20 mg Kaliumcarbonat versetzt und 2 Stunden bei Raumtemperatur gerührt wird. Nach üblicher Aufarbeitung erhält man ein Rohprodukt, welches zur Reinigung unter Verwendung eines 10:1-Gemisches von Methylenchlorid und Methanol als Eluierungsmittel an Kieselgel chromatographiert wird. Auf diese Weise erhält man 96,2 mg (34%) (S)-N-[(1S,2R,3S oder R)-1-(Cyclohexylmethyl)-2,3-dihydroxy-3-(2-thiazolyl)propyl]-α-[(R)-α-(3,3-dimethyl-2-oxobutyl)hydrocinnamamido]imidazol-4-propionamid als Harz, MS: 638 $(M+H)^+$.

In analoger Weise wie oben beschrieben wurden die folgenden Verbindungen hergestellt:

- Aus 1-(t-Butoxycarbonyl)-N-[(R)-α-(3,3-dimethyl-2-oxobutyl)hydrocinnamoyl]-L-histidin und t-Butyl (4S,5R)-4-(cyclohexylmethyl)-5-[(S oder R)-hydroxy-(2-thiazolyl)methyl]-2,2-dimethyl-3-oxazolidincarboxylat das (S)-N-[(1S,2R,3R oder S)-1-(Cyclohexylmethyl)-2,3-dihydroxy-3-(2-thiazolyl)propyl]-α-[(R)-α-3,3-dimethyl-2-oxobutyl)hydrocinnamamido]imidazol-4-propionamid als Harz, MS: 638 $(M+H)^+$;
- Aus 1-(t-Butoxycarbonyl)-N-[(R)-α-(3,3-dimethyl-2-oxobutyl)hydrocinnamoyl]-L-histidin und t-Butyl (4S,5R)-4-(cyclohexylmethyl)-2,2-dimethyl-5-[(αR oder S,2R oder S)-tetrahydro-2-hydroxy-2-furfuryl]-3-oxazolidincarboxylat bzw. t-Butyl (4S,5R)-4-(cyclohexylmethyl)-2,2-dimethyl-5-[(αR oder S,2S oder R)-tetrahydro-2-hydroxy-2-furfuryl]-3-oxazolidincarboxylat das (S)-N-[(1S,2R,3R oder S)-1-(Cyclohexylmethyl)-2,3-dihydroxy-3-[(R oder S)-tetrahydro-2-furyl]propyl]-α-[(R)-α-(3,3-dimethyl-2-oxobutyl)hydrocinnamamido]imidazol-4-propionamid sowie das epimere (S)-N-[(1S,2R,3R oder S)-1-(Cyclohexylmethyl)-2,3-dihydroxy-3-[(S oder R)-tetrahydro-2-furyl]propyl]-α-[(R)-α-(3,3-dimethyl-2-oxobutyl)hydrocinnamamido]imidazol-4-propionamid, beide jeweils als farblose armorphe Festkörper, MS (jeweils): 625 $(M+H)^+$.

Das als Ausgangstoff eingesetzte 1-(t-Butoxycarbonyl)-N-[(R)-α-(3,3-dimethyl-2-oxobutyl)-hydrocinnamoyl]-L-histidin wurde wie folgt hergestellt:

Eine Suspension von 3,0 g (12 mMol) (R)-α-(Pivaloylmethyl)hydrozimtsäure (vgl. EPA 0.184.550) und 2,66 g (11 mMol) L-Histidinmethylester-dihydrochlorid in 340 ml Dimethylformamid wird bei Raumtemperatur unter einer Stickstoffatmosphäre mit 3,45 g (34 mMol) Triäthylamin und 4,58 g (12 mMol) HBTU versetzt. Das Reaktionsgemisch wird 5 Stunden bei Raumtemperatur gerührt und anschliessend im Hochvakuum eingedampft. Der Rückstand wird in 500 ml Essigester gelöst und nacheinander mit 100 ml Wasser, dreimal je 100 ml gesättigter Natriumbicarbonatlösung und 100 ml gesättigter Natriumchloridlösung gewaschen. Die organische Phase wird über Natriumsulfat getrocknet, unter vermindertem Druck eingedampft, und das erhaltene gelbliche Rohprodukt an Kieselgel mit einem 95:5-Gemisch von Methylenchlorid und Methanol, welches 0,1% Ammoniak enthält, chromatographiert. Man erhält auf diese Weise 3,6 g N-[(R)-α-(3,3-Dimethyl-2-oxobutyl)hydrocinnamoyl]-L-histidinmethylester als farblosen Schaum, MS: 399 $(M)^+$.

Eine Lösung von 3,56 g (8,9 mMol) N-[(R)-α-3,3-Dimethyl-2-oxobutyl)hydrocinnamoyl]-L-histidinmethylester und 9,36 ml 1N Natronlauge in 50 ml Methanol wird 15 Stunden bei Raumtemperatur gerührt und danach in der Kälte unter vermindertem Druck eingedampft. Man löst den Rückstand in 70 ml Dioxan und 30 ml Wasser, tropft bei Raumtemperatur eine Lösung von 2,95 (13,5 mMol) Di-t-butyldicarbonat zu und rührt danach 15 Stunden bei Raumtemperatur. Zur Aufarbeitung wird die Reaktionslösung unter vermindertem Druck auf ca. 1/3 ihres Volumens eingeengt und dann mit 200 ml Essigester verdünnt. Nach Zugabe von 50 ml Eiswasser wird das Reaktionsgemisch auf pH 2,5 gestellt, und die wässrige Phase mit festem Natriumchlorid gesättigt. Die wässrige Phase wird noch zweimal mit Essigester extrahiert, und die vereinigten Essigesterphasen über Natriumsulfat getrocknet und eingedampft. Das erhaltene Rohpodukt wird an Kieselgel mit einem 95:5-Gemisch von Methylenchlorid und Methanol, welches 0,1% Essigsäure enthält, chromatographiert, wobei man 3,5 g 1-(t-Butoxycarbonyl)-N-[(R)-α-(3,3-dimethyl-2-oxobutyl)-hydrocinnamoyl]-L-histidin als farbloses Pulver erhält, MS: 486 $(M+H)^+$.

Die als Ausgangsstoffe eingesetzten t-Butyl (4S,5R)-4-(cyclohexylmethyl)-5-[(R oder S)-hydroxy-(2-thiazolyl)methyl]-2,2-dimethyl-3-oxazolidincarboxylat und t-Butyl (4S, 5R)-4-(cyclohexylmethyl)-5-[(S oder R)-hydroxy-(2-thiazolyl)methyl]-2,2-dimethyl-3-oxazolidincarboxylat wurden wie folgt hergestellt:

Man versetzt 14,7 g (51,86 mMol) t-Butyl (1S,2S:2R = 2:1)-1-(cyclohexylmethyl)-2-hydroxy-3-butenyl]-carbamat [J. Med. Chem., 30, 1729 (1987)] in 150 ml 2,2-Dimethoxypropan mit 832 mg (4,37 mMol) p-Toluolsulfonsäure-monohydrat und rührt die Reaktionslösung 3 Stunden bei Raumtemperatur. Danach wird mit 600 ml Essigester verdünnt, und die Reaktionslösung mit 2N Kaliumbicarbonatlösung gewaschen. Die organische Phase wird in üblicher Weise aufgearbeitet, und das erhaltene Rohprodukt durch Chromatographie an Kieselgel mit einem 80:1-Gemisch von Methylenchlorid und Essigester gereinigt, wobei man 11,5 g (68%) t-Butyl (4S,5S:5R = 2:1)-4-(cyclohexylmethyl)-2,2-dimethyl-5-vinyl-3-oxazolidincarboxylat erhält, MS:

308 $(M-CH_3)^+$; $R_f$-Wert 0,5 in einem 80:1-Gemisch von Methylenchlorid und Essigester.

Man löst 11,5 g (35,55 mMol) t-Butyl (4S,5S:5R = 2:1)-4-(cyclohexylmethyl)-2,2-dimethyl-5-vinyl-3-oxazolidincarboxylat in 200 ml Methanol, kühlt die Lösung auf -78° und leitet Ozongas bis zur Blaufärbung durch die Lösung (ca. 40 Minuten). Danach stellt man die Ozonzufuhr ab und leitet bei -75° solange Argon durch die Lösung bis die Blaufärbung wieder verschwindet (ca. 15 Minuten). Anschliessend gibt man bei -75° 5,0 ml (35,55 mMol) Dimethylsulfid zu, lässt das Reaktionsgemisch auf Raumtemperatur erwärmen und leitet noch weitere 2 Stunden Argon durch die Lösung, bevor man diese unter vermindertem Druck eindampft. Der erhaltene Rückstand (16 g einer hellgelben Flüssigkeit) wird in 260 ml Methanol gelöst, mit 3,05 g Kaliumcarbonat versetzt und 2 Stunden bei Raumtemperatur gerührt. Anschliessend wird zwischen Essigester und Wasser verteilt, die organische Phase in üblicher Weise aufgearbeitet, und das Rohprodukt an Kieselgel mit einem 20:1-Gemisch von Methylchlorid und Essigester als Eluierungsmittel chromatographiert, wobei man 7,2 g (62%) t-Butyl (4S,5R)-4-(cyclohexylmethyl)-5-formyl-2,2-dimethyl-3-oxazolidincarboxylat als gelbes Harz, MS: 310 $(M-CH_3)^+$; Rf-Wert 0,4 in einem 20:1-Gemisch von Methylenchlorid und Essigester.

Man kühlt 4,67 ml Thiazol (65,7 mMol) in 150 ml Tetrahydrofuran auf -78° und tropft bei dieser Temperatur 41,3 ml einer 1,6M Lösung von Butyllithium in Hexan (66,1 mMol) zu. Danach wird die rosarote Lösung noch 15 Minuten bei -78° gerührt und dann tropfenweise mit 5,37 g (16,5 mMol) t-Butyl (4S,5R)-4-(cyclohexylmethyl)-5-formyl-2,3-dimethyl-3-oxazolidincarboxylat versetzt. Danach entfernt man das Kühlbad und lässt das Reaktionsgemisch auf Raumtemperatur erwärmen, zersetzt es mit Wasser und extrahiert mit Essigester. Nach üblicher weiterer Aufarbeitung reinigt und isoliert man die beiden gebildeten Epimeren durch Chromatographie an Kieselgel unter Verwendung eines 4:1-Gemisches von Hexen und Essigester, wobei man 600 mg (9%) t-Butyl (4S,5R)-4-(cyclohexylmethyl)-5-[(R oder S)-hydroxy-(2-thiazolyl)methyl]-2,2-dimethyl-3-oxazolidincarboxylat (Rf-Wert 0,4 in einem 4:1-Gemisch von Aether und Hexan) und 1,4 g (20%) t-Butyl (4S,5R)-4-(cyclohexylmethyl)-5-[(S oder R)-hydroxy-(2-thiazolyl)methyl]-2,2-dimethyl-3-oxazolidincarboxylat (Rf-Wert 0,35 in einem 4:1-Gemisch von Aether und Hexan) jeweils als braune Feststoffe, MS (jeweils): 411 $(M)^+$.

Die als Ausgangstoffe eingesetzten t-Butyl (4S,5R)-4-(cyclohexylmethyl)-2,2-dimethyl-5-[(αR oder S,2R oder S)-tetrahydro-2-hydroxy-2-furfuryl[-3-oxazolidincarboxylat und t-Butyl (4S,5R)-4-(cyclohexylmethyl)-2,2-dimethyl-5-[(αR oder S,2S oder R)-tetrahydro-2-hydroxy-2-furfuryl]-3-oxazolidincarboxylat wurden wie folgt hergestellt:

In analoger Weise wie in Beispiel 15 beschrieben wurde t-Butyl (4S,5R)-4-(cyclohexylmethyl)-5-formyl-2,2-dimethyl-3-oxazolidincarboxylat mit 2-Lithiofuran umgesetzt, wobei ein 1:2-Epimerengemisch von t-Butyl (4S,5R)-4-(cyclohexylmethyl)-5-[(S oder R)-α-hydroxyfurfuryl]-2,2-dimethyl-3-oxazolidincarboxylat (Rf-Wert 0,5 in einem 20:1-Gemisch von Methylenchlorid und Methanol) und t-Butyl (4S,5R)-4-(cyclohexylmethyl)-5-[-(R oder S)-α-hydroxyfurfuryl]-2,2-dimethyl-3-oxazolidincarboxylat (Rf-Wert 0,45 in einem 20:1-Gemisch von Methylenchlorid und Methanol) als gelb gefärbte Kristalle erhalten werden, welche leicht chromatographisch aufgetrennt werden können, MS (jeweils): 322 $(M-CH_3\text{-Isobuten})^+$.

Man löst 500 mg t-Butyl (4S,5R)-4-(cyclohexylmethyl)-5-[(S oder R)-α-hydroxyfurfuryl]-2,2-dimethyl-3-oxazolidincarboxylat in 150 ml Aethanol, gibt 0,5 g Rhodium/Aluminiumoxyd zu und hydriert 48 Stunden in einem Autoklaven bei einem Druck von 10 bar und bei 50°. Danach wird der Katalysator abfiltriert, das Lösungsmittel unter vermindertem Druck abgedampft, und das Rohprodukt bestehend aus den beiden epimeren Verbindungen zur Auftrennung und Reinigung chromatographiert wobei man 170 mg (34%) t-Butyl (4S,5R)-4-(cyclohexylmethyl)-2,2-dimethyl-5-[(αR oder S,2S oder R)-tetrahydro-2-hydroxy-2-furfuryl]-3-oxazolidincarboxylat (Rf-Wert 0,5 in einem 1:1-Gemisch von Petroläther und Aether) und 176 mg (35%) t-Butyl (4S,5R)-4-(cyclohexylmethyl)-2,2-dimethyl-5-[(αR oder S,2S oder R)-tetrahydro-2-hydroxy-2-furfuryl]-3-oxazolidincarboxylat (Rf-Wert 0,4 in einem 1:1-Gemisch von Petroläther und Aether) erhält, MS (jeweils): 398 $(M+H)^+$.

## Beispiel 17

In analoger Weise wie in Beispiel 16 beschrieben wird durch Umsetzen von t-Butyl (4S,5R)-4-(cyclohexylmethyl)-5-[(R oder S)-hydroxy-[(R oder S)-tetrahydro-2-furyl]methyl]-2,2-dimethyl-3-oxazolidincarboxylat mit N-[(S)-α-t-Butylsulfonyl)methyl]hydrocinnamoyl]-3-(2,4-dinitrophenyl)-L-histidin das (S)-α-[(S)-α-[(t-Butylsulfonyl)methyl]hydrocinnamamido]-N-[(1S,2R,3R oder S)-1-(cyclohexylmethyl)-2,3-dihydroxy-3-[-(R oder S)-tetrahydro-2-furyl]propyl]imidazol-4-propionamid in Form eines gelben amorphen Pulvers erhalten, MS: 827 $(M+H)^+$. Die Abspaltung der Dinitrophenylgruppe mit Thiomilchsäure erfolgt in analoger

Weise zu Beispiel 13 und liefert (S)-α-[(S)-α-[(t-Butylsulfonyl) methyl]hydrocinnamamido]-N-[(1S,2R,3R oder S)-1-(cyclohexylmethyl)-2,3-dihydroxy-3-[(R oder S)-tetrahydro-2-furyl]propyl]imidazol-4-propionamid als hellgelber amorpher Festkörper, MS: 661 $(M+H)^+$.

Das als Ausgangsstoff eingesetzte geschützte Propionamid wurde wie folgt hergestellt:

In analoger Weise wie in Beispiel 16 beschrieben wurde t-Butyl (4S,5R)-4-(cyclohexylmethyl)-5-[(R oder S)-hydroxy-[(R oder S)-tetrahydro-2-furyl]methyl]-2,2-dimethyl-3-oxazolidincarboxylat und t-Butyl (4S,5R)-4-(cyclohexylmethyl)-5-[(R oder S)-hydroxy-[(R oder S)-tetrahydro-2-furyl]methyl]-2,2-dimethyl-3-oxazolidincarboxylat aus t-Butyl (4S,5R)-4-(cyclohexylmethyl)-5-[(R oder S)-hydroxyfurfuryl]-2,2-dimethyl-3-oxazolidincarboxylat hergestellt, wobei die beiden Epimeren aus dem erhaltenen 1:1-Epimerengemisch isoliert wurden, Rf-Wert 0,5 bzw. 0,4 in einem 1:1-Gemisch von Aether und Hexan, MS (jeweils): 398 $(M+H)^+$.

## Beispiel 18

In analoger Weise wie in den Beispielen 1 und 4 beschrieben wurden die folgenden Verbindungen hergestellt:

- Das (S)-N-[(1S,2RS,3RS,4SR)-5-(Benzyloxy)-1-(cyclohexylmethyl)-2,3-dihydroxy-4-methylpentyl]-α-[(S)-α-[(t-butylsulfonyl)methyl]hydrocinnamamido]imidazol-4-propionamid als Festkörper, Schmelzpunkt 89° (aus Methylenchlorid/Aether/Hexan) und das epimere (S)-N-[(1S,2S oder R,3S oder R,4SR)-5-(Benzyloxy)-1-(cyclohexylmethyl)-2,3-dihydroxy-4-methylpentyl]-α-[(S)-α-[(t-butylsulfonyl)methyl]hydrocinnamamido]-imidazol-4-propionamid als Festkörper, Schmelzpunkt 85° (aus Methylenchlorid/Aether/Hexan), wobei jedoch zur Herstellung des Grignardreagens rac. 3-Benzyloxy-2-methyl-1-propylbromid verwendet wurde;
- Das (S)-α-[(S)-α-[(t-Butylsulfonyl)methyl]hydrocinnamamido]-N-[(1S,2R,3S)-1-(cyclohexylmethyl)-3-cyclopentyl-2,3-dihydroxypropyl]imidazol-4-propionamid als Festkörper, Schmelzpunkt >125° (Zers.), wobei zur Herstellung des Grignardsreagens Cyclopentylbromid verwendet wurde;
- Das (R)-α-[(S)-α-[(t-Butylsulfonyl)methyl]hydrocinnamamido]-N-[(1S,2R,3S)-1-(cyclohexylmethyl)-3-cyclopropyl-2,3-dihydroxypropyl]-4-thiazolpropionamid als weisser Festkörper, Schmelzpunkt 75° (Zers.; aus Methylenchlorid/Aether/Hexan) und das epimere (S)-α-[(S)-α-[(t-Butylsulfonyl)methyl]-hydrocinnamamido]-N-[(1S,2R,3S)-1-(cyclohexylmethyl)-3-cyclopropyl-2,3-dihydroxypropyl]-4-thiazolpropionamid als weisser Festkörper, Schmelzpunkt 91° (Zers.; aus Methylenchlorid/Aether/Hexan), wobei zur Herstellung des Grignardreagens Cyclopropylbromid und anstelle von $(Fmoc)_2$His-OH Boc-(RS)-Thiazolylalanin verwendet wurde, welches seinerseits nach S. Rosenberg et al., J. Med. Chem., 30, 1224 (1987) hergestellt wurde;
- Das (S)-α-[(t-Butylsulfonyl)methyl]-N-[(S)-2-butoxy-1-[[(1S,2R,3S)-1-(cyclohexylmethyl)-2,3-dihydroxy-4-methylpentyl]carbamoyl]äthyl]hydrocinnamamid als weisser Festkörper, Schmelzpunkt 167° (aus Methylenchlorid/Aether/Hexan), wobei zur Herstellung des Grignardreagens Isopropylbromid und anstelle von $(Fmoc)_2$His-OH (Fmoc)Ser(O-t-butyl) verwendet wurde;
- Das (S)-α-[(S)-α-[(t-Butylsulfonyl)methyl]hydrocinnamamido]-N-[(1S,2R,3S)-1-(cyclohexylmethyl)-2,3-dihydroxy4-methyl]pentyl]-2-thiophenpropionamid als weisser Festkörper, Schmelzpunkt 176° (aus Methylenchlorid/Aether), wobei zur Herstellung des Grignardreagens Isopropylbromid und anstelle von $(Fmoc)_2$His-OH Boc-Thienylalanin verwendet wurde.

## Beispiel 19

Eine methanolische Lösung von (S)-N-[(1S,2R oder S,3R oder S,4SR)-5-(Benzyloxy)-1-(cyclohexylmethyl)-2,3-dihydroxy-4-methylpentyl]-α-[(S)-α-[(t-butylsulfonyl)methyl]hydrocinnamido]imidazol-4-propionamid wird mit 5%igem Palladium auf Kohle über Nacht in einer Wasserstoffatmosphäre gerührt. Danach wird der Katalysator abfiltriert, und das Filtrat eingeeingt, wobei man (S)-α-[(S)-α-[(t-Butylsulfonyl)-methyl]hydrocinnamamido]-N-[(1S,2S oder R,3S oder R,4SR)-1-(cyclohexylmethyl)-2,3,5-trihydroxypentyl]-imidazol-4-propionamid als Schaum erhält, MS: 649 $(M+H)^+$.

In analoger Weise erhält man aus (S)-N-[(1S,2S oder R,3S oder R,4SR)-5-(Benzyloxy)-1-(cyclohexylmethyl)-2,3-dihydroxy-4-methylpentyl]-α-[(S)-α-[(t-butylsulfonyl)methyl]hydrocinnamido]imidazol-4-propionamid das (S)-α-[(S)-α-[(t-Butylsulfonyl)methyl]hydrocinnamamido]-N-[(1S,2RS,3RS,4SR)-1-(cyclohexylmethyl)-2,3,5-trihydroxypentyl]imidazol-4-propionamid in Form eines Schaums, MS 649 $(M+H)^+$.

## Beispiel 20

In analoger Weise wie in Beispiel 10 beschrieben, wurde aus (S)-α-[(S)-α-[(t-Butylsulfonyl)methyl]-hydrocinnamamido]-N-[(1S,2R,3S)-1-(cyclohexylmethyl)-3-cyclopropyl-2,3-dihydroxypropyl]imidazol-4-propionamid und Chloressigsäureanhydrid/Triäthylamin anstelle von Essigsäureanydrid/Ameisensäure/Pyridin das (1S,2R)-2-[(S)-1-[(S)-α-[(S)-α-[(t-Butylsulfonyl)methyl]-hydrocinnamamido]imidazol-4-propionamido]-2-cyclohexyläthyl]-1-cyclopropyläthylen-bis(chloracetat) als Festkörper hergestellt, MS: 793 $(M+H)^+$. Diese Verbindung liess sich durch 90-minütiges Erhitzen zum Rückfluss in Diäthylamin in (1S,2R)-2-[(S)-1-[(S)-α-[(S)-α-[(t-Butylsulfonyl)methyl]hydrocinnamamido]-imidazol-4-propionamido]-2-cyclohexyläthyl]-1-cyclopropyläthylen-bis(diäthylaminoacetat) überführen, MS 857 $(M+H)^+$.

## Beispiel A

Eine sterilfiltrierte wässrige Lösung von (S)-α-[(S)-α-[(t-Butylsulfonyl)methyl]hydrocinnamamido]-N-[-(1S,2R,3S)-1-(cyclohexylmethyl)-2,3-dihydroxy -4-methylpentyl]imidazol-4-propionamid wird unter Erwärmen mit einer sterilen Gelatinelösung, welche als Konservierungsmittel Phenol enthält, unter aseptischen Bedinungen so vermischt, dass 1,0 ml Lösung folgende Zusammensetzung hat:

| | |
|---|---|
| (S)-α-[(S)-α-[(t-Butylsulfonyl)methyl]hydrocinnamamido]-N-[(1S,2R,3S)-1-(cyclohexylmethyl)-2,3-dihydroxy-4-methylpentyl]imidazol-4-propionamid | 3,0 mg |
| Gelatine | 150,0 mg |
| Phenol | 4,7 mg |
| dest. Wasser bis zu | 1,0 ml |

Die Mischung wird unter aseptischen Bedingungen in Vialen zu 1,0 ml abgefüllt.

Beispiel B

Man löst 5 mg (S)-α-[(S)-α-[(t-Butylsulfonyl)methyl]hydrocinnamamido]-N-[(1S,2R,3S)-1-(cyclohexylmethyl)-2,3-dihydroxy -4-methylpentyl]imidazol-4-propionamid in 1 ml einer wässrigen Lösung mit 20 mg Mannit. Die Lösung wird sterilfiltriert und unter aseptischen Bedingungen in eine 2 ml Ampulle gefüllt, tiefgekühlt und lyophilisiert. Vor Gebrauch wird das Lyophilisat in 1 ml destilliertem Wasser oder 1 ml physiologischer Salzlösung gelöst. Die Lösung wird intramuskulär oder intravenös angewendet. Diese Formulierung kann auch in Doppelkammerspritzampullen abgefüllt werden.

Beispiel C

In einer Mischung von 3,5 ml Myglyol 812 und 0,08 g Benzylalkohol werden 500 mg fein gemahlenes (5,0 µm) (S)-α-[(S)-α-[(t-Butylsulfonyl)methyl]hydrocinnamamido]-N-[(1S,2R,3S)-1-(cyclohexylmethyl)-2,3-dihydroxy -4-methylpentyl]imidazol-4-propionamid suspendiert. Diese Suspension wird in einen Behälter mit Dosierventil eingefüllt. Es werden 5,0 g Freon 12 unter Druck durch das Ventil in den Behälter abgefüllt. Durch Schütteln wird das Freon in der Myglyol-Benzylalkoholmischung gelöst. Dieser Spraybehälter enthält ca. 100 Einzeldosen, die einzeln appliziert werden können.

Beispiel D

Wenn man nach den in den Beispielen A-C beschriebenen Verfahren arbeitet, können aus den folgenden, ebenfalls bevorzugten Verbindungen entsprechende galenische Präparate hergestellt werden:
(S)-N-[(1S,2R,3S)-1-(Cyclohexylmethyl)-3-cyclopropyl-2,3-dihydroxypropyl]-α-[(R)-α-(3,3-dimethyl-2-oxobutyl)hydrocinnamamido]imidazol-4-propionamid;
(S)-α-[(S)-α-[(t-Butylsulfonyl)methyl]hydrocinnamamido]-N-[(1S,2R,3S)-1-(cyclohexylmethyl)-3-cyclopropyl-2,3-dihydroxypropyl]imidazol-4-propionamid;
(S)-α-[(S)-α-[(t-Butylsulfonyl)methyl]hydrocinnamamido]-N-[(1S,2R,3S)-3-cyclobutyl-1-(cyclohexylmethyl)-2,3-dihydroxypropyl]imidazol-4-propionamid;
(S)-N-[(S)-1-[(2R oder S,4R,5S)-2-t-Butyl-5-isopropyl-1,3-dioxolan-4-yl]-2-cyclohexyläthyl]-α-[(S)-α-[(t-butylsulfonyl)methyl]hydrocinnamamido]imidazol-4-propionamid;
(1S,2R)-[(S)-1-[(S)-α-[(S)-α-[(t-Butylsulfonyl)methyl]hydrocinnamamido]imidazol-4-propionamido]-2-cyclohexyläthyl]-1-isopropyläthylen-bis(methoxyacetat);
(S)-α-[(S)-α-[(t-Butylsulfonyl)methyl]hydrocinnamamido]-N-[(1S,2R,3S)-1-(cyclohexylmethyl)-3-cyclopentyl-2,3-dihydroxypropyl]imidazol-4-propionamid;
(S)-α-[(S)-α-[(t-Butylsulfonyl)methyl]hydrocinnamamido]-N-[(1S,2R,3S)-1-(cyclohexylmethyl)-3-cyclopropyl-2,3-dihydroxypropyl]-4-thiazolpropionamid und
(S)-α-[(S)-α-[(t-Butylsulfonyl)methyl]hydrocinnamamido]-N-[(1S,2S oder R,3S oder R,4SR)-1-(cyclohexylmethyl)-2,3,5-trihydroxypentyl]imidazol-4-propionamid.

**Ansprüche**

1. Aminosäurederivate der allgemeinen Formel

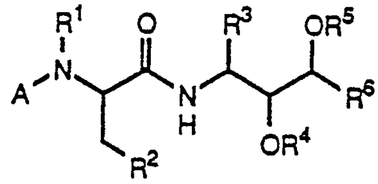

I

worin $R^1$ Wasserstoff oder Methyl, $R^2$ Aethyl, Propyl, Isopropyl, Imidazol-2-yl, Imidazol-4-yl, Pyrazol-3-yl, Thiazol-4-yl, Thien-2-yl oder t-Butoxy, $R^3$ Isobutyl, Cyclohexylmethyl oder Benzyl, $R^4$ und $R^5$ unabhängig voneinander je Wasserstoff, gegebenenfalls durch Amino, Monoalkylamino, Dialkylamino, Alkanoylamino, Alkanoyloxyamino, Carboxy, Alkoxy oder Hydroxy ein-oder mehrfach substituiertes Alkanoyl oder eine O-Schutzgruppe oder zusammen eine cyclische O-Schutzgruppe, $R^6$ eine der Gruppen

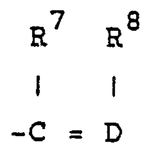

(a)

und

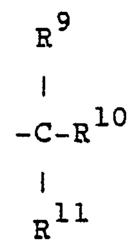

(b)

und A eine der Gruppen

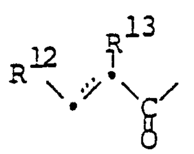

. (c) und -Y-Z (d)

bedeuten, worin D eine Methingruppe oder ein Stickstoffatom, $R^7$ Alkyl, Aryl oder Arylalkl, $R^8$ Wasserstoff, Alkyl, Aryl oder Arylalkyl, oder $R^7$ und $R^8$ zusammen mit den beiden sie verbindenden Atomen, Aryl, Heteroaryl, Cycloalkenyl oder Heterocycloalkenyl, $R^9$ Wasserstoff oder Alkyl und $R^{10}$ und $R^{11}$ unabhängig voneinander je Alkyl, Aryl, Arylalkyl, Cycloalkyl oder die Gruppe

$-CH_2-X-R^{14}$ (e)

oder zusammen mit dem Kohlenstoffatom, an das sie gebunden sind, Cycloalkyl oder Heterocycloalkyl bedeuten, mit der Massgabe, dass, falls $R^9$ Alkyl bedeutet, $R^{10}$ und $R^{11}$ ebenfalls Alkyl bedeuten, die gestrichelte Linie eine zusätzliche Bindung bedeuten kann, $R^{12}$ Phenyl, substituiertes Phenyl, Benzyl oder Naphthyl, und $R^{13}$ Wasserstoff, Alkoxycarbonylalkyl, Alkylcarbonylalkyl, Cycloalkylcarbonylalkyl, Heterocycloalkylcarbonylalkyl, Arylcarbonylalkyl, Aminocarbonylalkyl, substituiertes Aminocarbonylalkyl, Alkoxycarbonylhydroxyalkyl, Alkylcarbonylhydroxyalkyl, Cycloalkylcarbonylhydroxyalkyl, Heterocycloalkylcarbonylhydroxyalkyl, Arylcarbonylhydroxyalkyl, Aminocarbonylhydroxyalkyl, substituiertes Aminocarbonylhydroxyalkyl, Dialkoxyphosphoroxyalkyl, Diphenyloxyphosphoroxyalkyl, Arylalkyl, Alkoxycarbonylamino, Arylalkoxycarbonylamino, Alkylthioalkyl, Alkylsulfinylalkyl oder Alkylsulfonylalkyl bedeuten, mit der Massgabe, dass $R^{13}$ nicht Alkoxycarbonylamino oder Arylalkoxycarbonylamino bedeuten kann, wenn $R^{12}$ Phenyl, Benzyl oder $\alpha$-Naphthyl bedeutet, Y den N-terminal mit Z verbundenen, bivalenten Rest von gegebenenfalls N- und/oder $\alpha$-methyliertem Phenylalanin, Cyclohexylalanin, Tyrosin, O-Methyltyrosin, $\alpha$-Naphthylalanin oder Homophenylalanin, Z Acyl, X ein Sauer stoff- oder Schwefelatom oder die Gruppe -NH- und $R^{14}$ Wasserstoff, Alkyl, Cycloalkyl, Arylalkyl, Cycloalkylalkyl, Alkylcarbonyl, Arylcarbonyl oder Arylalkylcarbonyl

bedeuten,
in Form von optisch reinen Diastereomeren, Diastereomerengemischen, diastereomeren Racematen oder Gemischen von diastereomeren Racematen sowie pharmazeutisch verwendbare Salze dieser Verbindungen.

2. Verbindungen gemäss Anspruch 1, worin $R^2$ Aethyl, Propyl, Isopropyl, Imidazol-4-yl, Thiazol-4-yl, Thien-2-yl oder t-Butoxy, $R^4$ und $R^5$ unabhängig voneinander je Wasserstoff, gegebenenfalls durch Carboxy, Alkoxy oder Hydroxy ein- oder mehrfach substituiertes Alkanoyl oder eine O-Schutzgruppe oder zusammen eine cyclische O-Schutzgruppe, $R^{12}$ Phenyl, substituiertes Phenyl oder Naphthyl und $R^{13}$ Wasserstoff, Alkoxycarbonylalkyl, Alkylcarbonylalkyl, Cycloalkylcarbonylalkyl, Heterocycloalkylcarbonylalkyl, Arylcarbonylalkyl, Aminocarbonylalkyl, substituiertes Aminocarbonylalkyl, Alkoxycarbonylamino, Arylalkoxycarbonylamino, Alkylthioalkyl, Alkylsulfinylalkyl oder Alkylsulfonylalkyl bedeuten.

3. Verbindungen gemäss Anspruch 2, worin $R^2$ Aethyl, Propyl, Isopropyl oder Imidazol-4-yl, $R^4$ und $R^5$ je Wasserstoff, $R^7$ und $R^8$ unabhängig voneinander je Alkyl, Aryl oder Arylalkyl oder, zusammen mit den beiden sie verbindenden Atomen, Aryl, Heteroaryl, Cycloalkenyl oder Heterocycloalkenyl und $R^{14}$ Alkyl, Cycloalkyl, Arylalkyl, Cycloalkylalkyl, Alkylcarbonyl, Arylcarbonyl oder Arylalkylcarbonyl bedeuten.

4. Verbindungen gemäss Anspruch 1, worin $R^1$ Wasserstoff, $R^2$ Propyl, Imidazol-2-yl, Imidazol-4-yl, Thiazol-4-yl, Thien-2-yl oder t-Butoxy, $R^3$ Cyclohexylmethyl, $R^4$ und $R^5$ unabhängig voneinander je Wasserstoff, gegebenenfalls durch Dialkylamino, Carboxy oder Alkoxy einfach substituiertes Alkanoyl oder zusammen eine cyclische O-Schutzgruppe, $R^6$ die Gruppe (a) oder (b), A die Gruppe (c) oder (d), D eine Methingruppe, $R^7$ Alkyl, $R^8$ Wasserstoff oder Alkyl, oder $R^7$ und $R^8$ zusammen mit den beiden sie verbindenden Atomen, Aryl, Heteroaryl oder Cycloalkenyl, $R^9$ Wasserstoff oder Alkyl, $R^{10}$ und $R^{11}$ unabhängig voneinander je Alkyl oder die Gruppe (e) oder zusammen mit den Kohlenstoffatomen, an das sie gebunden sind, Cycloalkyl oder Heterocycloalkyl, $R^{12}$ Phenyl, substituiertes Phenyl, Benzyl oder Naphthyl, $R^{13}$ Alkylcarbonylalkyl, Heterocycloalkylcarbonylalkyl, Alkylcarbonylhydroxyalkyl, Dialkoxyphosphoroxyalkyl, Arylalkyl, Alkylsulfinylalkyl oder Alkylsulfonylalkyl, Y den N-terminal mit Z verbundenen, bivalenten Rest von Phenylalanin, Z Acyl, X ein Sauerstoffatom und $R^{14}$ Wasserstoff oder Arylalkyl bedeuten.

5. Verbindungen gemäss Anspruch 4, worin $R^1$ Wasserstoff, $R^2$ Imidazol-2-yl, Imidazol-4-yl oder Thiazol-4-yl, besonders bevorzugt Imidazol-4-yl, $R^3$ Cyclohexylmethyl, $R^4$ und $R^5$ unabhängig voneinander je Wasserstoff, gegebenenfalls durch Methoxy einfach substituiertes Alkanoyl oder zusammen das Acetal von Pivalinaldehyd, besonders bevorzugt je Wasserstoff, $R^6$ die Gruppe (b), A die Gruppe (c), $R^9$ Wasserstoff, $R^{10}$ und $R^{11}$ unabhängig voneinander je Alkyl oder zusammen mit dem Kohlenstoffatom, an das sie gebunden sind, Cycloalkyl, besonders bevorzugt Methyl, Aethyl, Cyclopropyl oder Cyclobutyl, $R^{12}$ Phenyl oder substituiertes Phenyl, besonders bevorzugt Phenyl, und $R^{13}$ Alkylcarbonylalkyl oder Alkylsulfonylalkyl, besonders bevorzugt $C_1$-$C_4$-Alkylcarbonylmethyl oder $C_1$-$C_4$-Alkylsulfonylmethyl, bedeuten.

6. Verbindungen gemäss Anspruch 5, worin $R^1$ Wasserstoff, $R^2$ Imidazol-4-yl, $R^3$ Cyclohexylmethyl, $R^4$ und $R^5$ je Wasserstoff, $R^6$ die Gruppe (b), $R^9$ Wasserstoff, $R^{10}$ und $R^{11}$ je Methyl oder Aethyl oder zusammen mit dem Kohlenstoffatom, an das sie gebunden sind, Cyclopropyl oder Cyclobutyl, $R^{12}$ Phenyl und $R^{13}$ $C_1$-$C_4$-Alkylcarbonylmethyl oder $C_1$-$C_4$-Alkylsulfonylmethyl bedeuten.

7. (S)-N-[(1S,2R,3RS)-1-(Cyclohexylmethyl)-4-äthyl-2,3-dihydroxyhexyl]-α-[(R)-α-(3,3-dimethyl-2-oxobutyl)hydrocinnamamido]imidazol-4-propionamid, (S)-N-[(1S,2R,3S)-3-Cyclohexyl-1-(cyclohexylmethyl) -2,3-dihydroxypropyl]-α-[(R)-α-(3,3-dimethyl-2-oxobutyl)hydrocinnamamido]-imidazol-4-propionamid, (S)-N-[-(1S,2R,3S)-1-(Cyclohexylmethyl)-2,3-dihydroxy-4-methylpentyl]-α-[(R)-α-(3,3-dimethyl-2-oxobutyl)-hydrocinnamamido]imidazol-4-propionamid, (S)-α-[(S)-α-[(t-Butylsulfonyl)methyl]hydrocinnamamido]-N-[-(1S,2R,3S)-1-(cyclohexylmethyl)-2,3-dihydroxy-3-phenylpropyl]imidazol-4-propionamid, (S)-α-[(S)-α-[(t-Butylsulfonyl)methyl]hydrocinnamamido]-N-[(1S,2R,3R)-1-(cyclohexylmethyl)-2,3-dihydroxy-3-phenylpropyl]-imidazol-4-propionamid, (S)-α-[(S)-α-[(t-Butylsulfonyl)methyl]hydrocinnamamido]-N-[(1S,2R,3RS)-1-(cyclohexylmethyl)-2,3-dihydroxy-4,4-dimethylpentyl]imidazol-4-propionamid, (S)-α-[(S)-α-[(t-Butylsulfonyl)-methyl]hydrocinnamamido]-N-[(1S,2R,3S)-1-(cyclohexylmethyl)-2,3-dihydroxy-4-methylpentyl]imidazol-4-propionamid oder (S)-α-[(S)-α-{(t-Butylsulfonyl)methyl]hydrocinnamamido]-N-[(1S,2R,3S)-3-cyclohexyl-1-(cyclohexylmethyl)-2,3-dihydroxypropyl]imidazol-4-propionamid.

8. (S)-α-[(S)-α-[(t-Butylsulfonyl)methyl]hydrocinnamamido]-N-[(1S,2R,3RS)-1-(cyclohexylmethyl)-2,3-dihydroxy-3-(2-furyl)propyl]imidazol-4-propionamid, (S)-N-[(1S,2R,3R oder S)-1-(Cyclohexylmethyl)-2,3-dihydroxy-3-[(R oder S)-tetrahydro-2-furyl]propyl]-α-[(R)-α-(3,3-dimethyl-2-oxobutyl)hydrocinnamamido]-imidazol-4-propionamid oder (S)-α-[(S)-α-[(t-Butylsulfonyl)methyl]hydrocinnamamido]-N-{(1S,2R,3R oder S,Z)-1-(cyclohexylmethyl)-2,3-dihydroxy-4-methyl-4-hexenyl] imidazol-4-propionamid.

9. (S)-N-[(1S,2R,3S)-1-(Cyclohexylmethyl)-3-cyclopropyl-2,3-dihydroxypropyl]-α-[(S)-α-[-(morpholinocarbonyl)methyl]hydrocinnamamido]imidazol-4-propionamid, (S)-α-[(S)-α-[(t-Butylsulfonyl)-methyl]-1-naphthalinpropionamido]-N-[(1S,2R,3S)-1-(cyclohexylmethyl)-3-cyclopropyl-2,3-dihydroxypropyl]-imidazol-4-propionamid, (S)-α-[(S)-2-[(t-Butylsulfonyl)methyl]-4-phenylbutyramido]-N-[(1S,2R,3S)-1-(cyclohexylmethyl)-3-cyclopropyl-2,3-dihydroxypropyl]imidazol-4-propionamid, (S)-α-[(R)-α-[(t-Butylsulfonyl)-methyl]hydrocinnamamido]-N-[(1S,2R,3S)-1-(cyclohexylmethyl)-3-cyclopropyl-2,3-dihydroxypropyl]imidazol-4-propionamid, (S)-N-[(S)-1-[(2R oder S,4R,5S)-2-t-Butyl-5-isopropyl-1,3-dioxolan-4-yl]-2-cyclohexyläthyl]-α-[(S)-α-[(t-butylsulfonyl)methyl]hydrocinnamamido]imidazol-4-propionamid oder (1R,2S)-1-[(S)-1-[[N-[(S)-α-[(t-Butylsulfonyl)methyl]cinnamoyl]-L-histidyl]amino]-2-cyclohexyläthyl]-2-cyclopropyläthylen-bis-(methoxyacetat).

10. (S)-N-[(1S,2R,3S)-1-(Cyclohexylmethyl)-3-cyclcpropyl-2,3-dihydroxypropyl]-α-[(R)-α-(3,3-dimethyl-2-oxobutyl)hydrocinnamamido]imidazol-4-propionamid.

11. (S)-α-[(S)-α-[(t-Butylsulfonyl)methyl]hydrocinnamamido]-N-[(1S,2R,3S)-1-(cyclohexylmethyl)-3-cyclopropyl-2,3-dihydroxypropyl]imidazol-4-propionamid.

12. (S)-α-[(S)-α-[(t-Butylsulfonyl)methyl]hydrocinnamamido]-N-[(1S,2R,3S)-3-cyclobutyl-1-(cyclohexylmethyl)-2,3-dihydroxypropyl]imidazol-4-propionamid.

13. (S)-N-[(S)-1-[(4R,5S)-2-t-Butyl-5-cyclopropyl-1,3-dioxolan-4-yl]-2-cyclohexyläthyl]-α-[(S)-α-[(t-butylsulfonyl)methyl]hydrocinnamamido]imidazol-4-propionamid.

14. (1S,2R)-[(S)-1-[(S)-α-[(S)-α-[(t-Butylsulfonyl)methyl]hydrocinnamamido]imidazol-4-propionamido]-2-cyclohexyläthyl]-1-isopropyläthylen-bis(methoxyacetat).

15. (S)-α-[(S)-α-[(t-Butylsulfonyl)methyl]hydrocinnamamido]-N-[(1S,2R,3S)-1-(cyclohexylmethyl)-3-cyclopentyl-2,3-dihydroxypropyl]imidazol-4-propionamid.

16. (S)-α-[(S)-α-[(t-Butylsulfonyl)methyl]hydrocinnamamido]-N-[(1S,2R,3S)-1-(cyclohexylmethyl)-3-cyclopropyl-2,3-dihydroxypropyl]-4-thiazolpropionamid.

17. (S)-α-[(S)-α-[(t-Butylsulfonyl)methyl]hydrocinnamamido]-N-[(1S,2S oder R,3S oder R,4SR)-1-(cyclohexylmethyl)-2,3,5-trihydroxypentyl]imidazol-4-propionamid.

18. Verbindungen der allgemeinen Formeln

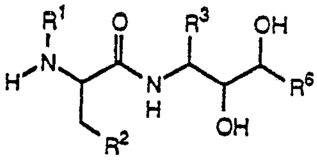

II

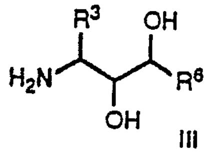

III

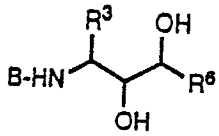

V

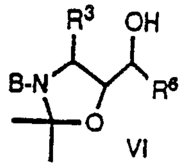

VI

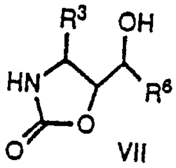

VII

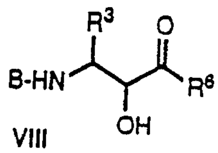

VIII

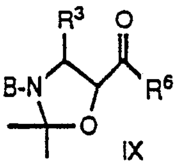

IX

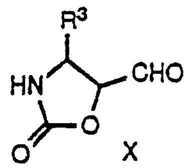

X

und

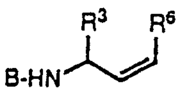

XVII

worin $R^1$ Wasserstoff oder Methyl, $R^2$ Aethyl, Propyl, Isopropyl, Imidazol-2-yl, Imidazol-4-yl, Pyrazol-3-yl, Thiazol-4-yl, Thien-2-yl odeer t-Butoxy, $R^3$ Isobutyl, Cyclohexylmethyl oder Benzyl und $R^6$ eine der Gruppen

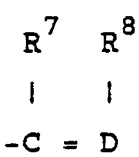

(a)

und

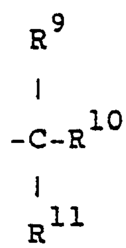

(b)

bedeuten, worin D eine Methingruppe oder ein Stickstoffatom, $R^7$ Alkyl, Aryl oder Arylalkyl, $R^8$ Wasserstoff, Alkyl, Aryl oder Arylalkyl, oder $R^7$ und $R^8$ zusammen mit den beiden sie verbindenden Atomen, Aryl,

Heteroaryl, Cycloalkenyl oder Heterocycloalkenyl, $R^9$ Wasserstoff oder Alkyl und $R^{10}$ und $R^{11}$ unabhängig voneinander je Alkyl, Aryl, Arylalkyl, Cycloalkyl oder die Gruppe

$-CH_2-X-R^{14}$ (e)

oder zusammen mit dem Kohlenstoffatom, an das sie gebunden sind, Cycloalkyl oder Heterocycloalkyl und $R^{14}$ Wasserstoff, Alkyl, Cycloalkyl, Arylalkyl, Cycloalkylalkyl, Alkylcarbonyl, Arylcarbonyl oder Arylalkylcarbonyl bedeuten, mit der Massgabe, dass, falls $R^9$ Alkyl bedeutet, $R^{10}$ und $R^{11}$ ebenfalls Alkyl bedeuten.

19. Aminosäurederivate gemäss einem der Ansprüche 1-17 zur Anwendung als therapeutische Wirkstoffe.

20. Aminosäurederivate gemäss einem der Ansprüche 1-17 zur Anwendung bei der Bekämpfung bzw. Verhütung von Blut hochdruck und Herzinsuffizienz.

21. Verfahren zur Herstellung einer Verbindung gemäss einem der Ansprüche 1-17, dadurch gekennzeichnet, dass man

a) eine Verbindung der allgemeinen Formel

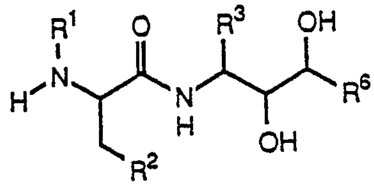

II

worin $R^1$, $R^2$, $R^3$ und $R^6$ die in Anspruch 1 angegebene Bedeutung besitzen,
mit einem die Gruppe

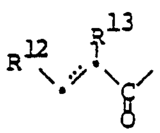

. (c) oder -Y-Z (d)

worin $R^{12}$, $R^{13}$, Y, Z und die gestrichelte Linie die in Anspruch 1 angegebene Bedeutung besitzen,
abgebenden Acylierungsmittel umsetzt, oder

b) eine Verbindung der allgemeinen Formel

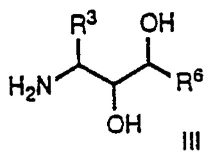

III

worin $R^3$ und $R^6$ die in Anspruch 1 angegebene Bedeutung besitzen,
mit einer Verbindung der allgemeinen Formel

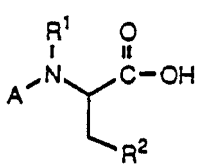

IV

worin $R^1$, $R^2$ und A die in Anspruch 1 angegebene Bedeutung besitzen,
oder einem aktivierten Derivat davon umsetzt, oder

c) eine Verbindung der Formel I, worin $R^4$ und $R^5$ je Wasserstoff bedeuten und die übrigen Symbole die in Anspruch 1 angegebene Bedeutung besitzen, mit einem gegebenenfalls durch Amino, Monoalkylamino, Dialkylamino, Alkanoylamino, Alkanoyloxyamino, Carboxy, Alkoxy oder Hydroxy ein- oder mehrfach substituierten Alkanoylierungsmittel oder einem eine O-Schutzgruppe bildenden Mittel umsetzt, und

d) erwünschtenfalls ein Gemisch diastereomerer Racemate in die diastereomeren Racemate oder optisch reinen Diastereomeren auftrennt, und/oder

e) erwünschtenfalls ein Diastereomerengemisch in die optisch reinen Diastereomeren auftrennt, und/oder

f) erwünschtenfalls eine erhaltene Verbindung in ein pharmazeutisch verwendbares Salz überführt.

22. Arzneimittel, enthaltend ein Aminosäurederivat gemäss einem der Ansprüche 1-17 und ein therapeutisch inertes Excipiens.

23. Mittel zur Bekämpfung bze. Verhütung von Bluthockdruck und Herzinsuffizienz, enthaltend ein Aminosäurederivat gemäss einem der Ansprüche 1-17 und ein therapeutisch iner tes Excipiens.

24. Verwendung eines Aminosäurederivates gemäss einem der Ansprüche 1-17 bei der Bekämpfung bzw. Verhütung von Krankheiten.

25. Verwendung eines Aminosäurederivates gemäss einem der Ansprüche 1-17 bei der Bekämpfung bzw., Verhütung von Bluthochdruck und Herzinsuffizienz.

26. Verwendung eines Aminosäurederivates gemäss einem der Ansprüche 1-17 zur Herstellung von Mitteln gegen Bluthochdruck und/oder Herzinsuffizienz.

Patentansprüche für folgenden Vertragsstaat: ES

1. Verfahren zur Herstellung von Aminosäurederivaten der allgemeinen Formel

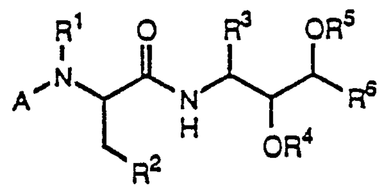

I

worin $R^1$ Wasserstoff oder Methyl, $R^2$ Aethyl, Propyl, Isopropyl, Imidazol-2-yl, Imidazol-4-yl, Pyrazol-3-yl, Thiazol-4-yl, Thien-2-yl oder t-Butoxy, $R^3$ Isobutyl, Cyclohexylmethyl oder Benzyl, $R^4$ und $R^5$ unabhängig voneinander je Wasserstoff, gegebenenfalls durch Amino, Monoalkylamino, Dialkylamino, Alkanoylamino, Alkanoyloxyamino, Carboxy, Alkoxy oder Hydroxy ein-oder mehrfach substituiertes Alkanoyl oder eine O-Schutzgruppe oder zusammen eine cyclische O-Schutzgruppe, $R^6$ eine der Gruppen

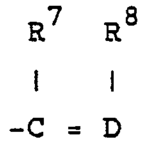

(a)

und

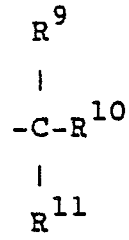

(b)

und A eine der Gruppen

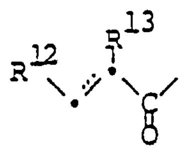

(c) und -Y-Z (d)

bedeuten, worin D eine Methingruppe oder ein Stickstoffatom, $R^7$ Alkyl, Aryl oder Arylalkl, $R^8$ Wasserstoff, Alkyl, Aryl oder Arylalkyl, oder $R^7$ und $R^8$ zusammen mit den beiden sie verbindenden Atomen, Aryl, Heteroaryl, Cycloalkenyl oder Heterocycloalkenyl, $R^9$ Wasserstoff oder Alkyl und $R^{10}$ und $R^{11}$ unabhängig voneinander je Alkyl, Aryl, Arylalkyl, Cycloalkyl oder die Gruppe

$-CH_2-X-R^{14}$ (e)

oder zusammen mit dem Kohlenstoffatom, an das sie gebunden sind, Cycloalkyl oder Heterocycloalkyl bedeuten, mit der Massgabe, dass, falls $R^9$ Alkyl bedeutet, $R^{10}$ und $R^{11}$ ebenfalls Alkyl bedeuten, die gestrichelte Linie eine zusätzliche Bindung bedeuten kann, $R^{12}$ Phenyl, substituiertes Phenyl, Benzyl oder Naphthyl, und $R^{13}$ Wasserstoff, Alkoxycarbonylalkyl, Alkylcarbonylalkyl, Cycloalkylcarbonylalkyl, Heterocycloalkylcarbonylalkyl, Arylcarbonylalkyl, Aminocarbonylalkyl, substituiertes Aminocarbonylalkyl, Alkoxycarbonylhydroxyalkyl, Alkylcarbonylhydroxyalkyl, Cycloalkylcarbonylhydroxyalkyl, Heterocycloalkylcarbonylhydroxyalkyl, Arylcarbonylhydroxyalkyl, Aminocarbonylhydroxyalkyl, substituiertes Aminocarbonylhydroxyalkyl, Dialkoxyphosphoroxyalkyl, Diphenyloxyphosphoroxyalkyl, Arylalkyl, Alkoxycarbonylamino, Arylalkoxycarbonylamino, Alkylthioalkyl, Alkylsulfinylalkyl oder Alkylsulfonylalkyl bedeuten, mit der Massgabe, dass $R^{13}$ nicht Alkoxycarbonylamino oder Arylalkoxycarbonylamino bedeuten kann, wenn $R^{12}$ Phenyl, Benzyl oder α-Naphthyl bedeutet, Y den N-terminal mit Z verbundenen, bivalenten Rest von gegebenenfalls N- und/oder α-methyliertem Phenylalanin, Cyclohexylalanin, Tyrosin, O-Methyltyrosin, α-Naphthylalanin oder Homophenylalanin, Z Acyl, X ein Sauer stoff- oder Schwefelatom oder die Gruppe -NH- und $R^{14}$ Wasserstoff, Alkyl, Cycloalkyl, Arylalkyl, Cycloalkylalkyl, Alkylcarbonyl, Arylcarbonyl oder Arylalkylcarbonyl bedeuten,

in Form von optisch reinen Diastereomeren, Diastereomerengemischen, diastereomeren Racematen oder Gemischen von diastereomeren Racematen sowie pharmazeutisch verwendbaren Salzen dieser Verbindungen, dadurch gekennzeichnet, dass man

a) eine Verbindung der allgemeinen Formel

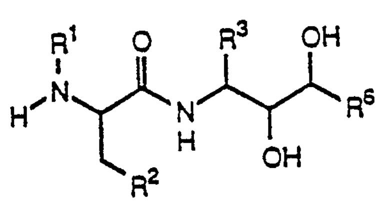

II

worin $R^1$, $R^2$, $R^3$ und $R^6$ die oben angegebene Bedeutung besitzen,
mit einem die Gruppe

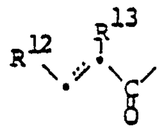 . (c) oder -Y-Z (d)

worin $R^{12}$, $R^{13}$, Y, Z und die gestrichelte Linie die oben angegebene Bedeutung besitzen, abgebenden Acylierungsmittel umsetzt, oder

b) eine Verbindung der allgemeinen Formel

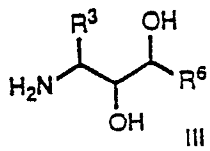

III

worin $R^3$ und $R^6$ die oben angegebene Bedeutung besitzen, mit einer Verbindung der allgemeinen Formel

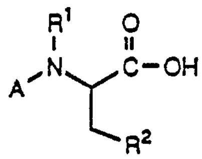

IV

worin $R^1$, $R^2$ und A die oben angegebene Bedeutung besitzen, oder einem aktivierten Derivat davon umsetzt, oder

c) eine Verbindung der Formel I, worin $R^4$ und $R^5$ je Wasserstoff bedeuten und die übrigen Symbole die oben angegebene Bedeutung besitzen, mit einem gegebenenfalls durch Amino, Monoalkylamino, Dialkylamino, Alkanoylamino, Alkanoyloxyamino, Carboxy, Alkoxy oder Hydroxy ein- oder mehrfach substituierten Alkanoylierungsmittel oder einem eine O-Schutzgruppe bildenden Mittel umsetzt, und

d) erwünschtenfalls ein Gemisch diastereomerer Racemate in die diastereomeren Racemate oder optisch reinen Diastereomeren auftrennt, und/oder

e) erwünschtenfalls ein Diastereomerengemisch in die optisch reinen Diastereomeren auftrennt, und/oder

f) erwünschtenfalls eine erhaltene Verbindung in ein pharmazeutisch verwendbares Salz überführt.

2. Verfahren gemäss Anspruch 1, worin $R^2$ Aethyl, Propyl, Isopropyl, Imidazol-4-yl, Thiazol-4-yl, Thien-2-yl oder t-Butoxy, $R^4$ und $R^5$ unabhängig voneinander je Wasserstoff, gegebenenfalls durch Carboxy, Alkoxy oder Hydroxy ein- oder mehrfach substituiertes Alkanoyl oder eine O-Schutzgruppe oder zusammen eine cyclische O-Schutzgruppe, $R^{12}$ Phenyl, substituiertes Phenyl oder Naphthyl und $R^{13}$ Wasserstoff, Alkoxycarbonylalkyl, Alkylcarbonylalkyl, Cycloalkylcarbonylalkyl, Heterocycloalkylcarbonylalkyl, Arylcarbonylalkyl, Aminocarbonylalkyl, substituiertes Aminocarbonylalkyl, Alkoxycarbonylamino, Arylalkoxycarbonylamino, Alkylthioalkyl, Alkylsulfinylalkyl oder Alkylsulfonylalkyl bedeuten.

3. Verfahren gemäss Anspruch 2, worin $R^2$ Aethyl, Propyl, Isopropyl oder Imidazol-4-yl, $R^4$ und $R^5$ je Wasserstoff, $R^7$ und $R^8$ unabhängig voneinander je Alkyl, Aryl oder Arylalkyl oder, zusammen mit den beiden sie verbindenden Atomen, Aryl, Heteroaryl, Cycloalkenyl oder Heterocycloalkenyl und $R^{14}$ Alkyl, Cycloalkyl, Arylalkyl, Cycloalkylalkyl, Alkylcarbonyl, Arylcarbonyl oder Arylalkylcarbonyl bedeuten.

4. Verfahren gemäss Anspruch 1, worin $R^1$ Wasserstoff, $R^2$ Propyl, Imidazol-2-yl, Imiazol-4-yl, Thiazol-4-yl, Thien-2-yl oder t-Butoxy, $R^3$ Cyclohexylmethyl, $R^4$ und $R^5$ unabhängig voneinander je Wasserstoff, gegebenenfalls durch Dialkylamino, Carboxy oder Alkoxy einfach substituiertes Alkanoyl oder zusammen eine cyclische O-Schutzgruppe, $R^6$ die Gruppe (a) oder (b), A die Gruppe (c) oder (d), D eine Methingruppe, $R^7$ Alkyl, $R^8$ Wasserstoff oder Alkyl, oder $R^7$ und $R^8$ zusammen mit den beiden sie verbindenden

Atomen, Aryl, Heteroaryl oder Cycloalkenyl, $R^9$ Wasserstoff oder Alkyl, $R^{10}$ und $R^{11}$ unabhängig voneinander je Alkyl oder die Gruppe (e) oder zusammen mit den Kohlenstoffatomen, an das sie gebunden sind, Cycloalkyl oder Heterocycloalkyl, $R^{12}$ Phenyl, substituiertes Phenyl, Benzyl oder Naphthyl, $R^{13}$ Alkylcarbonylalkyl, Heterocycloalkylcarbonylalkyl, Alkylcarbonylhydroxyalkyl, Dialkoxyphosphoroxyalkyl, Arylalkyl, Alkylsulfinylalkyl oder Alkylsulfonylalkyl, Y den N-terminal mit Z verbundenen, bivalenten Rest von Phenylalanin, Z Acyl, X ein Sauerstoffatom und $R^{14}$ Wasserstoff oder Arylalkyl bedeuten.

5. Verfahren gemäss Anspruch 4, worin $R^1$ Wasserstoff, $R^2$ Imidazol-2-yl, Imidazol-4-yl oder Thiazol-4-yl, besonders bevorzugt Imidazol-4-yl, $R^3$ Cyclohexylmethyl, $R^4$ und $R^5$ unabhängig voneinander je Wasserstoff, gegebenenfalls durch Methoxy einfach substituiertes Alkanoyl oder zusammen das Acetal von Pivalinaldehyd, besonders bevorzugt je Wasserstoff, $R^6$ die Gruppe (b), A die Gruppe (c), $R^9$ Wasserstoff, $R^{10}$ und $R^{11}$ unabhängig voneinander je Alkyl oder zusammen mit dem Kohlenstoffatom, an das sie gebunden sind, Cycloalkyl, besonders bevorzugt Methyl, Aethyl, Cyclopropyl oder Cyclobutyl, $R^{12}$ Phenyl oder substituiertes Phenyl, besonders bevorzugt Phenyl, und $R^{13}$ Alkylcarbonylalkyl oder Alkylsulfonylalkyl, besonders bevorzugt $C_1$-$C_4$-Alkylcarbonylmethyl oder $C_1$-$C_4$-Alkylsulfonylmethyl, bedeuten.

6. Verfahren gemäss Anspruch 5, worin $R^1$ Wasserstoff, $R^2$ Imidazol-4-yl, $R^3$ Cyclohexylmethyl, $R^4$ und $R^5$ je Wasserstoff, $R^6$ die Gruppe (b), $R^9$ Wasserstoff, $R^{10}$ und $R^{11}$ je Methyl oder Aethyl oder zusammen mit dem Kohlenstoffatom, an das sie gebunden sind, Cyclopropyl oder Cyclobutyl, $R^{12}$ Phenyl und $R^{13}$ $C_1$-$C_4$-Alkylcarbonylmethyl oder $C_1$-$C_4$-Alkylsulfonylmethyl bedeuten.

7. Verfahren gemäss Anspruch 1, dadurch gekennzeichnet, dass man (S)-N-[(1S,2R,3S)-1-(Cyclohexylmethyl)-3-cyclopropyl-2,3-dihydroxypropyl]-α-[(R)-α-(3,3-dimethyl-2-oxobutyl)-hydrocinnamamido]imidazol-4-propionamid herstellt.

8. Verfahren gemäss Anspruch 1, dadurch gekennzeichnet, dass man (S)-α-[(S)-α-[(t-Butylsulfonyl)-methyl]hydrocinnamamido]-N-[(1S,2R,3S)-1-(cyclohexylmethyl)-3-cyclopropyl-2,3-dihydroxypropyl]imidazol-4-propionamid herstellt.

9. Verfahren gemäss Anspruch 1, dadurch gekennzeichnet, dass man (S)-α-[(S)-α-[(t-Butylsulfonyl)-methyl]hydrocinnamamido]-N-[(1S,2R,3S)-3-cyclobutyl-1-(cyclohexylmethyl)-2,3-dihydroxypropyl]imidazol-4-propionamid herstellt.

10. Verfahren gemäss Anspruch 1, dadurch gekennzeichnet, dass man (S)-N-[(S)-1-[(4R,5S)-2-t-Butyl-5-cyclopropyl-1,3-dioxolan-4-yl]-2-cyclohexyläthyl]-α-[(S)-α-[(t-butylsulfonyl)methyl]hydrocinnamamido]-imidazol-4-propionamid herstellt.

11. Verfahren gemäss Anspruch 1, dadurch gekennzeichnet, dass man (1S,2R)-[(S)-1-[(S)-α-[(S)-α-[(t-Butylsulfonyl)methyl]hydrocinnamamido]imidazol-4-propionamido]-2-cyclohexyläthyl]-1-isopropyläthylen-bis-(methoxyacetat) herstellt.

12. Verfahren gemäss Anspruch 1, dadurch gekennzeichnet, dass man (S)-α-[(S)-α-[(t-Butylsulfonyl)-methyl]hydrocinnamamido]-N-[(1S,2R,3S)-1-(cyclohexylmethyl)-3-cyclopentyl-2,3-dihydroxypropyl]imidazol-4-propionamid herstellt.

13. Verfahren gemäss Anspruch 1, dadurch gekennzeichnet, dass man (S)-α-[(S)-α-[(t-Butylsulfonyl)-methyl]hydrocinnamamido]-N-[(1S,2R,3S)-1-(cyclohexylmethyl)-3-cyclopropyl-2,3-dihydroxypropyl]-4-thiazolpropionamid herstellt.

14. Verfahren gemäss Anspruch 1, dadurch gekennzeichnet, dass man (S)-α-[(S)-α-[(t-Butylsulfonyl)-methyl]hydrocinnamamido]-N-[(1S,2S oder R,3S oder R,4SR)-1-(cyclohexylmethyl)-2,3,5-trihydroxypentyl]-imidazol-4-propionamid herstellt.

15. Verwendung eines Aminosäurederivates der in Anspruch 1 angegebenen Formel I in Form eines optisch reinen Diastereomeren, eines Diastereomerengemisches, eines diastereomeren Racemates oder eines Gemisches von diastereomeren Racematen oder ein pharmazeutisch verwendbaren Salzes einer solchen Verbindung zur Herstellung von Mitteln gegen Bluthochdruck und/oder Herzinsuffizienz.

Patentansprüche für folgenden Vertragsstaat: GR

1. Verfahren zur Herstellung von Aminosäurederivaten der allgemeinen Formel

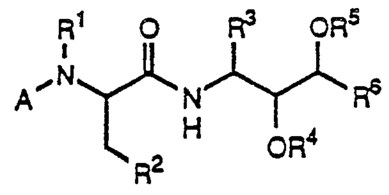

I

worin $R^1$ Wasserstoff oder Methyl, $R^2$ Aethyl, Propyl, Isopropyl, Imidazol-2-yl, Imidazol-4-yl, Pyrazol-3-yl, Thiazol-4-yl, Thien-2-yl oder t-Butoxy, $R^3$ Isobutyl, Cyclohexylmethyl oder Benzyl, $R^4$ und $R^5$ unabhängig voneinander je Wasserstoff, gegebenenfalls durch Amino, Monoalkylamino, Dialkylamino, Alkanoylamino, Alkanoyloxyamino, Carboxy, Alkoxy oder Hydroxy ein-oder mehrfach substituiertes Alkanoyl oder eine O-Schutzgruppe oder zusammen eine cyclische O-Schutzgruppe, $R^6$ eine der Gruppen

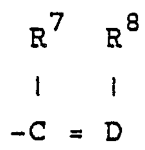

(a)

und

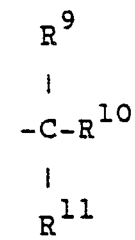

(b)

und A eine der Gruppen

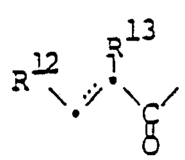

(c) und -Y-Z (d)

bedeuten, worin D eine Methingruppe oder ein Stickstoffatom, $R^7$ Alkyl, Aryl oder Arylalkl, $R^8$ Wasserstoff, Alkyl, Aryl oder Arylalkyl, oder $R^7$ und $R^8$ zusammen mit den beiden sie verbindenden Atomen, Aryl, Heteroaryl, Cycloalkenyl oder Heterocycloalkenyl, $R^9$ Wasserstoff oder Alkyl und $R^{10}$ und $R^{11}$ unabhängig voneinander je Alkyl, Aryl, Arylalkyl, Cycloalkyl oder die Gruppe

$-CH_2-X-R^{14}$ (e)

oder zusammen mit dem Kohlenstoffatom, an das sie gebunden sind, Cycloalkyl oder Heterocycloalkyl bedeuten, mit der Massgabe, dass, falls $R^9$ Alkyl bedeutet, $R^{10}$ und $R^{11}$ ebenfalls Alkyl bedeuten, die gestrichelte Linie eine zusätzliche Bindung bedeuten kann, $R^{12}$ Phenyl, substituiertes Phenyl, Benzyl oder Naphthyl, und $R^{13}$ Wasserstoff, Alkoxycarbonylalkyl, Alkylcarbonylalkyl, Cycloalkylcarbonylalkyl, Heterocycloalkylcarbonylalkyl, Arylcarbonylalkyl, Aminocarbonylalkyl, substituiertes Aminocarbonylalkyl, Alkoxycarbonylhydroxyalkyl, Alkylcarbonylhydroxyalkyl, Cycloalkylcarbonylhydroxyalkyl, Heterocycloalkylcarbonylhydroxyalkyl, Arylcarbonylhydroxyalkyl, Aminocarbonylhydroxyalkyl, substituiertes Aminocarbonylhydroxyalkyl, Dialkoxyphosphoroxyalkyl, Diphenyloxyphosphoroxyalkyl, Arylalkyl, Alkoxycarbonylamino, Arylalkoxycarbonylamino, Alkylthioalkyl, Alkylsulfinylalkyl oder Alkylsulfonylalkyl bedeuten, mit der Massgabe, dass $R^{13}$ nicht Alkoxycarbonylamino oder Arylalkoxycarbonylamino bedeuten kann, wenn $R^{12}$ Phenyl, Benzyl oder $\alpha$-Naphthyl bedeutet, Y den N-terminal mit Z verbundenen, bivalenten Rest von gegebenenfalls N-und/oder $\alpha$-methyliertem Phenylalanin, Cyclohexylalanin, Tyrosin, O-Methyltyrosin, $\alpha$-Naphthylalanin oder Homophenylalanin, Z Acyl, X ein Sauer stoff- oder Schwefelatom oder die Gruppe -NH- und $R^{14}$ Wasserstoff, Alkyl, Cycloalkyl, Arylalkyl, Cycloalkylalkyl, Alkylcarbonyl, Arylcarbonyl oder Arylalkylcarbonyl bedeuten,

in Form von optisch reinen Diastereomeren, Diastereomerengemischen, diastereomeren Racematen oder Gemischen von diastereomeren Racematen sowie pharmazeutisch verwendbaren Salzen dieser Verbindungen, dadurch gekennzeichnet, dass man

a) eine Verbindung der allgemeinen Formel

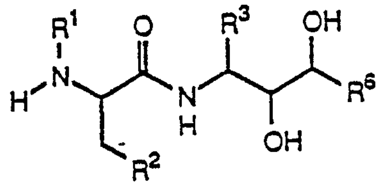

II

worin R', $R^2$, $R^3$ und $R^6$ die oben angegebene Bedeutung besitzen, mit einem die Gruppe

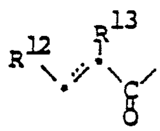

. (c) oder -Y-Z (d)

worin $R'^2$, $R'^3$, Y, Z und die gestrichelte Linie die oben angegebene Bedeutung besitzen, abgebenden Acylierungsmittel umsetzt, oder

b) eine Verbindung der allgemeinen Formel

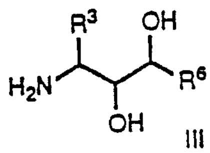

III .

worin $R^3$ und $R^6$ die oben angegebene Bedeutung besitzen, mit einer Verbindung der allgemeinen Formel

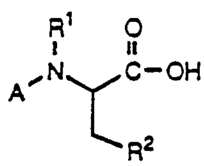

IV

worin R', $R^2$ und A die oben angegebene Bedeutung besitzen, oder einem aktivierten Derivat davon umsetzt, oder

c) eine Verbindung der Formel I, worin $R^4$ und $R^5$ je Wasserstoff bedeuten und die übrigen Symbole die oben angegebene Bedeutung besitzen, mit einem gegebenenfalls durch Amino, Monoalkylamino, Dialkylamino, Alkanoylamino, Alkanoyloxyamino, Carboxy, Alkoxy oder Hydroxy ein- oder mehrfach substituierten Alkanoylierungsmittel oder einem eine O-Schutzgruppe bildenden Mittel umsetzt, und

d) erwünschtenfalls ein Gemisch diastereomerer Racemate in die diastereomeren Racemate oder optisch reinen Diastereomeren auftrennt, und/oder

e) erwünschtenfalls ein Diastereomerengemisch in die optisch reinen Diastereomeren auftrennt, und/oder

f) erwünschtenfalls eine erhaltene Verbindung in ein pharmazeutisch verwendbares Salz überführt.

2. Verfahren gemäss Anspruch 1, worin $R^2$ Aethyl, Propyl, Isopropyl, Imidazol-4-yl, Thiazol-4-yl, Thien-2-yl oder t-Butoxy, $R^4$ und $R^5$ unabhängig voneinander je Wasserstoff, gegebenenfalls durch Carboxy, Alkoxy oder Hydroxy ein- oder mehrfach substituiertes Alkanoyl oder eine O-Schutzgruppe oder zusammen eine cyclische O-Schutzgruppe, $R^{12}$ Phenyl, substituiertes Phenyl oder Naphthyl und $R^{13}$ Wasserstoff, Alkoxycarbonylalkyl, Alkylcarbonylalkyl, Cycloalkylcarbonylalkyl, Heterocycloalkylcarbonylalkyl, Arylcarbonylalkyl, Aminocarbonylalkyl, substituiertes Aminocarbonylalkyl, Alkoxycarbonylamino, Arylalkoxycarbonylamino, Alkylthioalkyl, Alkylsulfinylalkyl oder Alkylsulfonylalkyl bedeuten.

3. Verfahren gemäss Anspruch 2, worin $R^2$ Aethyl, Propyl, Isopropyl oder Imidazol-4-yl, $R^4$ und $R^5$ je Wasserstoff, $R^7$ und $R^8$ unabhängig voneinander je Alkyl, Aryl oder Arylalkyl oder, zusammen mit den beiden sie verbindenden Atomen, Aryl, Heteroaryl, Cycloalkenyl oder Heterocycloalkenyl und $R^{14}$ Alkyl, Cycloalkyl, Arylalkyl, Cycloalkylalkyl, Alkylcarbonyl, Arylcarbonyl oder Arylalkylcarbonyl bedeuten.

4. Verfahren gemäss Anspruch 1, worin $R^1$ Wasserstoff, $R^2$ Propyl, Imidazol-2-yl, Imidazol-4-yl, Thiazol-4-yl, Thien-2-yl oder t-Butoxy, $R^3$ Cyclohexylmethyl, $R^4$ und $R^5$ unabhängig voneinander je Wasserstoff, gegebenenfalls durch Dialkylamino, Carboxy oder Alkoxy einfach substituiertes Alkanoyl oder zusammen eine cyclische O-Schutzgruppe, $R^6$ die Gruppe (a) oder (b), A die Gruppe (c) oder (d), D eine Methingruppe, $R^7$ Alkyl, $R^8$ Wasserstoff oder Alkyl, oder $R^7$ und $R^8$ zusammen mit den beiden sie verbindenden Atomen, Aryl, Heteroaryl oder Cycloalkenyl, $R^9$ Wasserstoff oder Alkyl, $R^{10}$ und $R^{11}$ unabhängig voneinander je Alkyl oder die Gruppe (e) oder zusammen mit den Kohlenstoffatomen, an das sie gebunden sind, Cycloalkyl oder Heterocycloalkyl, $R^{12}$ Phenyl, substituiertes Phenyl, Benzyl oder Naphthyl, $R^{13}$ Alkylcarbonylalkyl, Heterocycloalkylcarbonylalkyl, Alkylcarbonylhydroxyalkyl, Dialkoxyphosphoroxyalkyl, Arylalkyl, Alkylsulfinylalkyl oder Alkylsulfonylalkyl, Y den N-terminal mit Z verbundenen, bivalenten Rest von Phenylalanin, Z Acyl, X ein Sauerstoffatom und $R^{14}$ Wasserstoff oder Arylalkyl bedeuten.

5. Verfahren gemäss Anspruch 4, worin $R^1$ Wasserstoff, $R^2$ Imidazol-2-yl, Imidazol-4-yl oder Thiazol-4-yl, besonders bevorzugt Imidazol-4-yl, $R^3$ Cyclohexylmethyl, $R^4$ und $R^5$ unabhängig voneinander je Wasserstoff, gegebenenfalls durch Methoxy einfach substituiertes Alkanoyl oder zusammen das Acetal von Pivalinaldehyd, besonders bevorzugt je Wasserstoff, $R^6$ die Gruppe (b), A die Gruppe (c), $R^9$ Wasserstoff, $R^{10}$ und $R^{11}$ unabhängig voneinander je Alkyl oder zusammen mit dem Kohlenstoffatom, an das sie gebunden sind, Cycloalkyl, besonders bevorzugt Methyl, Aethyl, Cyclopropyl oder Cyclobutyl, $R^{12}$ Phenyl oder substituiertes Phenyl, besonders bevorzugt Phenyl, und $R^{13}$ Alkylcarbonylalkyl oder Alkylsulfonylalkyl, besonders bevorzugt $C_1$-$C_4$-Alkylcarbonylmethyl oder $C_1$-$C_4$-Alkylsulfonylmethyl, bedeuten.

6. Verfahren gemäss Anspruch 5, worin $R^1$ Wasserstoff, $R^2$ Imidazol-4-yl, $R^3$ Cyclohexylmethyl, $R^4$ und $R^5$ je Wasserstoff, $R^6$ die Gruppe (b), $R^9$ Wasserstoff, $R^{10}$ und $R^{11}$ je Methyl oder Aethyl oder zusammen mit dem Kohlenstoffatom, an das sie gebunden sind, Cyclopropyl oder Cyclobutyl, $R^{12}$ Phenyl und $R^{13}$ $C_1$-$C_4$-Alkylcarbonylmethyl oder $C_1$-$C_4$-Alkylsulfonylmethyl bedeuten.

7. Verfahren gemäss Anspruch 1, dadurch gekennzeichnet, dass man (S)-N-[(1S,2R,3S)-1-(Cyclohexylmethyl)-3-cyclopropyl-2,3-dihydroxypropyl]-α-[(R)-α-(3,3-dimethyl-2-oxobutyl)-hydrocinnamamido]imidazol-4-propionamid herstellt.

8. Verfahren gemäss Anspruch 1, dadurch gekennzeichnet, dass man (S)-α-[(S)-α-[(t-Butylsulfonyl)-methyl]hydrocinnamamido]-N-[(1S,2R,3S)-1-(cyclohexylmethyl)-3-cyclopropyl-2,3-dihydroxypropyl]imidazol-4-propionamid herstellt.

9. Verfahren gemäss Anspruch 1, dadurch gekennzeichnet, dass man (S)-α-[(S)-α-[(t-Butylsulfonyl)-methyl]hydrocinnamamido]-N-[(1S,2R,3S)-3-cyclobutyl-1-(cyclohexylmethyl)-2,3-dihydroxypropyl]imidazol-4-propionamid herstellt.

10. Verfahren gemäss Anspruch 1, dadurch gekennzeichnet, dass man (S)-N-[(S)-1-[(4R,5S)-2-t-Butyl-5-cyclopropyl-1,3-dioxolan-4-yl]-2-cyclohexyläthyl]-α-[(S)-α-[(t-butylsulfonyl)methyl]hydrocinnamamido]-imidazol-4-propionamid herstellt.

11. Verfahren gemäss Anspruch 1, dadurch gekennzeichnet, dass man (1S,2R)-[(S)-1-[(S)-α-[(S)-α-[(t-Butylsulfonyl)methyl]hydrocinnamamido]imidazol-4-propionamido]-2-cyclohexyläthyl]-1-isopropyläthylen-bis-(methoxyacetat) herstellt.

12. Verfahren gemäss Anspruch 1, dadurch gekennzeichnet, dass man (S)-α-[(S)-α-[(t-Butylsulfonyl)-methyl]hydrocinnamamido]-N-[(1S,2R,3S)-1-(cyclohexylmethyl)-3-cyclopentyl-2,3-dihydroxypropyl]imidazol-4-propionamid herstellt.

13. Verfahren gemäss Anspruch 1, dadurch gekennzeichnet, dass man (S)-α-[(S)-α-[(t-Butylsulfonyl)-methyl]hydrocinnamamido]-N-[(1S,2R,3S)-1-(cyclohexylmethyl)-3-cyclopropyl-2,3-dihydroxypropyl]-4-thiazolpropionamid herstellt.

14. Verfahren gemäss Anspruch 1, dadurch gekennzeichnet, dass man (S)-α-[(S)-α-[(t-Butylsulfonyl)-methyl]hydrocinnamamido]-N-[(1S,2S oder R,3S oder R,4SR)-1-(cyclohexylmethyl)-2,3,5-trihydroxypentyl]-imidazol-4-propionamid herstellt.

15. Verwendung eines Aminosäurederivates der in Anspruch 1 angegebenen Formel I in Form eines optisch reinen Diastereomeren, eines Diastereomerengemisches, eines diastereomeren Racemates oder eines Gemisches von diastereomeren Racematen oder ein pharmazeutisch verwendbaren Salzes einer solchen Verbindung zur Herstellung von Mitteln gegen Bluthochdruck und/oder Herzinsuffizienz.

16. Verbindungen der allgemeinen Formeln

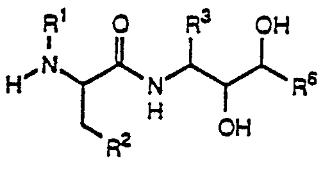

II

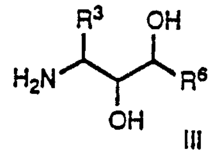

III

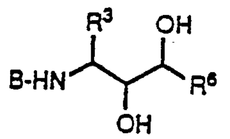

V

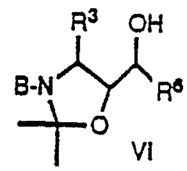

VI

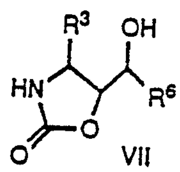

VII

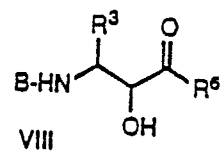

VIII

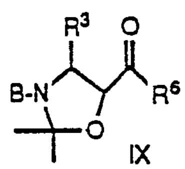

IX

,

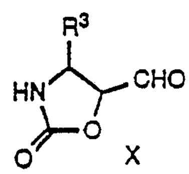

X

und

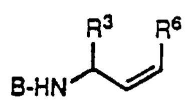

XVII

worin $R^1$ Wasserstoff oder Methyl, $R^2$ Aethyl, Propyl, Isopropyl, Imidazol-2-yl, Imidazol-4-yl, Pyrazol-3-yl, Thiazol-4-yl, Thien-2-yl odeer t-Butoxy, $R^3$ Isobutyl, Cyclohexylmethyl oder Benzyl und $R^6$ eine der Gruppen

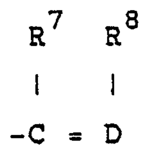

(a)

und

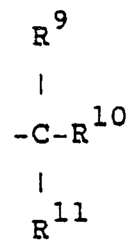

(b)

bedeuten, worin D eine Methingruppe oder ein Stickstoffatom, $R^7$ Alkyl, Aryl oder Arylalkyl, $R^8$ Wasserstoff, Alkyl, Aryl oder Arylalkyl, oder $R^7$ und $R^8$ zusammen mit den beiden sie verbindenden Atomen, Aryl, Heteroaryl, Cycloalkenyl oder Heterocycloalkenyl, $R^9$ Wasserstoff oder Alkyl und $R^{10}$ und $R^{11}$ unabhängig voneinander je Alkyl, Aryl, Arylalkyl, Cycloalkyl oder die Gruppe

$-CH_2-X-R^{14}$ (e)

oder zusammen mit dem Kohlenstoffatom, an das sie gebunden sind, Cycloalkyl oder Heterocycloalkyl und $R^{14}$ Wasserstoff, Alkyl, Cycloalkyl, Arylalkyl, Cycloalkylalkyl, Alkylcarbonyl, Arylcarbonyl oder Arylalkylcarbonyl bedeuten, mit der Massgabe, dass, falls $R^9$ Alkyl bedeutet, $R^{10}$ und $R^{11}$ ebenfalls Alkyl bedeuten.